# EUROPEAN PATENT APPLICATION

(11) **EP 4 332 226 A2**
(43) Date of publication of application: **06.03.2024**
(21) Application number: 24150956.1
(22) Date of filing: 07.11.2017
(51) Int. Cl.: C12N 15/63

(54) **AN ENGINEERED TWO-PART CELLULAR DEVICE FOR DISCOVERY AND CHARACTERISATION OF T-CELL RECEPTOR INTERACTION WITH COGNATE ANTIGEN**

(30) Priority: 07.11.2016 DK PA201670874
(62) Divisional of application: 17801380.1
(71) Applicant: Genovie AB, 151 36 Södertälje (SE)
(72) Inventor: JARVIS, Reagan, 151 36 Södertälje (SE); HILL, Ryan, 151 36 Södertälje (SE); PASE, Luke, 151 36 Södertälje (SE)
(74) Representative: Aera A/S

(57) **Abstract**

The present invention relates to a two-part device, wherein a first part is an engineered antigen-presenting cell system (eAPCS), and a second part is an engineered TCR-presenting cell system (eTPCS).

## Description

### Field of the invention

The present invention relates to the construction and use of an engineered cellular device comprising two parts, wherein each part comprises a distinct engineered cell system that are interacted with one another in operation of the system. The first part of the two-part cellular device is an antigen-presenting cell (APC) system that is engineered by genome editing to render the APC null for cell surface presentation of human leukocyte antigen (HLA) molecules, HLA-like molecules and distinct forms of antigen-presenting molecules and antigenic molecules. In addition, the engineered APC system (eAPCS) contains genomic integration sites for insertion of antigen-presenting molecule encoding open reading frames (ORFs), and optionally insertion of genetically encoded analyte antigens. Genetic donor vectors designed to target the genomic integration sites of the APC as to rapidly deliver analyte antigen molecule- and/or antigen-presenting complex encoding ORFs completes the eAPCS. The second part of the two-part cellular device is a T-cell Receptor (TCR)-presenting cell system that is engineered by genome editing to render the cell null for surface expression of TCR chains. This engineered TCR-presenting cell system (eTPCS) remains competent to express TCR heterodimeric complexes on the cell surface in a CD3 complex context upon introduction of TCR-encoding ORFs, and is also competent to respond to TCR ligation in a detectable manner. Genetic donor vectors designed to target the genomic integration sites of the TCR-presenting cell as to rapidly deliver analyte TCR ORFs completes the eTPCS. This engineered two-part cellular device is designed for standardised analysis of TCR/antigen interactions in the native functional context of cell-cell communication.

### Introduction to the invention

Immune surveillance by T lymphocytes (T-cells) is a central function in the adaptive immunity of all jawed vertebrates. Immune surveillance by T-cells is achieved through a rich functional diversity across T-cell subtypes, which serve to eliminate pathogen-infected and neoplastic cells and orchestrate adaptive immune responses to invading pathogens, commensal microorganisms, commensal non-self factors such as molecular components of foodstuffs, and even maintain immune tolerance of self. In order to respond to various foreign and self factors, T-cells must be able to specifically detect molecular constituents of these foreign and self factors. Thus T-cells must be able to detect a large cross-section of the self and non-self molecules that an individual encounters, with sufficient specificity to mount efficient responses against pathogenic organisms and diseased self, while avoiding the mounting of such responses against health self. The highly complex nature of this task becomes clear when considering the practically unlimited diversity of both foreign and self molecules, and that pathogenic organisms are under evolutionary pressure to evade detection by T-cells.

### The T-cell Receptor (TCR)

T-cells are primarily defined by the expression of a T-cell receptor (TCR). The TCR is the component of the T-cell that is responsible for interacting with and sensing the targets of T-cell adaptive immunity. In general terms, the TCR is comprised of a heterodimeric protein complex presented on the cell surface. Each of the two TCR chains are composed of two extracellular domains, being the variable (V)-region and the constant (C)-region, both of the immunoglobulin superfamily (IgSF) domain, forming antiparallel β-sheets. These are anchored in the cell membrane by a type-I transmembrane domain, which adjoins a short cytoplasmic tail. The quality of the T-cells to adapt and detect diverse molecular constituents arises from variation in the TCR chains that is generated during T-cell genesis. This variation is generated by somatic recombination in a similar manner to antibody genesis in B-cells.

### TCR chain diversity

The T cell pool consists of several functionally and phenotypically heterogeneous subpopulations. However, T cells may be broadly classified as αβ or γδ according to the somatically rearranged TCR form they express at their surface. There exist two TCR chain pair forms; TCR alpha (TRA) and TCR beta (TRB) pairs; and TRC gamma (TRG) and TCR delta (TRD) pairs. T-cells expressing TRA:TRB pairs are referred to as αβ T-cells, while T-cells expressing TRG:TRD pairs are often referred to as γδ T-cells.

TCRs of both αβ and γδ forms are responsible for recognition of diverse ligands, or `antigens', and each T-cell generates αβ or γδ receptor chains de novo during T-cell maturation. These de novo TCR chain pairs achieve diversity of recognition through generation of receptor sequence diversity in a process called somatic V(D)J recombination after which each T-cell expresses copies of a single distinctly rearranged TCR. At the TRA and TRG loci, a number of discrete variable (V) and functional (J) gene segments are available for recombination and juxtaposed to a constant (C) gene segments, thus referred to as VJ recombination. Recombination at the TRB and TRD loci additionally includes a diversity (D) gene segment, and is referred to as VDJ recombination.

Each recombined TCR possess potential for unique ligand specificity, determined by the structure of the ligand-binding site formed by the α and β chains in the case of αβ T-cells or γ and δ chains in the case of γδ T-cells. The structural diversity of TCRs is largely confined to three short hairpin loops on each chain, called complementarity-determining regions (CDR). Three CDRs are contributed from each chain of the receptor chain pair, and collectively these six CDR loops sit at the membrane-distal end of the TCR extracellular domain to form the antigen-binding site.

Sequence diversity in each TCR chain is achieved in two modes. First, the random selection of gene segments for recombination provides basal sequence diversity. For example, TRB recombination occurs between 47 unique V, 2 unique D and 13 unique J germline gene segments. In general, the V gene segment contributes both the CDR1 and CDR2 loops, and are thus germline encoded. The second mode to generate sequence diversity occurs within the hypervariable CDR3 loops, which are generated by random deletion of template nucleotides and addition of non-template nucleotides, at the junctions between recombining V, (D) and J gene segments.

### TCR:CD3 Complex

Mature αβ and γδ TCR chain pairs are presented at the cell surface in a complex with a number of accessory CD3 subunits, denoted ε, γ, δ and ζ. These subunits associate with αβ or γδ TCRs as three dimers (εγ, εδ, ζζ). This TCR:CD3 complex forms the unit for initiation of cellular signalling responses upon engagement of a αβ or γδ TCR with cognate antigen. The CD3 accessories associated as a TCR:CD3 complex contribute signalling motifs called immunoreceptor tyrosine-based activation motifs (ITAMs). CD3ε, CD3γ and CD3δ each contribute a single ITAM while the CD3ζ homodimer contains 3 ITAMs. The three CD3 dimers (εγ, εδ, ζζ) that assemble with the TCR thus contribute 10 ITAMs. Upon TCR ligation with cognate antigen, phosphorylation of the tandem tyrosine residues creates paired docking sites for proteins that contain Src homology 2 (SH2) domains, such as the critical ζ-chain-associated protein of 70 kDa (ZAP-70). Recruitment of such proteins initiate the formation of TCR:CD3 signalling complexes that are ultimately responsible for T-cell activation and differentiation.

### αβ T-cells

αβ T-cells are generally more abundant in humans than their γδ T-cell counterparts. A majority of αβ T-cells interact with peptide antigens that are presented by HLA complexes on the cell surface. These peptide-HLA (pHLA)-recognising T-cells were the first to be described and are by far the best characterised. More rare forms of αβ T-cells have also been described. Mucosal-associated invariant T (MAIT) cells appear to have a relatively limited α and β chain diversity, and recognise bacterial metabolites rather than protein fragments. The invariant natural killer T-cells (iNK T-cells) and germline-encoded mycolyl-reactive T-cells (GEM T-cells) are restricted to recognition of glycolipids that are cross-presented by non-HLA molecules. iNK T-cells are largely considered to interact with CD1d-presented glycolipids, whereas GEM T-cells interact with CD1b-presented glycolipids. Further forms of T-cells are thought to interact with glycolipids in the context of CD1a and CD1c, however, such cells are yet to be characterised in significant detail.

### Conventional αβ T-cells

The key feature of most αβ T-cells is the recognition of peptide antigens in the context of HLA molecules. These are often referred to as 'conventional' αβ T-cells. Within an individual, self-HLA molecules present peptides from self and foreign proteins to T-cells, providing the essential basis for adaptive immunity against malignancies and foreign pathogens, adaptive tolerance towards commensal organisms, foodstuffs and self. The HLA locus that encodes HLA proteins is the most gene-dense and polymorphic region of the human genome, and there are in excess of 12,000 alleles described in humans. The high degree of polymorphism in the HLA locus ensures a diversity of peptide antigen presentation between individuals, which is important for immunity at the population level.

### HLA class I and II

There are two forms of classical HLA complexes: HLA class I (HLAI) and HLA class II (HLAII). There are three classical HLAI genes: *HLA-A, HLA-B, HLA-C.* These genes encode a membrane-spanning α-chain, which associates with an invariant β2-micro-globulin (β2M) chain. The HLAI α-chain is composed of three domains with an immunoglobulin fold: α1, α2 and α3. The α3 domain is membrane-proximal and largely invariant, while the α1 and α2 domains together form the polymorphic membrane-distal antigen-binding cleft. There are six classical HLAII genes: *HLA-DPA1, HLA-DPB1, HLA-DQA1, HLA-DQB1, HLA-DRA,* and *HLA-DRB1.* These genes encode paired DP, DQ and DR heterodimeric HLA complexes comprising a α-chain and a β-chain. Each chain has two major structural domains with an immunoglobulin fold, where the α2 and β2 domain comprise membrane-proximal and largely invariant modules similar to that of HLAI α3 domain. The HLAII α2 and β2 domains together form the membrane-distal antigen-binding cleft and are regions of high polymorphism.

The antigen-binding cleft of HLAI and HLAII comprises two anti-parallel α-helices on a platform of eight anti-parallel β-sheets. In this cleft the peptide antigen is bound and presented in an extended conformation. The peptide-contacting residues in HLAI and HLAII are the location of most of the sequence polymorphism, which constitutes the molecular basis of the diverse peptide repertoires presented by different HLA alleles. The peptide makes extensive contacts with the antigen-binding cleft and as a result each HLA allele imposes distinct sequence constraints and preferences on the presented peptides. A given peptide will thus only bind a limited number of HLAs, and reciprocally each allele only accommodates a particular fraction of the peptide collection from a given protein. The set of HLAI and HLAII alleles that is present in each individual is called the HLA haplotype. The polymorphism of HLAI and HLAII genes and the co-dominant expression of inherited alleles drives very large diversity of HLA haplotype across the human population, which when coupled to the enormous sequence diversity of αβ TCRs, presents high obstacles to standardisation of analysis of these HLA-antigen-TCR interactions.

### αβ TCR engagement of HLAI and HLAII

αβ TCRs recognize peptides as part of a mixed pHLA binding interface formed by residues of both the HLA and the peptide antigen (altered self). HLAI complexes are presented on the surface of nearly all nucleated cells and are generally considered to present peptides derived from endogenous proteins. T-cells can thus interrogate the endogenous cellular proteome of an HLAI-presenting cell by sampling pHLAI complexes of an interacting cell. Engagement of HLAI requires the expression of the TCR co-receptor CD8 by the interacting T-cell, thus HLAI sampling is restricted to CD8⁺ αβ T-cells. In contrast, the surface presentation of HLAII complexes is largely restricted to professional APC, and are generally considered to present peptides derived from proteins exogenous to the presenting cell. An interacting T-cell can therefore interrogate the proteome of the extracellular microenvironment in which the presenting cell resides. The engagement of HLAII requires the expression of the TCR co-receptor CD4 by the interacting T-cell, thus HLAII sampling is restricted to CD4⁺ αβ T-cells.

### Thymic selection of αβ TCRs

The role of αβ TCRs as described above is the detection of pHLA complexes, such that the TCR-presenting T-cell can raise responses germane to the role of that T-cell in establishing immunity. It should be considered that the αβ TCR repertoire generated within an individual must account for the immense and unforeseen diversity of all foreign antigens likely to be encountered in the context of a specific haplotype and prior to their actual occurrence. This outcome is achieved on a background where extremely diverse and numerous αβ TCRs are generated in a quasi-randomised manner with the potential to recognise unspecified pHLA complexes while only being specifically instructed to avoid strong interactions with self pHLA. This is carefully orchestrated during T-cell maturation in a process call thymic selection.

During the first step of T-cell maturation in the thymus, T-cells bearing αβ TCRs that are incapable of interacting with self-pHLA complexes with sufficient affinity, are deprived of a survival signal and eliminated. This step called positive selection assures that the surviving T-cells carry a TCR repertoire that is at least potentially capable of recognizing foreign or altered peptides presented in the right HLA context. Subsequently, αβ TCR that strongly interact with self-pHLA and thus have the potential to drive autoimmunity are actively removed through a process of negative selection. This combination of positive and negative selection results in only T-cells bearing αβ TCRs with low affinity for self-pHLA populating the periphery. This establishes an αβ T-cell repertoire that is self-restricted but not self-reactive. This highly individualised nature of T-cell genesis against HLA haplotype underscores the challenges in standardised analysis αβ TCR-antigen-HLA interactions. Moreover, it forms the basis of both graft rejection and graft versus host disease and the general principle that αβ TCRs identified in one individual may have completely different effect in a second individual, which has clear implications for TCR-based and T-cell based therapeutic and diagnostic strategies emerging in clinical practice.

### Unconventional αβ T-cells

The non-HLA-restricted, or 'unconventional', forms of αβ T-cells have very different molecular antigen targets. These unconventional αβ T-cells do not engage classical HLA complexes, but rather engage conserved HLA-like proteins such as the CD1 family or MR1. The CD1 family comprises four forms involved in antigen cross-presentation (CD1a,b,c and d). These cell surface complexes have an α-chain resembling HLAI, which forms heterodimers with β2-M. A small hydrophobic pocket presented at the membrane distal surface of the α-chain forms a binding site for pathogen-derived lipid-based antigens. Innate like NK T-cells (iNK T-cells) form the best-understood example of lipid/CD1 family recognition with GEM T-cells representing another prominent example. 'Type I' iNK T-cells are known to interact strongly with the lipid α-GalCer in the context of CD1d. These iNK T-cells display very limited TCR diversity with a fixed TCR α-chain (Vα10/Jα18) and a limited number of β-chains (with restricted vβ usage) and they have been likened to innate pathogen-associated molecular patterns (PAMPS) recognition receptors such as Toll-like and Nod-like receptors. In contrast, 'type II' NK T-cells present a more diverse TCR repertoire, and appear to have a more diverse mode of CD1d-lipid complex engagement. GEM T-cells recognise mycobacteria-derived glycolipids presented by CD1b, however, the molecular details of antigen presentation by CD1a, b and c as well as their T-cell recognition are only beginning to be understood.

MAIT cells largely express an invariant TCR α-chain (TRAV1-2 ligated to TRAJ33, TRAJ20, or TRAJ12), which is capable of pairing with an array of TCR β-chains. Instead of peptides or lipids MAIT TCRs can bind pathogen-derived folate- and riboflavin-based metabolites presented by the HLAI-like molecule, MR1. The limited but significant diversity in the TCRs observed on MAIT TCRs appear to enable the recognition of diverse but related metabolites in the context of the conserved MR1.

It is not well-understood how non-classical HLA-restricted αβ T-cell TCRs are selected in the thymus during maturation. However, it appears likely that the fundamental process of negative and positive selection outlined above still applies and some evidence suggests that this occurs in specialized niches within the thymus.

### γδ T-cells

In contrast to the detailed mechanistic understanding of αβ TCR genesis and pHLA engagement, relatively little is known about the antigen targets and context of their γδ T-cell counterparts. This is in part due to their relatively low abundance in the circulating T-cell compartment. However, it is broadly considered that γδ T-cells are not strictly HLA restricted and appear to recognize surface antigen more freely not unlike antibodies. Additionally, more recently it has become appreciated that γδ T-cells can dominate the resident T-cell compartment of epithelial tissues, the main interaction site of the immune system with foreign antigen. In addition, various mechanisms for γδ T-cell tumour immunuosurveillance and surveillance of other forms of dysregulated-self are beginning to emerge in the literature. The specific antigen targets of both innate-like and adaptive γδ T-cells remain poorly defined but the tissue distribution and fast recognition of PAMPs suggests a fundamental role for γδ T-cells both early in responses to foreign antigens as well as early in life when the adaptive immune system is still maturing.

The diverse functions of γδ T-cells appear to be based on different Vγ Vδ gene segment usage and can be broadly understood in two main categories in which γδ T-cells with largely invariant TCRs mediate innate-like recognition of PAMPs very early during infection. Beyond PAMPs these type of γδ T-cells are furthermore believed to recognize self-molecules, including phosphoantigens that could provide very early signatures of cellular stress, infection and potentially neoplastic development. Recognition of PAMPs and such so-called danger associated molecular patterns (DAMPS) as well as the large numbers of tissue-restricted innate-like γδ T-cells strongly suggests that these cells are suited to respond rapidly to antigenic challenge without the need for prior activation, homing and clonal expansion.

A second form of γδ T-cells are considered to be more adaptive in nature, with a highly diverse γδ TCR repertoire and the ability to peripherally circulate and access lymphoid tissues directly. Such antigen-specific γδ T-cells to common human pathogens such as CMV have been described and they appear to form a memory response. However, it has also been observed that γδ T-cells show only relatively limited clonal proliferation after activation and little data is available on the extent of TCR diversity and specific responses of γδ T-cells in peripheral circulation, or in tissues. Furthermore, while it is generally considered that γδ TCRs do not interact with pHLA complexes, and thus do not engage with peptide antigens in this context only few antigen targets of γδ T-cells have been characterised and the underlying molecular framework is only poorly understood.

The low frequency of peripheral γδ T-cells and the difficulty to study tissue-resident T-cells in humans has limited our knowledge of how this important and diverse type of T-cells participate in adaptive immune responses. This emerging area of research would require more reliable technologies with which to capture and characterise rare γδ T-cells, isolate their TCR pairs, and to identify their cognate antigens.

### Antigens and Antigen-presenting cells

In the context of T-cells and TCRs, antigens may be defined as any molecule that may be engaged by a TCR and resulting in a signal being transduced within the T-cell. The most well characterised T-cell antigens are peptides presented in an HLAI and HLAII complex, and which are engaged by conventional αβ T-cells. However, in recent years it has become apparent that non-conventional αβ T-cells and γδ T-cells are able to engage a wide range of biomolecules as antigens, including lipids, lipopeptides, glyco-peptides, glycolipds and a range of metabolites and catabolites. In addition, it has emerged that γδ T-cells may be able to engage fully folded proteins directly in an antibody-like fashion. Therefore, the view of T-cell antigens being largely restricted to HLA-presented peptides has expanded over the past two decades to include almost any biomolecule. With this concept in mind, it is relevant to define what may be considered an antigen-presenting cell (APC).

As defined in the above sections, HLAI and HLAII have a disparate expression profiles across cell types. It is widely accepted that nearly all nucleated cells present HLAI complexes on the cell surface, and are thus competent to present peptide antigens for T-cell sampling. In contrast, HLAII has a restricted expression profile, and at least in steady state conditions is only expressed on the surface of cells that have a specialist role in antigen presentation, including dendritic cells (DC), macrophage and B-cells. These specialist cell types are often referred to as professional APC. For the purposes of this document, the term APC is used to describe any nucleated cell that is capable of presenting an antigen for sampling by αβ or γδ T-cells. Such antigens are not restricted to those presented as 'cargo' in specific antigen-presenting complexes such as HLA and HLA-like molecules, but may also include any cell-surface presented moiety that is able to engage a αβ or γδ TCR-bearing cell.

### Therapeutic use of TCRs

Adoptive transfer of primary T-cells was first trialled in a clinical setting in the early 1990s, starting with ex vivo expanded T-cells polarised towards viral antigens to confer viral immunity in immunocompromised patients. Similar approaches using primary T-cells expanded ex vivo against specific cancer antigens were soon after trialled in treatment of malignancies. One limitation in these early approaches that continues to be a challenge today is a lack of understanding of the nature and diversity of T-cells clashing with the need to finely-optimize their composition in the therapeutic product. At present, the use of ex vivo expanded primary T-cells has largely been abandoned by the pharmaceutical industry with the exception of a handful of initiatives using primary T-cells with specificity for viral antigens.

In recent years the ability to reliably introduce genetic material into primary human cells has seen a variety of experimental genetically modified T-cell therapeutics arise. Such therapeutic cell products aim to harness the power of T-cell responses and redirect T-cell specificity towards a disease-associated antigen target, for example, an antigen uniquely expressed by malignant cells. These have largely relied on the transfer of a chimeric antigen receptor (CAR) into recipient T-cells, rather than actual TCR chain pairs. A CAR represents a targeting moiety (most often a single-chain antibody element targeting a surface expressed protein of malignant cells) grafted to signal receptor elements such as the ζ-chain of the CD3 complex, to produce a synthetic chimeric receptor that mimics CD3-TCR function. These so-called CAR T-cell (CAR-T) products have met mixed success in clinical trials to date and despite their potential are not easy to translate beyond tumours with inherent unique molecular targets such as B-cell malignancies. Alternatively, the transfer of full-length TCR chain pair ORFs into T-cells is of emerging interest. Such TCR-engineered T-cell therapeutics are at present limited by challenging manufacturing processes, and like the CAR-T products, a dearth of validated antigen targets and targeting constructs. To date this has been focused on the use of αβ TCRs for recognition of peptide antigens presented by HLAI on malignant cells and a fundamental challenge of this approach is the need for antigens that are specific to malignant cells.

It has been considered that since the TCR-pHLA interaction is of relatively low-affinity, native TCRs are likely to be suboptimal for TCR-engineered T-cell therapies. Several approaches have been devised to affinity-mature TCRs in vitro, in much the same manner as single-chain antibody affinity maturation. These TCR affinity maturation approaches generally also utilise a single-chain formats, wherein the V-region of one chain is fused to V-region of another chain to make a single polypeptide construct. Such single polypeptides may then be used in phage- or yeast- display systems adapted from antibody engineering workflows, and passed through rounds of selection based on target binding. Two inherent limitations exist in such a single-chain TCR approach in terms of yielding functional TCR chain pairs. Firstly, the selection is based on binding affinity to the target. However, it has been well documented that TCR affinity does not always correlate to the strength or competency of TCR signalling output. Secondly, the selection of single-chain constructs based on affinity does not always translate to equivalent affinities once they are reconstituted as full-length receptors.

In a therapeutic context, there exists an additional and crucial limitation in affinity-matured TCR pairs. That is, considering their sequences have been altered, the resulting constructs by definition have no longer been subject to thymic selection, wherein TCRs that react strongly to self-antigens are deleted from the repertoire. Therefore, these modified TCRs carry an inherent risk of being auto-reactive, which is very difficult to rule out in vitro using current methods. For the same reason, any selected or engineered TCR for therapeutic application needs to be individualised. If TCRs are artificially engineered or native TCRs applied across individuals, cross-reactivities have to be ruled out on the basis of the HLA haplotype and presented peptide repertoire of each specific individual in order to avoid potentially catastrophic autoimmunity. This is due to the fact that thymic selection is conducted on a background of all available HLA molecules specific only to that given individual. The likelihood of such cross-reactivity is unclear. However, the ability of our TCR repertoire to recognize pHLA complexes of other individuals of the same species as foreign is a fundamental property of adaptive immunity and underpins graft rejection and graft versus host disease. Recent clinical trials using a matured TCR chain pair against the cancer-specific melanoma associated antigen (MAGE) highlighted the potential problem of bypassing thymic selection. When autologous T-cells harbouring the matured TCRs were infused back to two cancer patients, these patients rapidly developed a fatal heart disease. Subsequent studies determined that the MAGE-specific matured TCRs were cross-reactive with an HLAI-presented peptide from the heart protein titin. This strongly suggests that cross-reactivity is a distinct possibility in therapeutic use of TCRs.

A distinct avenue of utilising TCRs for therapeutic purposes is in their use as affinity reagents in much the same manner as antibody therapeutic substances. Single-chain TCR molecules have been trialled for delivery of conjugated drug substances to specific HLA-antigen expressing cell populations. Such an approach is generally considered safer than CAR-T or TCR engineered T-cell therapeutics, as administration of the drug substance may simply be withdrawn. However, the potential for cross-reactivity and off target effects that are difficult to predict remains a potential limitation in this setting.

### TCR repertoire detection in clinical diagnostics

In a related aspect, there is an emerging interest in using the detection of the abundance of specific TCR sequences for clinical diagnostic purposes. With the rise of deep-sequencing methods in particular, it is possible to capture the full TCR diversity within an individual globally and for matched αβ pairs in specific contexts. This potentially represents a means to diagnose specific conditions and disease states simply by detecting the abundance of expanded T-cell clones, as proxy readout for established immune response against a disease-associated antigen in the patient. However, such global approaches are currently limited to very strong immune responses with established clinical time-points and suffer from the underlying difficulty in identifying the specific antigen target of any particular TCR identified via sequencing.

### Therapeutic and diagnostic use of T-cell antigens

The fundamental strength of harnessing adaptive immune responses translates into a central technical challenge in that the exquisite specificity of the TCR-antigen interaction requires detailed knowledge of the antigens specifically associated with each pathogen, cancer cell or autoimmune disease. Furthermore, each antigen may be presented by a specific antigen presenting complex, or allele thereof, such that antigen discovery has be performed for each relevant HLA gene and allele. For several infectious diseases like HIV, influenza and CMV that are associated with strong adaptive immune responses and generally display conserved epitope response hierarchies, the most important epitopes have been mapped in context of some common HLA. Similarly, the fields of cancer, allergy and autoimmunity have seen increased and systematic efforts to map the associated T-cell antigens. However, these are challenging procedures and the efforts to systematically describe T-cell antigens associated with different clinical contexts are hindered by the absence of efficient, robust, fast and scalable protocols.

Specifically, cancer cells represent a challenging and important aspect as most of the peptides presented on the surface of malignant cells are self antigens or very similar to self antigens. Therefore, thymic selection will have deleted TCRs that could strongly recognize these peptides, while at the same time the tumour has evolved to evade immune recognition. This means that potent immune responses against established tumours are relatively rare and targets difficult to predict or discover. However, these responses do exist and, importantly, are generally associated with better outcome. The target of such responses, tumour-associated-antigens (TAA), will in most cases have distinguishing characteristics from self and be derived from proteins that are overex-pressed during cancer development, otherwise absent from the cell type at this stage of development or specifically altered through genetic mutation or post-translational modifications such as phosphorylation.

When available, the knowledge of such epitopes makes it possible to interrogate the associated T-cell response for fundamental discovery, diagnostic purposes and for example as a test of vaccine efficacy. Importantly, they also provide highly specific targets for T-cell tolerization in allergy and autoimmunity and, crucially, point towards valuable targets for specific immunotherapy and against malignant cells. Malignancies represent a particularly valuable target as the promise of cellular immunotherapies and the progress in the T-cell manipulations are slowed by a lack of validated target TAAs that go beyond the few cases where specific markers for the type of cancer happen to be available.

In the light of the potential of cellular therapy and lack of validated targets the identification of promising TCR antigens remains one of the most pressing bottlenecks of TCR-based immunotherapy, particularly in the effort to treat cancer.

### Technological aspects of TCR and T-cell antigen analyses

Overall, the development of TCR-based therapies is still in its early stages, and success has been limited. Diagnostic approaches, while of immense potential, have seldom been deployed into controlled clinical studies that aim to assess patient disease states or response to therapy. Underdeveloped techniques for the reliable capture of native TCR chain pairs, and the systematic analysis of TCR-antigen interactions at high-throughput and in a functional context of cell-cell communication, has been the main hurdle to the development of TCR-based therapies and diagnostics.

Deep sequencing approaches have led to an improved understanding of T-cell receptor diversity in heath and disease. However, these approaches have generally focused on short stretches spanning the CDR3 regions, mainly of the TCR β-chain. Most studies have ignored the contribution of the TCR α-chain, and few have sought to analyse paired αβ chains as well as the antigen specificity of TCRs determined to be of interest. Recent workflows using single cell encapsulation and genetic barcoding has enabled the pairing of native TCR αβ or γδ chain pairs and analysis of full-length sequences, however, such workflows remain experimental.

Isolated TCR chain pairs may be analysed in terms of antigen specificity in either biophysical or functional modes. Biophysical analysis requires the recombinant production of both the TCR as well as the analyte antigen in soluble form. In the case of HLA-restricted TCRs this would thus require the generation of all individual TCRs as well as the cognate pHLA complexes. This is technically highly challenging, slow and very low-throughput. Furthermore, such analysis would only provide interaction affinities, which are not well-correlated with functional characteristics in predictable ways.

Until recently, the detailed functional analysis of isolated TCR sequences in a cellular context has been limited to laborious protocols of transfection of analyte TCR chain pairs into primary T-cells or immortal T-cell lines, and detection of cellular responses by traditional flow cytometric analysis of cell activation, or detection of secreted factors from the transfected cells upon antigen challenge. In a recent publication by Guo et al, rapid cloning, expression, and functional characterization of paired TCR chains from single-cells was reported (Molecular Therapy - Methods and clinical development (2016) 3:15054). In this study, analyte human αβ TCR pairs were expressed in a reporter cell line that lacked αβ TCR expression, and which contained a green fluorescent protein (GFP) reporter system linked to the Nur77 promoter that is activated upon TCR stimulation. This system remains inefficient due to the lack of standardised TCR integration into the reporter cell line genome, and does not provide a systematic manner for cell-bound antigen challenge by an APC element.

Similar to workflows for identification of TCRs against known T-cell antigens, the de novo discovery of novel T-cell antigens in health and disease remains highly challenging. Most approaches remain biophysical in nature, and aim to produce candidate antigens that may be tested in immunisation protocols, or through identifying cognate TCRs as addressed above. Little or no standardisation exists in the field of T-cell antigen discovery, and the field is largely restricted to academic study.

With the accumulating interest in TCRs and their cognate antigens in both therapeutic and diagnostic use, and the emergence of means to capture significant numbers of native TCR αβ and γδ chain pairs, there remains a lack of reliable high-throughput and standardised technologies for the systematic analysis of TCR-antigen interactions. Importantly, there is a lack of standardised systems for functional analysis of TCR chain pairs in the native context of cell-cell communication wherein both the TCR and antigen are presented by a viable cell. Moreover, there is a lack of systems that may achieve TCR candidate selection, and/or affinity maturation of TCR chain pairs, in the relevant context of cell-cell communication.

As described, there is currently a lack of standardised technologies for the high-throughput generation of cells with TCR pairs expressed in a native cellular context. It is highly desirable to possess a system in which full-length TCR pairs may be inserted as single copies into the genome of a TCR-presenting cell such that said TCRs are presented in a native CD3 cell-surface complex for analysis and selection. A CD3 complex-presented TCR pair assures that affinity analyses are reflective of the actual native TCR composition, which is not the case for single-chain TCR and other non-native TCR-display technology. Moreover, the presentation of TCR pairs in a CD3 complex on a viable cell is an absolute requirement for functional analysis of TCR pairs. Functional analysis, meaning analysis of TCR signalling output, is of critical importance in TCR selection and engineering workflows where signal output is the parameter that is generally of greatest importance for use in the context of cellular therapeutics, and is not well correlated with the affinity of a TCR with cognate antigen/HLA as is determined within other display platforms.

### Detailed description of the invention

The present invention addresses the above-mentioned needs. In particular, the present invention relates to the construction and use of an engineered two-part cellular device for discovery and characterisation of TCRs and T-cell antigens. The two parts of the overall cellular device are comprised of distinct engineered cell types that are contacted with one another in operation of the device. The device is used for standardised functional analysis of responsiveness of analyte TCRs towards analyte antigens. The readout of such responsiveness is used for the identification and selection of TCRs, T-cell antigens, and detailed functional characterisation of the TCR/antigen interactions. The device presents analyte antigens through a first cell population, the engineered antigen presenting cell (eAPC) that is prepared using the eAPC system (eAPCS). The device presents analyte TCRs through a second cell population, the engineered TCR-presenting cell (eTPC) that is prepared using the eTPC system (eTPCS).

The two-part device of the invention contains features that enable a high degree of standardisation, reproducibility and critically restricts the complexity of analyte TCR and T-cell antigens collections. Primarily, this standardisation is achieved through defined and highly controlled integration of analyte antigen-presenting complexes and antigens to the genome of eAPC, and TCR to the genome of eTPC. This permits rapid cycle times to generate analyte cell populations, and greatly reduces costs of current random-integration and viral vector platforms. Moreover, the present invention permits the generation of cell-based arrays of analyte entities in both a eAPC- and eTPC- centric manner, meaning that large libraries of vectors can be integrated into either cell population, and assayed as libraries of cells expressing single analyte entities per cell. Single-copy genomic receiver sites being engineered into the genome of eAPC and eTPC achieve this, such that when provided by an ORF (for HLA, antigen or TCR chain) within the matched donor vectors, only a single copy of an ORF can be integrated from the vector pool. This means that if the vector pool comprises ORFs of mixed identify, each integrated cell will only integrate a single identity; essentially creating cell-based arrays akin to other display platforms like bacteriophage. This high level of standardisation and reduction of complexity is in contrast to the current methods using primary or immortal cell cultures and extended outgrowth and endogenous singling responses to achieve outputs, or in partial systemisation of APC- or T-cell- centric inputs. Importantly, the two-part engineered cell device that represents the present invention achieves standardisation of relevant functional responses in the context of cell-cell communication. This is critical for reducing time and costs of discovery and clinical testing of new TCR and T-cell antigen candidates by increasing the predictability of their effects in vivo.

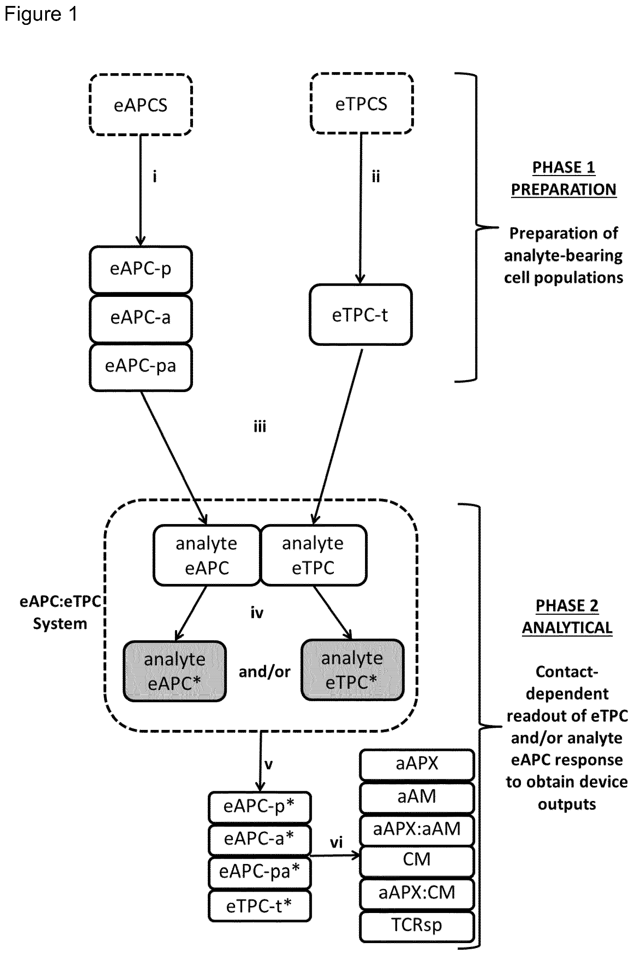

The present invention relates to the provision and operation of a two-part device, wherein a first part is an **engineered antigen-presenting cell system (eAPCS),** and a second part is an **engineered TCR-presenting cell system (eTPCS).** Overall, this device represents a multicellular analytical system comprised of two distinct types of engineered human cells for functional analyses of TCR recognition of T-cell antigens. The primary outputs of this device are cells that express analyte antigen or analyte TCR, which subsequently may be used to determine the terminal outputs of the device as specific T-cell antigens or TCR sequences, respectively **(****Figure 1****).**

The two-part device is operated in two phases comprising
i. Phase 1, the preparation of analyte antigen and analyte TCR bearing cell populations and their assembly into a combined system contacting two analyte cell populations
ii. Phase 2, the readout of responses intrinsic to either of the contacted cell populations of the combined system to obtain the outputs of the device,
wherein the eAPCS and the eTPCS provide the means to prepare analyte eAPC and eTPC populations, respectively, to be assembled into the combined eAPC:eTPC system **(****figure** 1, **steps i and** ii).

The eAPCS is defined as a system to prepare various forms of analyte eAPC populations that are provided to the combined eAPC:eTPC system, wherein the analyte eAPC populations are defined as one of the following
i. an eAPC-p
ii. an eAPC-a
iii. an eAPC-pa
iv. Libraries of thereof
wherein the selection of the input analyte eAPC population to the combined eAPC:eTPC system is determined by the nature of the antigens that are the subject of the operation of the device. That is, the required eAPC population combined into the eAPC:eTPC system is defined by the required primary output from the device, and/or the required terminal output from the device **(****figure** 1, **step** i).

The eTPCS is defined as a system to prepare analyte eTPC populations that are provided to the combined eAPC:eTPC system, wherein the analyte eTPC populations are defined as one of the following
i. an eTPC-t
ii. Libraries of eTPC-t
wherein the selection of input analyte eTPC populations to the combined eAPC:eTPC system is determined by the nature TCRs that are the subject of the operation of the device. That is, the required eTPC population combined into the eAPC-eTPC system is defined by the required primary output from the device, and/or the required terminal output from the device **(****figure** 1, **step** ii).

Primary outputs from the device are selected cell populations, which have or have not responded to the analyte presented by the reciprocal cell provided in the eAPC:eTPC system. That is, such a primary output may be represented as a single cell, or a pool of cells, that have been selected on the presence or absence of a reported response within the combined eAPC:eTPC system **(****Figure 1** **step v).** A response within an analyte eAPC is only provoked by engagement of a cognate TCR presented by a contacting eTPC. A response within an analyte eTPC is only provoked by engagement of a cognate antigen presented by a contacting eTPC **(****figure 1** **step iv).**

A selection of analyte eAPC and/or analyte eTPC from the combined eAPC:eTPC system may be made on the basis of a response in the contacting cell. That is, an analyte eAPC may be selected on that basis of a reported response, or lack thereof, in the contacting analyte eTPC. Conversely, an analyte eTPC may be selected on that basis of a reported response, or lack thereof, in the contacting analyte eAPC.

Primary eAPC outputs from the device are selected cells, wherein selection is made based on the presence or absence of a reported signal response, and these cells may comprise one or more of
i. an eAPC-p
ii. an eAPC-a
iii. an eAPC-pa
wherein the selected cells may comprise a single cell, a pool of cells of the same identity, a pool of cells of different identities **(****Figure 1** **step v).**

Primary eTPC outputs from the device are selected cells, wherein selection is made based on the presence or absence of a reported signal response, and these cells comprise eTPC-t, wherein selected cells may comprise a single cell, a pool of cells of the same identity, a pool of cells of different identities **(****Figure 1** **step v).**

When the two-part cellular device is operated, the selection of analyte cell populations to prepare and combine into an eAPC:eTPC system is determined by the nature of analyte that each of the eAPC and eTPC populations present.

An eAPC-p is defined as expressing an antigen-presenting complex (aAPX) on the cell surface. An aAPX may have a cargo molecule (CM) loaded as antigen cargo. An aAPX loaded with antigen cargo is defined as an aAPX:CM complex.

An eAPC-a is defined as expressing an analyte antigen molecule (aAM). Such an aAM may be presented on the cell surface, and/or expressed or processed intracellularly such that it represents a cargo aAM that may be loaded into an aAPX.

An eAPC-pa is defined as expressing both an aAPX and an aAM. The aAM may be expressed or processed intracellularly such that it represents a cargo aAM that may be loaded into an aAPX. eAPC-pa thus expresses an aAPX at the cell surface, and may also express aAM loaded as cargo into said aAPX, which is defined as an aAPX:aAM complex. It is possible for an eAPC-pa does not load aAM as cargo into an aAPX to form the aAPX:aAM complex. Moreover, due to the nature of biological systems, it is unavoidable that aAPX:CM complexes are also present in the aAPC-pa.

An eTPC-t is defined as possessing an analyte pair of TCR chains that are expressed as TCR surface proteins in complex with CD3 (TCRsp), wherein CD3 represents the surface complex in which a TCR heterodimeric pair is presented and is defined as possessing ε, γ, δ and ζ subunits that associate with pair of complementary TCR chains as three dimers (εγ, εδ, ζζ). The expression of CD3 and a pair of complementary TCR chains is required for presentation at the cell surface, thus, the absence of expression of either TCR or CD3 will prevent the reciprocal from presenting on the cell surface. In the preparation of an eTPC-t, the expression of an αβ or γδ TCR pair and/or CD3 is used as a positive selection. Therefore, regardless of the nature of the TCR α, β, γ or δ chain combinations used to prepare the eTPC-t, only cells with a pair of complementary TCR chains presented on the surface of the cell in complex with CD3 are designated eTPC-t.

The above primary outputs all represent cell populations that are derived from a selection made upon their reported response in the combined eAPC:eTPC system. Each analyte eAPC presents a set of potential analyte antigens, intrinsic cargo molecules and/or analyte antigen presenting complexes, whereas the eTPC presents analyte TCRsp. It is from the selected cells that the selected analyte molecules may be obtained as terminal device outputs **(****Figure 1****, step vi).**

An eAPC-p is defined as presenting an aAPX on the cell surface, and may present aAPX:CM complex at the cell surface. Thus an eAPC-p that is selected from the combined eAPC:eTPC system based on a reported response, may be used to determine aAPX, CM and/or a aAPC:CM terminal device outputs, wherein these output identities have been selected on the ability of the determined analyte antigen to form a complex with, and/or report a signal response stimulated by, an analyte TCRsp presented by the analyte eTPC-t contacted with the selected eAPC-p in the combined eAPC:eTPC system.

An eAPC-a is defined as presenting an aAM on the cell surface or intracellularly. Thus an eAPC-a that is selected from the combined eAPC:eTPC system based on a reported response, may be used to determine an aAM terminal device output, wherein this output identity is selected on the ability of the determined analyte to form a complex with, and/or report a signal response stimulated by, an analyte TCRsp presented by the analyte eTPC-t contacted with the selected eAPC-a in the combined eAPC:eTPC system.

An eAPC-pa is defined as presenting an aAPX, aAM, aAPX, aAPX:aM, or an aAPX:CM at the cell surface. Thus, an eAPC-pa that is selected from the combined eAPC:eTPC system based on a reported response, may be used to determine aAM, aAPX, aAPX:aM and/or aAPX:CM terminal device outputs, wherein this output identity is selected on the ability of the determined analyte to form a complex with, and/or report a signal response stimulated by, an analyte TCRsp presented by the analyte eTPC-t contacted with the selected eAPC-pa in the combined eAPC:eTPC system.

As addressed above, selection of analyte eAPC may be made on basis of a reported response in a contacting eTPC. Therefore, any of eAPC-p, eAPC-a and eAPC-pa may be used to indirectly obtain the TCRsp output, if the TCRsp input(s) to the eTPCS are known prior to preparation of the analyte eTPC-t.

In the present context, an eTPC-t is defined as presenting TCRsp. Thus an eTPC-t that is selected from the combined eAPC:eTPC system based on a reported response, may be used to determine TCRsp terminal device outputs, wherein this output identity is selected on the ability of the determined analyte TCRsp to form a complex with, and/or report a signal response stimulated by, an analyte antigen presented by the analyte eAPC contacted with the selected eTPC in the combined eAPC:eTPC system.

As addressed above, selection of analyte eTPC may be made on basis of a reported response in a contacting eAPC. Therefore, an eTPC may be used to indirectly obtain any of the analyte antigen outputs, aAM, aAPX, aAPX:aM and/or aAPX:CM, if those analyte antigen input(s) to the eAPCS are known prior to preparation of the analyte eAPC.

### Description of the eAPCS

As mentioned above, the present invention relates to the provision of a two-part cellular device, wherein each part is an engineered cellular system. The first engineered cell system is an engineered multicomponent eAPCS that is used to prepare analyte eAPC for combination into a two-part eACP:eTPC system within the device.

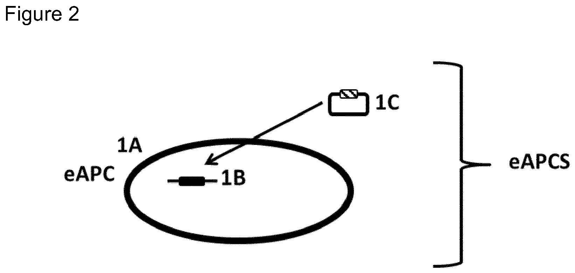

The minimal form of eAPCS is a multicomponent system wherein a first component is an **eAPC,** designated **component 1A,** containing a second component as a genomic receiver site **component 1B,** and a third component as a genetic donor vector, **designated component 1C (****Figure 2****).**

An eAPC represents the base component of the eAPCS, to which all other components of the system relate. Therefore, the eAPC contains certain features, that are native or engineered, that make the eAPC suitable for use in both the eAPCS and the combined two-part device.

In the present context the eAPC, **component 1A**
i. Lacks endogenous surface expression of at least one family of aAPX and/or aAM and
ii. Contains at least one genomic receiver site, **designated component 1B**
wherein i) may be obtained by selection of a naturally occurring cell population lacking said expression of aAPX and/or aAM, or may be engineered to lack such expression, and ii) which is synthetic and which may be introduced by means of directed or undirected genome integration.

The selection of an eAPC cell candidate that lacks desired aAPX and/or aAM expression from naturally occurring cell populations can be achieved by methods well known in the art. This may be directly achieved by staining of target cells with affinity reagents specifically for the aAPX and/or aAM that are desired to be lacking from the eAPC, and selection of cells lacking target aAPX and/or aAM expression.

Engineering of cells to lack aAPX and/or aAM expression may be achieved by untargeted and targeted means. Untargeted mutagenesis of the cell can be achieved by providing a chemical, radiological or other mutagen to the cell, and then selecting cells lacking target aAPX and/or aAM expression. Targeted mutation of the genomic loci can be achieved via different means, including but not limited to site directed mutagenesis via
i. zinc-finger nucleases
ii. CRISPR/Cas9 mediated targeting
iii. Synthetic transcription activator-like effector nucleases (TALEN)
wherein said site-directed nucleases induce site-specific DNA-repair error mutagenesis (also known as non-homologous end-joining) at target loci, after which mutated cells are obtained by selecting cells lacking target aAPX and/or aAM expression.

The component 1A, eAPC, may optionally include additional T-cell co-stimulation receptors, wherein such features permit robust or varying forms of communication of the analyte eAPC to the analyte eTPC, wherein the tuneable communication is relevant to identification or characterisation of specific analyte TCRsp and/or analyte antigens. In the present context, different forms of CD28 ligation on the eTPC can be promoted by inclusion of one or more of CD80, CD86 and/or further B7 family proteins.

The component 1A ,eAPC, may optionally additionally include introduced cell surface adhesion molecule components, or ablation of endogenous cell surface adhesion molecules, to promote the eAPC engagement with analyte eTPC and formation of the immunological synapse, or to avoid tight binding and formation of deleterious cell clustering within the combined eAPC:eTPC system, respectively. Such adhesion molecules that may be introduced as additional ORFs to component 1A, or genetically ablated from 1A, can be selected from the integrin family of adhesion proteins.

An eAPC may optionally possesses the ability to process and load antigen as cargo into aAPX by native processing and loading machinery. An eAPC that possesses the ability to process and load antigen as cargo into aAPX by native processing and loading machinery, will also process and load cargo molecules (CM) that are intrinsic to the eAPC or the culture system in which it is contain, wherein aAPX that is loaded with a CM is designated as an aAPX:CM complex.

The second component of the minimal multicomponent eAPCS is a genetic donor vector, **component 1C,** which is used for integration of at least one ORF encoding at least one aAPX and/or aAM **(****Figure 2****).**

Component **1C** is a genetic donor vector that is coupled with the genomic receiver site of Component **1B** contained within the genome of the eAPC, **Component 1A. Component 1C** is designed for the integration of one or more ORFs encoding an aAPX and/or an aAM, encoded in the genetic donor vector, into the genomic receiver site, **1B,** wherein integration results in the expression of aAPX and/or an aAM by the target eAPC.

In the present context, a paired genetic donor vector and genomic receiver site is described as an integration couple.

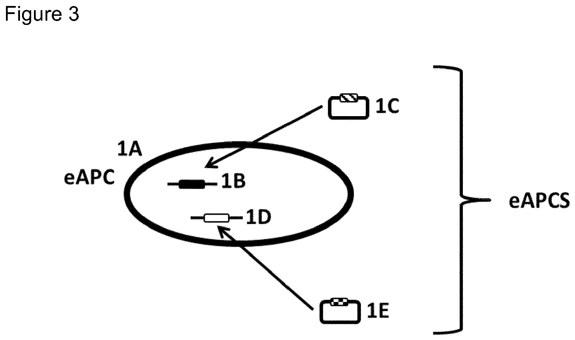

In an expanded form or the multicomponent eAPCS, the component **1A** eAPC may further contain a second genomic receiver site, designated component **1D,** which is coupled to a second genomic donor vector, designated component **1E,** that is also added to the system **(****Figure 3****).**

A multicomponent eAPCS may further comprise one or more additional integration couples.

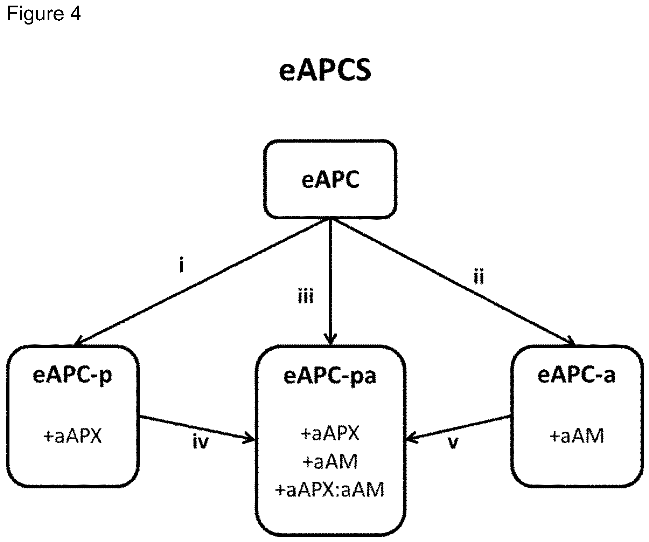

A multicomponent eAPCS, comprising an eAPC and either one or two integration couples, is used for preparation of the derivative eAPC forms
i. eAPC-p
ii. eAPC-a
iii. eAPC-pa
wherein each genetic donor vector may contain one or more ORFs encoding one or more aAPX and/or an aAM, to integrate said ORFs into the coupled genomic receiver sites, such that i) expresses at least one aAPX, ii) expresses at least one aAM and iii) expresses at least one aAPX and at least one aAM **(****Figure 4****).**

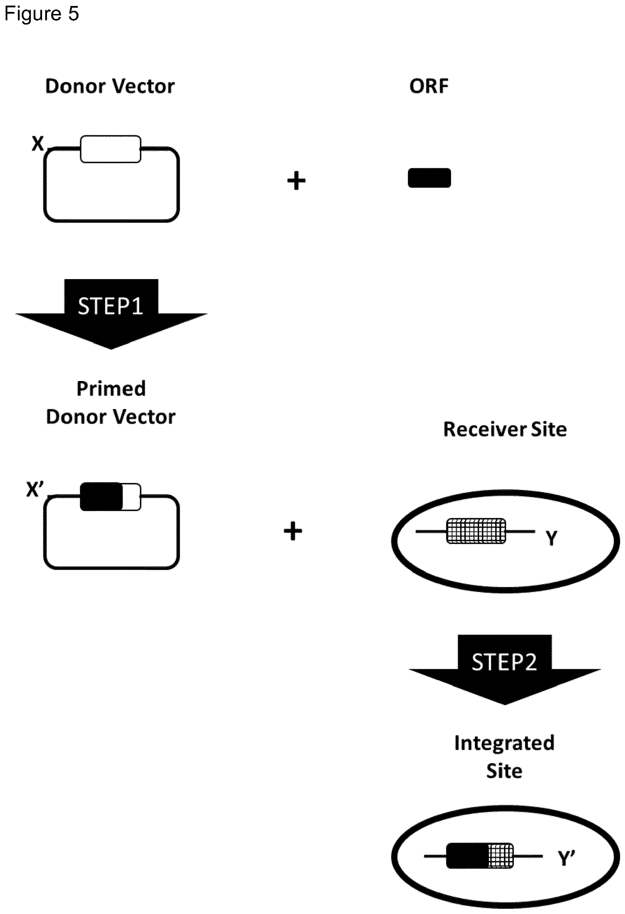

The genetic donor vector and genomic receiver sites operate as an integration couple subsystem of the eAPCS. A genetic donor vector must first be combined with target ORFs, such that base donor vector now encodes those target ORFs. The assembled primed donor vector is then introduced to the target eAPC to exchange target ORF(s) to the genomic receiver site, thus integrating the target ORFs to the coupled receiver site of the target cell **(****Figure 5****).**

A multicomponent eAPCS that comprises genetic donor vectors component 1C and/or 1E is combined with at least one ORF encoding at least one aAPX and/or aAM to obtain component 1C' and/or 1E', wherein the combination is defined as the ligation of genetic material into the correct coding frame(s), and in the correct orientation(s), of the genetic donor vector.

The combination of one or more ORFs into genetic donor vectors 1C and/or 1E may be performed multiple times with a library of unique ORFs as
i. single discrete reactions to obtain a discrete library of 1C' and/or 1E' vectors encoding multiple ORFs
ii. a singe reaction to obtain a pooled library of 1C' and/or 1E' vectors encoding multiple ORFs
wherein the discrete a discrete library may be combined with component 1A multiple times as to obtain a discrete library of eAPCs with unique ORFs encoding unique aAPX and/or aAM, or a pooled library may be combined with component 1A as a single event as to obtain a pooled library of eAPCs each with unique ORFs encoding unique aAPX and/or aAM.

The efficient integration of a predictable copy number of one or more ORFs into the genomic receiver site is highly advantageous for operation of a standardised eAPC, where analyte eAPC populations may be rapidly prepared and characterised. Thus, the genomic receiver site(s) and coupled donor vector(s) are critical to the function of the eAPC. Furthermore, it is strongly desirable to have an eAPC wherein component 1B and 1D, are insulated from one another, such that the donor vector component 1C cannot integrate at component 1B, and vice versa. In addition, it is also desirable that the component 1B and/or component 1D are amenable to a method of preparation of an eAPC wherein, the introduction of an analyte antigen is rapid, repeatable, with a high likelihood of correct integration and delivery of the desired number of copies of aAPX and/or aAM.

The genomic receiver site may be selected from the following
i. A synthetic construct designed for recombinase mediated cassette exchange (RMCE)
ii. A synthetic construct designed for site directed homologous recombination
iii. A native genomic site for site directed homologous recombination
wherein i) is preferred. The RMCE method may employ selected heterospecific sites that are specific for individual recombinase enzymes, such that each component 1B and 1D possess insulated specificity.

In the present context the genomic receiver site, component 1B and/or component 1D comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence.

The preferred genomic receiver site would comprise of two different arrangements using the following selected elements from the previously stated list of element. The first arrangement is for receiving a single ORF encoding one or more aAPX and/or one or more aAM chains and/or a selection mark of integration, via RMCE integration wherein the arrangement is
5' -[A] [B] [C] [D] [E] [F]- 3'
wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker
E) is element i) heterospecific recombinase site 2
F) is element v) Eukaryotic terminator.

The second arrangement is for receiving two ORF encoding one or more aAPX and/or one or more aAM and/or a selection marker of integration, via RMCE integration wherein the arrangement is
5' -[A] [B] [C] [D] [E] [F] [G] [H] [I]- 3'
wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker 1
E) is element v) a Eukaryotic bidirectional transcriptional terminator
F) is element vi) a FACS and/or MACS compatible encoded protein marker 2
G) is element xv) a Kozak consensus sequence
H) is element i) heterospecific recombinase site 2
I) is element iii) a constitutive or inducible Eukaryotic promoter
furthermore, in this second arrangement the elements F, G, and I are encoded in the antisense direction.

**Component 1C and/or 1E** comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence
xvi. Selection marker of integration
xvii. An antibiotic resistance cassette
xviii. A bacterial origin of replication
xix. A yeast origin of replication
xx. A cloning site

In a preferred embodiment of the genetic donor vector, component 1C and/or component 1E, would comprise of two different possible arrangements using the following selected elements from the previously stated list of elements.

The first arrangement is for delivery of a single ORF encoding one or more aAPX and/or one or more aAM and/or a selection mark of integration, via RMCE integration wherein the arrangement is
5' - [A] [B] [C] [D] [E] - 3'
wherein
A) is element i) heterospecific recombinase site 1
B) is element xv) a Kozak consensus sequence
C) is element xx) a cloning site of a single ORF encoding one or more aAPX and/or one or more aAM and/or element xvi) a selection marker of integration
D) is element i) heterospecific recombinase site 2
E) is element xvii) An antibiotic resistance cassette and element xviii) a bacterial origin of replication, in no specific orientation
furthermore, the elements viii and/or xiv may be used to link multiple aAPX and/or one or more aAM and/or element xvi together.

The second arrangement is for delivery of two ORF encoding one or more aAPX and/or aAM and/or a selection mark of integration, via RMCE integration wherein the arrangement is
5' - [A] [B] [C] [D] [E] [F]- 3'
wherein
A) is element i) heterospecific recombinase site 1
B) is element xv) a Kozak consensus sequence
C) is element xx) a cloning site for introduction of two or more ORF, with eukaryotic terminators, encoding one or more aAPX and/or one or more aAM and/or element xvi) a selection marker of integration
D) is element xv) a Kozak consensus sequence (antisense direction)
E) is element i) heterospecific recombinase site 2
F) is element xvii) An antibiotic resistance cassette and element xviii) a bacterial origin of replication, in no specific orientation
furthermore, the elements viii and/or xiv may be used to link multiple aAPX and/or aAM and/or element xvi together within each ORF.

### Preparing analyte eAPC populations in the eAPCS

The above described eAPCS system may be used in multiple ways to prepare distinct forms of analyte eAPC, or libraries thereof, that serve to present analyte aAPX, aAM, aAPX:aAM and aAPX:CM to the eTPC within the combined eAPC:eTPC system in operation of the two-part device.

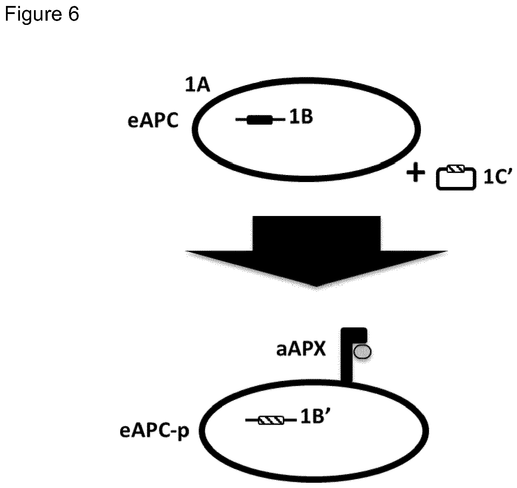

An eAPCS comprising a single integration couple may be used to prepare an eAPC-p from component 1A in one step, by providing component 1C' combined with an ORF for an aAPX, such that this aAPX is integrated to site 1B, to create 1B'. The resulting cell line expresses the provided aAPX, and it is presented at the cell surface **(****Figure** 6).

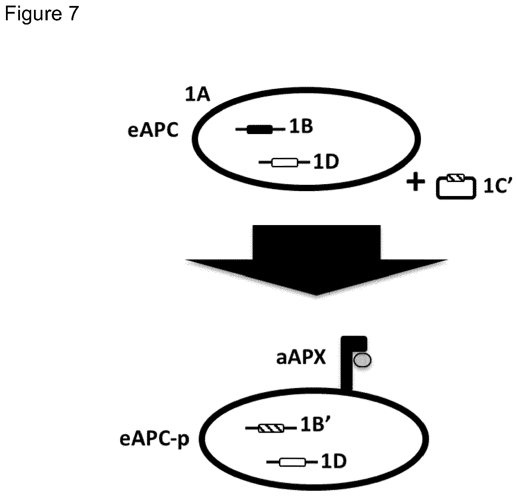

An eAPCS comprising two integration couples may be used to prepare an eAPC-p from component 1A in one step, by providing component 1C' combined with an ORF for an aAPX, such that this aAPX is integrated to site 1B, to create 1B'. The resulting cell line expresses the provided aAPX, and it is presented at the cell surface. The second integration couple 1D/1E remains unmodified and may be used for downstream integration steps **(****Figure 7****).**

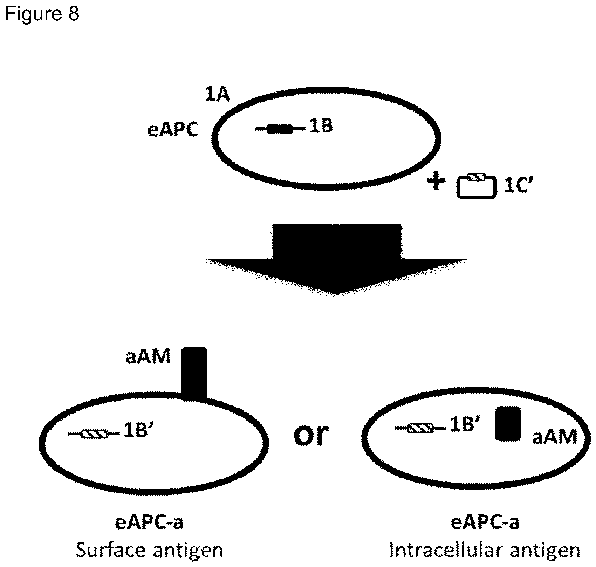

An eAPCS comprising a single integration couple may be used to prepare an eAPC-a from component 1A in one step, by providing component 1C' combined with an ORF for an aAM, such that this aAM is integrated to site 1B, to create 1B'. The resulting cell line expresses the provided aAM, and is presented either at the cell surface or retained intracellularly **(****Figure 8****).**

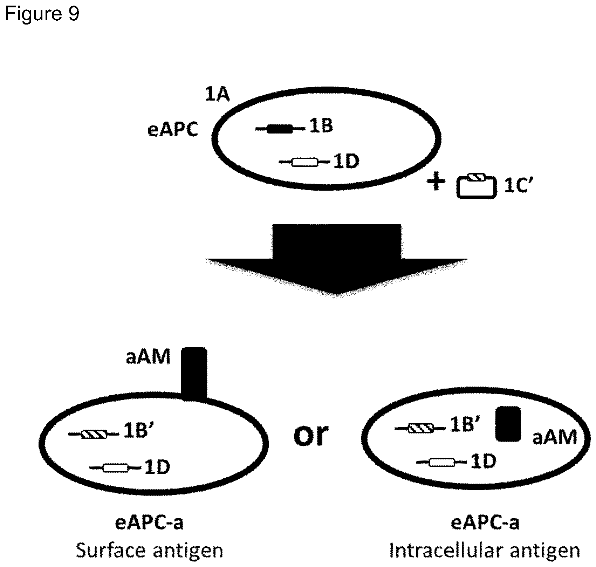

An eAPCS comprising two integration couples may be used to prepare an eAPC-a from component 1A in one step, by providing component 1C' combined with an ORF for an aAM, such that this aAM is integrated to site 1B, to create 1B'. The resulting cell line expresses the provided aAM, and is presented either at the cell surface or retained intracellularly. The second integration couple 1D/1E remains unmodified and may be used for downstream integration steps **(****Figure 9****).**

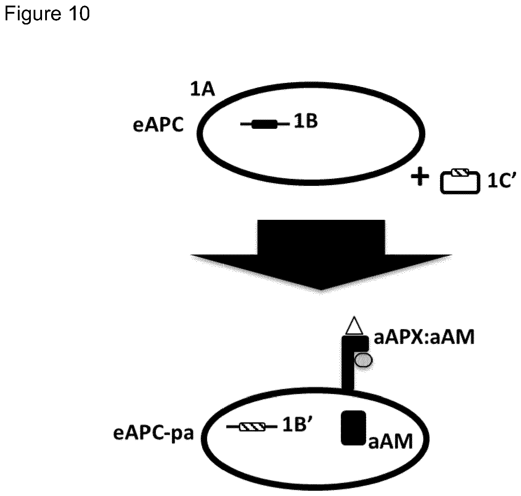

An eAPCS comprising a single integration couple may be used to prepare an eAPC-pa from component 1A in one step, by providing component 1C' combined with two ORFs, one encoding and aAPX and the other an aAM, such that both aAPX and aAM are integrated to site 1B, to create 1B'. The resulting cell line expresses the provided aAPX and aAM, and may present an aAPX:aAM at the cell surface **(****Figure 10****).**

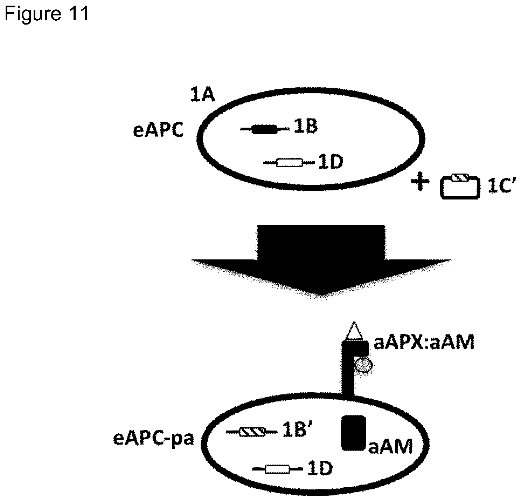

An eAPCS comprising two integration couples may be used to prepare an eAPC-pa from component 1A in one step, by providing component 1C' combined with two ORFs, one encoding and aAPX and the other an aAM, such that both aAPX and aAM are integrated to site 1B, to create 1B'. The resulting cell line expresses the provided aAPX and aAM, and may present an aAPX:aAM at the cell surface. The second integration couple 1D/1E remains unmodified and may be used for downstream integration steps **(****Figure 11****).**

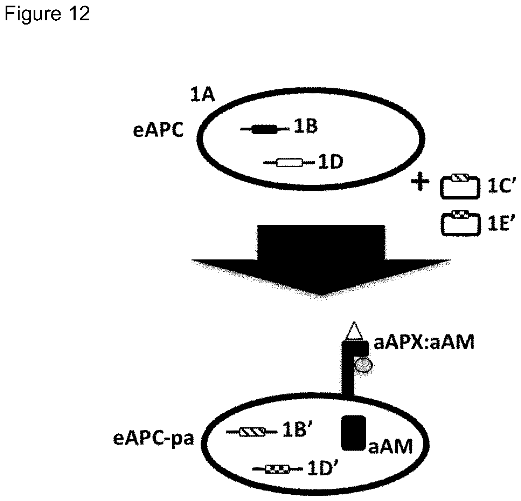

An eAPCS comprising two integration couples may be used to prepare an eAPC-pa from component 1A in one step, by providing component 1C' and 1E' each combined with one ORF encoding either an aAPX or an aAM, such that both aAPX and aAM are integrated to site 1B or 1D, to create 1B' and 1D'. The resulting cell line expresses the provided aAPX and aAM, and may present an aAPX:aAM at the cell surface **(****Figure 12****).**

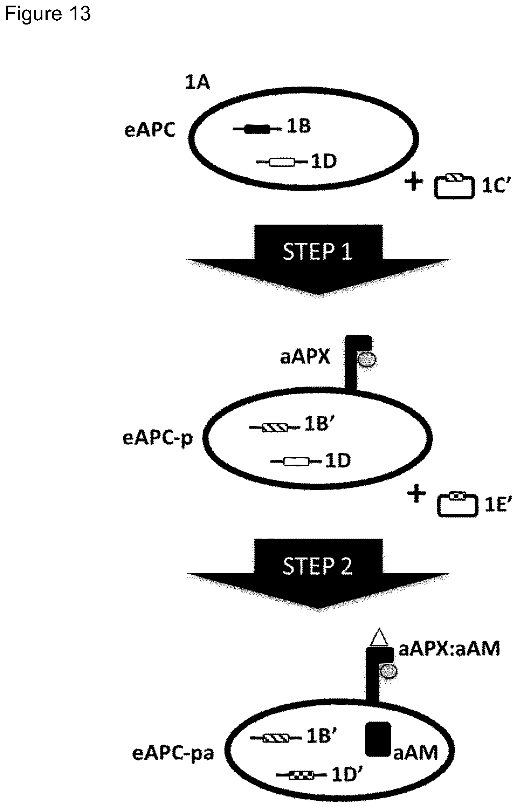

An eAPCS comprising two integration couples may be used to prepare an eAPC-pa from component 1A in two steps, by first providing component 1C' combined with an ORF encoding an aAPX such that this aAPX is integrated to site 1B, to create 1B'. The resulting cell line expresses the provided aAPX, and it is presented at the cell surface. The second integration couple 1D/1E remains unmodified. In the second step 1E' is provided wherein the donor vector is combined with an ORF encoding an aAM such that this aAM is integrated to site 1E, to create 1E'. The resulting cell line expresses the provided aAM, and this may be processed and loaded as cargo in the aAPX to form an aAPX:aAM complex on the cell surface **(****Figure 13****).**

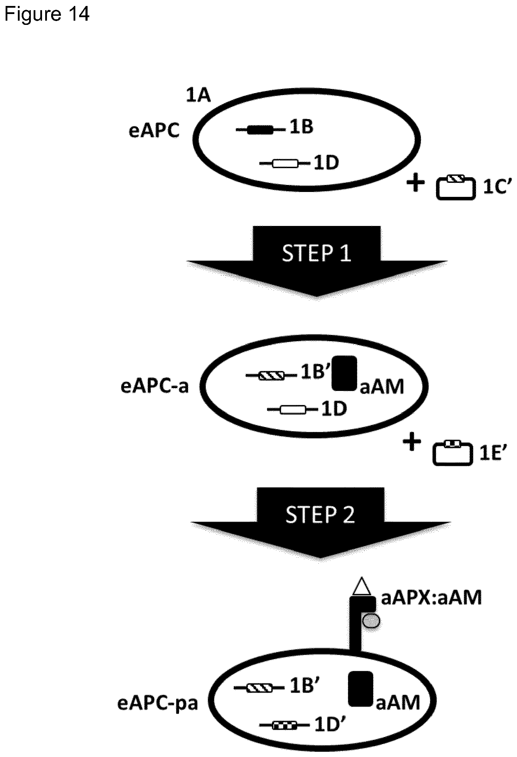

An eAPCS comprising two integration couples may be used to prepare an eAPC-pa from component 1A in two steps, by first providing component 1C' combined with an ORF encoding an aAM such that this aAM is integrated to site 1B, to create 1B'. The resulting cell line expresses the provided aAM (eAPC-a intermediate). The second integration couple 1D/1E remains unmodified. In the second step 1E' is provided wherein the donor vector is combined with an ORF encoding an aAPX such that this aAPX is integrated to site 1E, to create 1E'. The resulting cell line expresses the provided aAPX, which is presented on the cell surface. The aAM integrated in the first step may be processed and loaded as cargo in the aAPX to form an aAPX:aAM complex on the cell surface **(****Figure 14****).**

In the abovementioned examples of preparing analyte eAPC-p, eAPC-a and eAPC-pa populations from eAPC, the eAPCS system is used to provide known aAPX and aAM candidates in a defined manner to prepare discrete populations of analyte eAPC expressing defined aAPX and/or aAM. Such a process may be repeated many times to build libraries of eAPC-p, eAPC-a and eAPC-pa to provide to the combined eAPC:eTPC system in operation of the device. An alternative approach is to take pooled libraries of candidate aAPX and/or aAM ORFs combined with genetic donor vectors, and integrate these in a single reaction to obtain pooled libraries of analyte eAPC-p, eAPC-a or eAPC-pa that express multiple aAPX, aAM and/or aAPX:aAM. This process of converting a pool of vectors to a pool of eAPC-p, -a, and/or -pa will be referred to as **shotgun integration.** This is particularly useful when analysing large libraries of candidate aAM against a fixed aAPX, or vice versa.

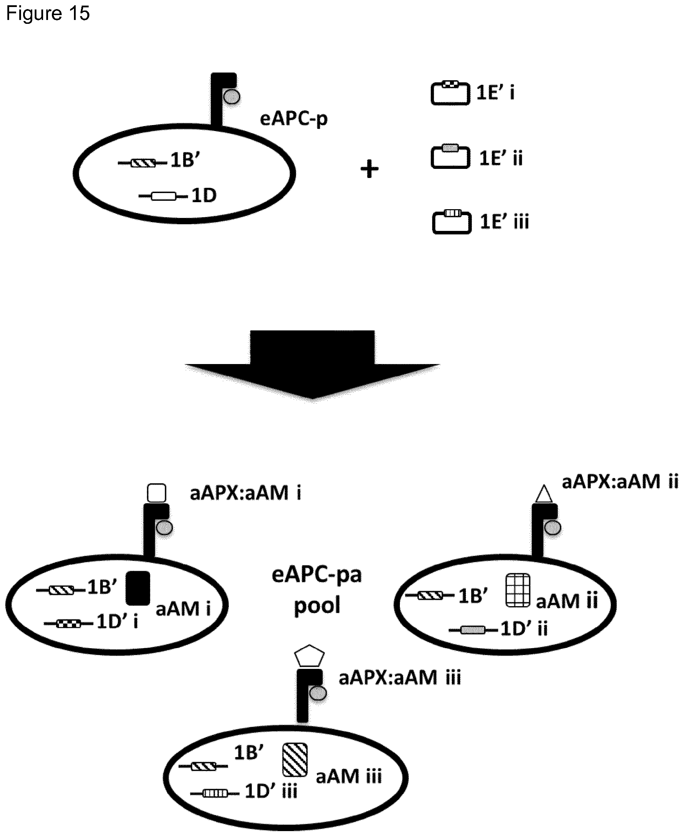

An eAPCS comprising two integration couples may be used to prepare an eAPC-pa from component 1A in two steps, by first providing component 1C' combined with an ORF encoding an aAPX such that this aAPX is integrated to site 1B, to create 1B'. The resulting cell line expresses the provided aAPX on the cell surface (eAPC-p inermedaite). The second integration couple 1D/1E remains unmodified. In the second step a library of multiple 1E' is provided wherein the library of donor vectors comprises a pool of vectors each combined with a single ORF encoding an aAM such that each aAM is integrated to site 1E, to create 1E', within single cells. The resulting pool of cells contains a collection of cells, wherein each cell has integrated a single random aAM ORF from the original pool of vectors. The aAM integrated in the second step may be processed and loaded as cargo in the aAPX integrated in the first step to form an aAPX:aAM complex on the cell surface **(****Figure 15****).**

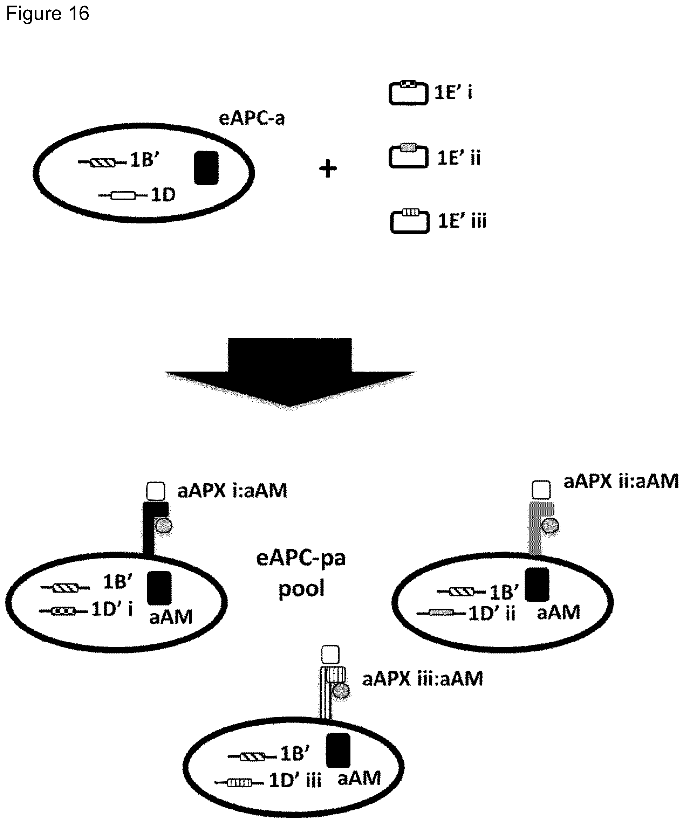

An eAPCS comprising two integration couples may be used to prepare an eAPC-pa from component 1A in two steps, by first providing component 1C' combined with an ORF encoding an aAM such that this aAM is integrated to site 1B, to create 1B'. The resulting cell line expresses the provided aAM (eAPC-a intermediate). The second integration couple 1D/1E remains unmodified. In the second step a library of multiple 1 E' is provided wherein the library of donor vectors comprises a pool of vectors each combined with a single ORF encoding an aAPX such that each aAPX is integrated to site 1E, to create 1E', within single cells. The resulting pool of cells contains a collection of cells, wherein each cell has integrated a single random aAPX ORF from the original pool of vectors. The aAM integrated in the first step may be processed and loaded as cargo in the aAPX integrated in the second step to form an aAPX:aAM complex on the cell surface **(****Figure 16****).**

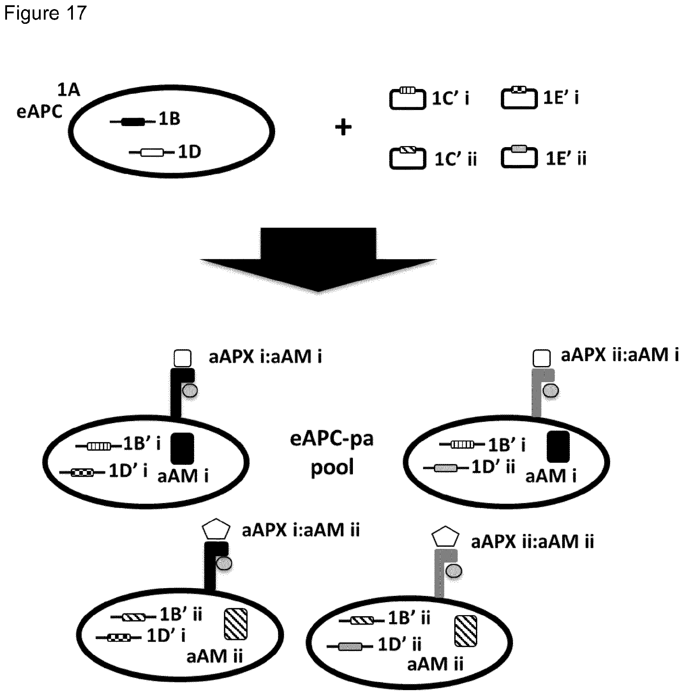

An eAPCS comprising two integration couples may be used to prepare an eAPC-pa from component 1A in one steps, by providing component 1C' and 1E' each combined with a library of ORFs encoding either a library of aAPX or a library of aAM, such that both aAPX and aAM are integrated to site 1B or 1D, to create 1B' and 1D'. The resulting pool of cells contains a collection of cells wherein each cell has integrated a single random aAPX ORF and a single random aAM ORF from the original pool of vectors. Within each cell in the pooled library, an integrated aAM may be processed and loaded as cargo in the aAPX integrated into the same cell to form an aAPX:aAM complex on the cell surface. Such a pooled library would contain all possible combinations of aAPX:aAM from the set of aAPX and aAM provided **(****Figure 17****).**

In the above-mentioned shotgun integration methods for providing pooled libraries of eAPC-pa, the robustness of the system relies on a single copy of the genomic receiver site. This is to ensure just a single analyte may be introduced into each cell via the integration couple. This single-copy genomic receiver site is an optional aspect of an eAPCS, as multiple copies of the same genomic receiver25 site may be beneficial in providing integration steps where multiple 'alleles' from a library of provided vectors may be obtained in the prepared eAPC.

In the present context, an aAPX may be selected from one of the following
i. One or more members of HLA class I
ii. One or more members of HLA class II
iii. One or more non-HLA antigen-presenting complex

An aAPX may be selected from one of the following
i. a polypeptide or complex of polypeptides provided as analyte antigen
ii. a peptide derived from a polypeptide provided as analyte antigen
iii. a peptide provided as analyte antigen
iv. a metabolite provided as analyte antigen
v. a polypeptide or complex of polypeptides translated from the analyte antigenic molecule ORF(s)
vi. a peptide derived from a polypeptide translated from the analyte antigenic molecule ORF(s)
vii. a peptide derived from altering the component A proteome
viii. a polypeptide derived from altering the component A proteome
ix. a metabolite derived from altering the component A metabolome

### Description of the eTPCS

As mentioned above, the present invention relates to the provision of a two-part cellular device, wherein each part is an engineered cellular system. The first engineered cell system is the eAPCS as described above. The second engineered cell system is an engineered multicomponent eTPCS that is used to prepare analyte eTPC for combination into a two-part eACP:eTPC system within the device.

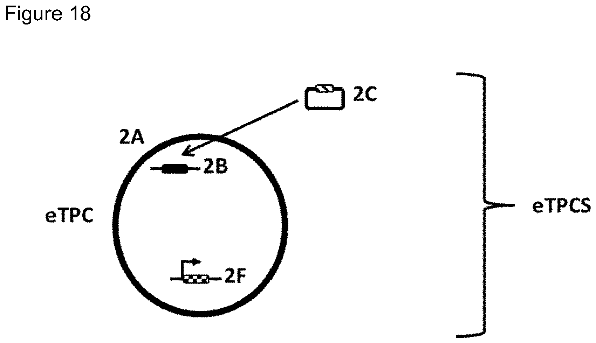

The minimal form of eTPCS is a multicomponent system wherein a first component is an **eTPC,** designated **component 2A,** and a second component is a genetic donor vector, **designated component 2C (****Figure 18****).**

An eTPC represents the base component of the eTPCS, to which all other components of the system relate. Therefore, the eTPC contains certain features, that are native or engineered, that make the eTPC suitable for use in both the eTPCS and the combined two-part device.

The eTPC, **component 2A,**
i. Lacks endogenous expression of TCR chains alpha, beta, delta and gamma, and
ii. Expresses CD3 proteins which are conditionally presented on the surface of the cell only when the cell expresses a complementary pair of TCR chains and
iii. Contains a further component **designated 2B,** a genomic receiver site for integration of a single ORF encoding at least one analyte TCR chain of alpha, beta, delta or gamma, and/or two ORFs encoding a pair of analyte TCR chains
wherein i may by obtained by selection of a naturally occurring cell population lacking said expression, or may be engineered to lack such expression; ii may by obtained by selection of a naturally occurring cell population comprising said expression, or may be engineered to comprise such expression; iii may be or introduced to the genome by means of genetic engineering.

The selection of an eTPC cell candidate that lacks TCR chains alpha, beta, delta and gamma from naturally occurring cell populations can be achieved by methods well known in the art. Staining of target cells with affinity reagents specifically for TCR chains alpha, beta, delta and gamma, and selection of cells TCR chains alpha, beta, delta and gamma may directly achieve this.

Engineering of eTPC to lack TCR chains alpha, beta, delta and gamma expression may be achieved by untargeted and targeted means. Untargeted mutagenesis of the cell can be achieved by providing a chemical, radiological or other mutagen to the cell, and then selecting cells lacking target aAPX and/or aAM expression. Targeted mutation of the genomic loci can be achieved via different means, including but not limited to site directed mutagenesis via
i. zinc-finger nucleases
ii. CRISPR/Cas9 mediated targeting (correct and acronym)
iii. Synthetic transcription activator-like effector nucleases (TALEN)
wherein said site-directed nucleases induce site-specific DNA-repair error mutagenesis at target loci, after which mutated cells are obtained by selecting cells lacking TCR alpha, beta, delta and gamma expression.

Options for Integration of CD3 and the components B and/or D are well known to those skilled in the art but may include homology directed recombination (HDR) and/or random integration methods, wherein HDR may be promoted by targeted mutation of the genomic loci at which HDR is to occur, and can be achieved via different means, including but not limited to site directed mutagenesis via
i. zinc-finger nucleases
ii. CRISPR/Cas9 mediated targeting
iii. Synthetic transcription activator-like effector nucleases (TALEN)
   wherein said site-directed nucleases induce site-specific DNA-repair by HDR at target loci. After such events, a proportion of cells will have incorporated HDR vector, an can be selected and/or determined via any combination of the following,
iv. Non-destructive phonotypical expression analysis
v. Destructive phonotypical expression analysis
vi. Genetic analysis
Wherein iv and vi are the preferred methods for selection and determination of successful genomic integration events.

Alternatively, viral vectors could be used to deliver the required components in a site-directed or undirected manner.

Considering that the eTPC component 2A is designed to be used in conjunction with the above described eAPC component 1A, within a combined eAPC:eTPC system, for the purpose of analyses of TCR and T-cell antigen interactions (Figure 1), in the preferred aspect the eTPC contains features that minimise the eTPC presenting factors that would interfere in such analyses.

The eTPC component 2A optionally lacks endogenous surface expression of at least one family of **aAPX** and/or **aAM,** wherein the lack of surface expression is selected as to minimise interference in matched analyses of target analyte antigens.

The family of **aAPX** may be any of the following
i. HLA class I
ii. HLA class II
iii. non-HLA antigen-presenting complex.

An **aAM** is selected from
i. a polypeptide or complex of polypeptides translated from the analyte antigenic molecule ORF(s)
ii. a peptide derived from a polypeptide translated from the analyte antigenic molecule ORF(s)
iii. a peptide derived from altering the component A proteome
iv. a polypeptide derived from altering the component A proteome
v. a metabolite derived from altering the component A metabolome

The component 2A eTPC may optionally additionally include T-cell co-receptors, wherein such features permit robust or varying forms of communication of the analyte eTPC to the analyte eAPC, wherein the tuneable communication is relevant to identification or characterisation of specific analyte TCRsp and/or analyte antigens.

The eTPC component 2**A** may optionally express CD4 and/or CD8, wherein expression of CD4 or CD8 restrict eTPC to engaging aAPX of type HLAII and HLAI, respectively.

In the present context, the eTPC component 2**A** may optionally expresses CD28 and/or CD45, wherein CD28 and CD45 contribute to signal sensitivity through positive feed forward effects on signalling, whereas they may also contribute to signal specificity through negatie feed back effects on signalling, as it relates to signalling though an expressed analyte TCRsp.

The component 2A eTPC may optionally additionally include introduced cell surface adhesion molecule components, or ablation of endogenous cell surface adhesion molecules, to promote the eTPC engagement with analyte eAPC and formation of the immunological synapse, or to avoid tight binding and formation of deleterious cell clustering within the combined eAPC:eTPC system, respectively.

Such adhesion molecules that may be introduced as additional ORFs to component 2A, or genetically ablated from 2A, can be selected from the integrin family of adhesion proteins.

The second component of the minimal multicomponent is a genetic donor vector, **component 2C,** which is used for integration of at least one ORF encoding at least one analyte TCR chain **(****Figure 18****).**

Component **2C** is a genetic donor vector that is coupled with the genomic receiver site of the, **2B,** contained within the genome of the eTPC, **Component 2A. Component 2C** is designed for the integration of one or more ORFs encoding an analyte TCR chain, encoded in the genetic donor vector, into the genomic receiver site, **2B,** wherein integration results in the expression of analyte TCR chains by the target eTPC.

A paired genetic donor vector and genomic receiver site is described as an integration couple.

An eTPC is designed to respond to stimulation by cognate antigen, as presented by the eAPC within the combined eAPC:eTCP system. It is thus desirable to have a standardised reporter readout for signalling response from stimulation of the expressed TCRsp.

In the present context, the eTPC component 2**A**, further contains a component **designated 2F,** a synthetic genomic TCR-stimulation response element selected from
i. A single component synthetic construct containing at least one native promoter and/or at least one synthetic promoter and at least one reporter
ii. A multi-component synthetic construct designed with at least one native promoter and/or at least one synthetic promoter and at least one reporter
wherein activation of i and/or ii is dependent on at least one signal transduction pathway selected from a synthetic pathway, a native pathway or a combination thereof.

A eTPC is designed to assay engagement of the presented TCRsp with a analyte antigen. The readout of engagement may be achieved through induction of a synthetic reporter element that responds to TCRsp engagement. Therefore, the eTPC, may further contain a component **designated 2F,** a synthetic genomic TCR-stimulation response element selected from
i. A single component synthetic construct containing at least one native promoter and/or at least one synthetic promoter and at least one reporter
ii. A multi-component synthetic construct designed with at least one native promoter and/or at least one synthetic promoter and at least one reporter
wherein activation of i and/or ii is dependent on at least one signal transduction pathway selected from a synthetic pathway, a native pathway or a combination thereof.

Synthetic TCR-stimulation response elements **(component 2F),** with synthetic as opposed to native promoters, are less likely to mimic a natural TCR stimulation response and thus represent a more distant approximation to natural T-cell stimulation. However, synthetic promoters provide greater flexibility for tuning and adjustment of the reporter response for the robust identification of eTPC-t presenting TCRsp that productively engage analyte antigen.

Single component synthetic constructs provide limited space for amplification or modulation of the signal reporter response, but are more straightforward to implement and predict the outcome. Multi-component constructs have the advantage of having a virtually unlimited space to construct complex response networks, however, this comes with the cost of being more difficult to implement and predict. A network also provides instances to initiate feedback loops, repression and activation of secondary response reporters.

Synthetic promoters can be linked to artificial synthetic signalling components, which can be either downstream of the initial natural TCR signalling pathway or substitute it. In such an instance, the CD3 complex can be coupled in-part or wholly to an artificial synthetic signalling pathway and response network. Such artificial pathways provide the advantage of being very modular and adaptable, for greater fine tuning or increased variety of responses.

The preferred embodiment utilises a multi-component synthetic construct with synthetic promoters and at least a partial synthetic signalling pathway. This provides the most robust means to ensure limited resting state signal response but with high coupled reporter signal when ligation of analyte antigen to the TCRsp occurs.

Regardless of the exact architecture of coupling the TCRsp stimulation to a component 2F the desired end outcome is a detectable reporter. In the preferred embodiment, a reporter that is amenable to fluorescent detection (i.e. FACS) and/or physical selection methods such as Magnetic Activated Cell Soting (MACS) is desired. Alternatively, the reporter could be a secreted or intracellular molecule for detection by spectrometric, fluorescent or luminescent assays, preferably in a non-destructive nature, wherein the populations can subsequently be selected based on the presence or absence of the reporter molecule. In addition, the response is not limited to a single reporter type but could be a combination of one or more different reporters.

In one context, a multi-component synthetic construct with synthetic promoters and a partial synthetic signalling pathway (Driver-Activator/Repressor + Amplifier-Reporter) would comprise of:
Driver-Activator/Repressor:
   a) a synthetic promoter
   b) a Kozak sequence
   c) a transcription factor,
   d) a first terminator
Amplifier-Reporter
   e) a second synthetic promoter
   f) a Kozak sequence
   g) a reporter
   h) a second terminator
wherein; a) Represents a synthetic promoter (Driver) that is designed to be coupled to the initial natural signalling pathway generated by TCRsp ligation to the analyte antigen. A minimum driver comprises of one or more transcription factor binding site and a core promoter, wherein the core promoter comprises, at a minimum, of a TATA Box, Initiator element (Inr) and transcriptional start site; b) Represents a Kozak sequence for efficient translational initiation of the transcription factor; c) Represents an ORF encoding a synthetic or natural transcription factor (Activator, or Repressor), which can bind to the DNA sequence of the second synthetic promoter; d) Represents a transcriptional terminator for efficient transcription and polyadenylation of the transcription factor mRNA transcript, and optional one or more untranslated 3' genetic element(s). These genetic elements may include, but are not limited to, transcript stabilizing or destabilizing elements, and unique identifier sequences; e) Represents a second synthetic promoter (Amplifier), which encodes one or more recognition sites for binding of the c) transcription factor and a core promoter, wherein the core promoter comprisess, at a minimum, of a TATA Box, Inr and transcriptional start site; f) Represents a Kozak sequence for efficient translational initiation of the reporter; g) Represents an ORF encoding a reporter protein; h) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences.

It is important to recognise that the Driver-Activator/Repressor coupled to Amplifier-Reporter paradigm can be extended to include additional Driver-Activator/Repressor and Amplifier-Reporter pairs and/or additional Amplifier-Reporter units. Furthermore, Activators can be replaced with Repressors to shutdown Amplifier-Reporter units and/or Driver-Activator/Repressor units, for negative selection methods and/or negative-feedback loops.

In a second context, a single-component synthetic construct with synthetic promoters (Driver-Reporter) would comprise of:
a) a synthetic promoter
b) a Kozak sequence
c) a reporter
d) a terminator
wherein; a) Represents a synthetic promoter (Driver) that is designed to be coupled to the initial natural signalling pathway generated by TCRsp ligation to the analyte antigen. A minimum driver comprises of one or more transcription factor binding site and a core promoter, wherein the core promoter comprises, at a minimum, of a TATA Box, Inr and transcriptional start site; b) Represents a Kozak sequence for efficient translational initiation of the reporter; c) Represents an ORF encoding a reporter protein; d) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences.

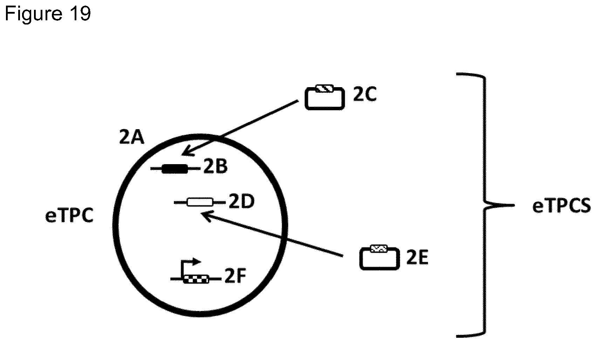

In an expanded form or the multicomponent eTPCS, the component **2A** eTPC may further contain a second genomic receiver site, designated component **2D,** which is coupled to a second genomic donor vector, designated component **2E,** that is also added to the system **(****Figure 19****).**

An eTPCS component 2A may further comprise one or more additional integration couples.

An eTPCS, comprising an eTPC and either one or two integration couples, is used for preparation of the eTPC-t used for assembly of the combined eAPC:eTPC system.

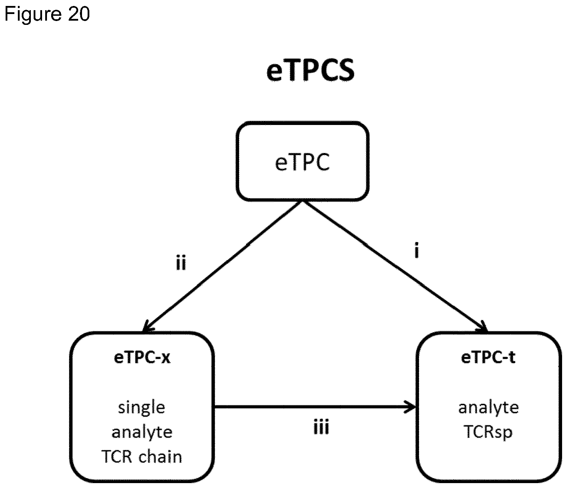

An eTPC-t may be prepared by integration of two complementary TCR chains to form a TCRsp, or may alternatively may be prepared by two steps by providing each complementary chain sequentially **(****Figure 20****).**

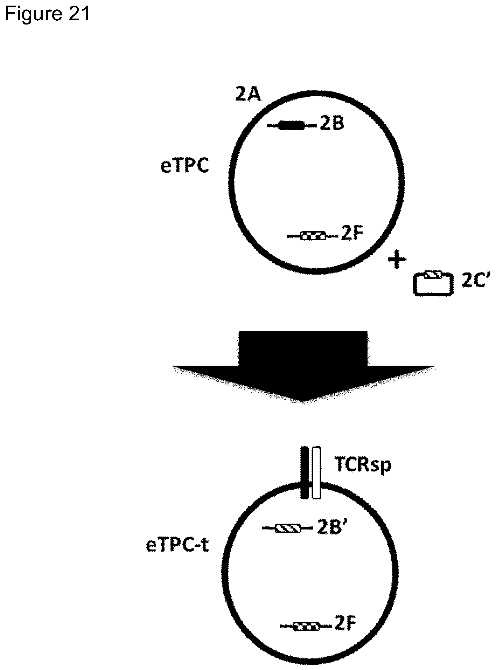

The genetic donor vector and genomic receiver sites operate as an integration couple subsystem of the eTPCS. A genetic donor vector must first be combined with target ORFs, such that base donor vector now encodes those target ORFs. The assembled primed donor vector is then introduced to the target eTPC to exchange target ORF(s) to the genomic receiver site, thus integrating the target ORFs to coupled receiver site of the target cell **(****Figure 21****).**

An eTPCS that comprises genetic donor vectors component 2C and/or 2E is combined with at least one ORF encoding at least one analyte TCR chain to obtain component 2C' and/or 2E', wherein the combination is defined as the ligation of genetic material into the correct coding frame(s), and in the correct orientation(s), of the genetic donor vector.

The combination of one or more ORFs into genetic donor vectors 2C and/or 2E may be performed multiple times with a library of unique ORFs as
i. single discrete reactions to obtain a discrete library of 2C' and/or 2E' vectors encoding multiple ORFs
ii. a singe reaction to obtain a pooled library of 2C' and/or 2E' vectors encoding multiple ORFs
wherein a discrete library may be combined with component 2A multiple times as to obtain a discrete library of eTPCs with unique ORFs encoding unique TCR chains, or a pooled library may be combined with component 2A one or more times as to obtain a pooled library of eTPC-t wherein each eTPC-t integrates a randomised ORFencoding analyte TCR chains derived from the original vector pool, such that each eTPC-t expresses a single random unique TCRsp

The efficient integration of a predictable copy number of one or more ORFs into the genomic receiver site is highly advantageous for operation of a standardised eTPC, where analyte eTPC populations may be rapidly prepared and characterised. Thus, the genomic receiver site(s) and coupled donor vector(s) are critical to the function of the eTPC. Furthermore, it is strongly desirable to have an eTPC wherein component 2B and 2D, are insulated from one another, such that the donor vector component 2C cannot integrate at component 2B, and vice versa. In addition, it is also desirable that the component 2B and/or component 2D are amenable to a method of preparation of an eTPC-t wherein, the introduction of a single pair of complementary TCR chains is rapid, repeatable, with a high likelihood of correct integration and delivery of only a single pair.

The genomic receiver site may be selected from the following
i. A synthetic construct designed for recombinase mediated cassette exchange (RMCE)
ii. A synthetic construct designed for site directed homologous recombination
wherein i) is the preferred form a genomic receiver site for RCME. The RMCE method may employ selected heterospecific sites that are specific for individual recombinase enzymes, such that each component 2B and 2D possess insulated specificity.

The genomic receiver site, component 2B and/or component 2D comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES) Viral self-cleaving peptide element
xiv. A kozak consensus sequence.

A preferred genomic receiver site would comprise of two different arrangements using the following selected elements from the previously stated list of element.

The first arrangement is for receiving a single ORF encoding one or more TCR chains and/or a selection mark of integration, via RMCE integration wherein the arrangement is
5' -[A] [B] [C] [D] [E] [F]- 3'
wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker
E) is element i) heterospecific recombinase site 2
F) is element v) Eukaryotic terminator

The second arrangement is for receiving a two ORF encoding one or more TCR chains and/or a selection mark of integration, via RMCE integration wherein the arrangement is
5' -[A] [B] [C] [D] [E] [F] [G] [H] [I]- 3'
wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker 1
E) is element v) a Eukaryotic bidirectional transcriptional terminator
F) is element vi) a FACS and/or MACS compatible encoded protein marker 2
G) is element xv) a Kozak consensus sequence
H) is element i) heterospecific recombinase site 2
I) is element iii) a constitutive or inducible Eukaryotic promoter
furthermore, in this second arrangement the elements F, G, and I are encoded in the antisense direction

**Component 2C and/or 2E** comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence
xvi. Selection marker of integration
xvii. An antibiotic resistance cassette
xviii. A bacterial origin of replication
xix. A yeast origin of replication
xx. A cloning site

A preferred genetic donor vector, component C and/or component E, would comprise of two different possible arrangements using the following selected elements from the previously stated list of elements.

The first arrangement is for receiving a single ORF encoding one or more TCR chains and/or a selection mark of integration, via RMCE integration wherein the arrangement is
5' - [A] [B] [C] [D] [E] - 3'
wherein
A) is element i) heterospecific recombinase site 1
B) is element xv) a Kozak consensus sequence
C) is element xx) a cloning site of a single ORF encoding one or more TCR chains and/or element xvi) a selection marker of integration
D) is element i) heterospecific recombinase site 2
E) is element xvii) An antibiotic resistance cassette and element xviii) a bacterial origin of replication, in no specific orientation
furthermore, the elements viii and/or xiv may be used to link multiple TCR chains and/or element xvi together.
5' - [A] [B] [C] [D] [E] [F]- 3'
wherein
A) is element i) heterospecific recombinase site 1
B) is element xv) a Kozak consensus sequence
C) is element xx) a cloning site for introduction of two or more ORF, with eukaryotic terminators, encoding one or more TCR chains and/or element xvi) a selection marker of integration
D) is element xv) a Kozak consensus sequence (antisense direction)
E) is element i) heterospecific recombinase site 2
F) is element xvii) An antibiotic resistance cassette and element xviii) a bacterial origin of replication, in no specific orientation
furthermore, the elements viii and/or xiv may be used to link multiple TCR chains and/or element xvi together within each ORF.

### Preparing analyte eTPC populations in the eTPCS

The above described eTPCS system may be used in multiple ways to prepare distinct forms of analyte eTPC populations, or libraries thereof, that serve to present analyte TCRsp to the eAPC within the combined eAPC:eTPC system in operation of the two-part device.

The eTPC-t populations that are created need to derive analyte TCR chains from certain sources with which to analyse candidate antigens.

The sources of analyte TCR chain encoding sequences can be derived from
i. Paired cloning of TCR chain ORF sequence(s) from primary T-cells
ii. Unpaired cloning of TCR chain ORF sequence(s) from primary T-cells
iii. Synthetic TCR chain ORF sequence(s)
wherein i is preferable for discovery of native TCRsp that are not likely to be generally cross reactive against self aAPX and/or the aAPX cargo due to thymic selection; ii may be used to identify candidate TCR affinity reagents; may be used in affinity maturation of TCR affinity reagents.

An eTPCS comprising a single integration couple may be used to prepare an eTPC-t from component 2A in one step, by providing component 2C' combined with an ORF for complementary pair of TCR chains, such that this analyte TCR pair is integrated to site 2B, to create 2B'. The resulting cell line expresses the provided TCR pair, and it is presented at the cell surface as a TCRsp **(****Figure 21****).**

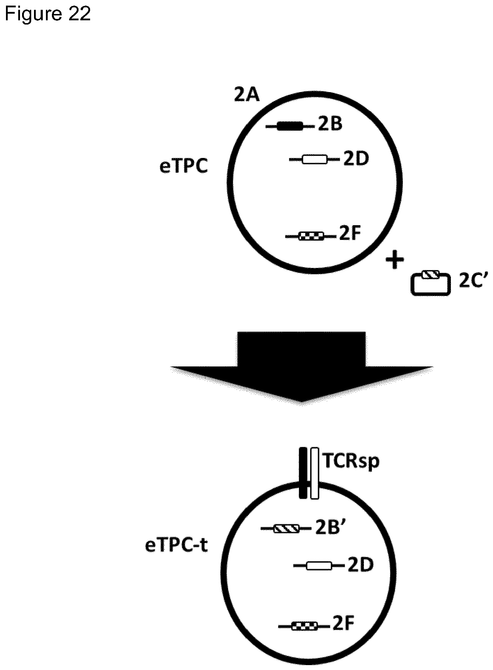

An eTPCS comprising two integration couples may be used to prepare an eTPC-t from component 2A in one step, by providing component 2C' combined with an ORF for complementary pair of TCR chains, such that this analyte TCR pair is integrated to site 2B, to create 2B'. The resulting cell line expresses the provided TCR pair, and it is presented at the cell surface as a TCRsp. The second integration couple 1D/1E remains unmodified and may be used for downstream integration steps **(****Figure 22****).**

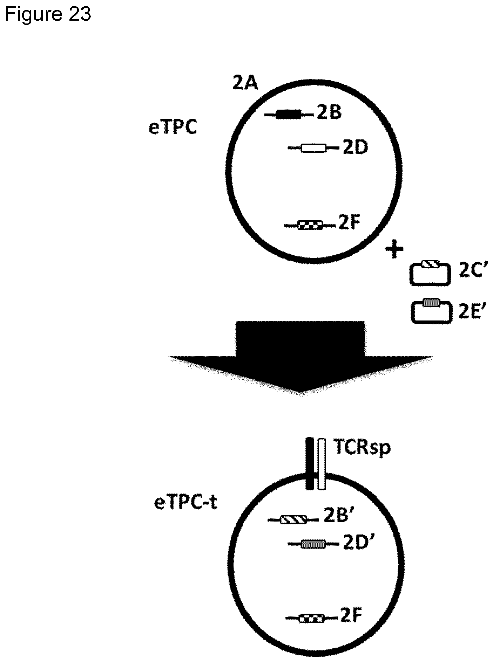

An eTPCS comprising two integration couples may be used to prepare an eTPC-t from component 2A in one step, by providing component 2C' and 2E' each combined with one ORF encoding one chain of a complementary TCR chain pair, such that both analyte TCR chains are integrated to site 2B or 2D, to create 2B' and 2D'. The resulting cell line expresses the provided TCR pair, and it is presented at the cell surface as a TCRsp. **(****Figure 23****).**

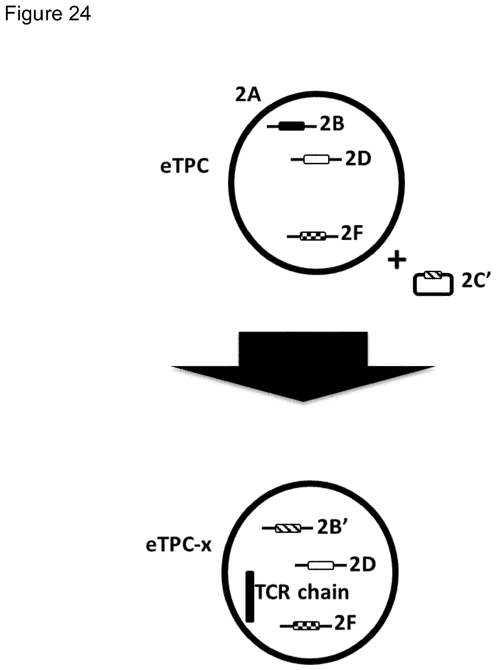

An eTPCS comprising two integration couples may be used to prepare an eTPC-x from component 2A in one step, by providing component 2C' combined with an ORF for a single analyte TCR chain, such that this analyte TCR chain is integrated to site 2B, to create 2B'. The resulting cell line expresses the provided TCR chain, but lacks a complementary chain and thus does not express the any surface TCR. The second integration couple 1D/1E remains unmodified and may be used for downstream integration steps **(****Figure 24****).**

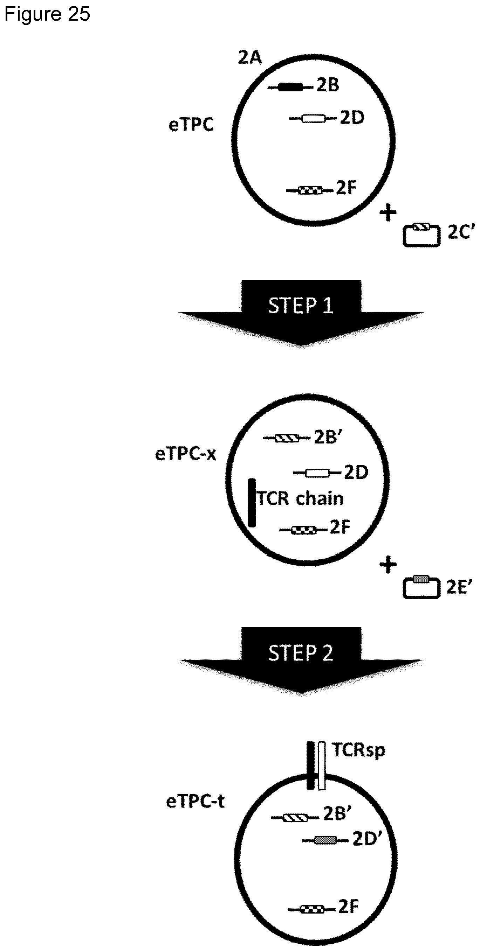

An eTPCS comprising two integration couples may be used to prepare an eTPC-t from component 2A in two steps, by first providing component 2C' combined with an ORF for a single analyte TCR chain, such that this analyte TCR chain is integrated to site 2B, to create 2B'. The resulting cell line expresses the provided TCR chain, but lacks a complementary chain and thus does not express the any surface TCR (eTPC-x intermediate). The second integration couple 1D/1E remains unmodified. In the second step 2E' is provided, wherein the vector is combined with an ORF for a second single analyte TCR chain, complementary to the previously integrated TCR chain, such that this second complementary analyte TCR chain is integrated to site 2D, to create 2D'. The resulting cell line expresses the provided complementary analyte TCR chain pair and it is presented at the cell surface as a TCRsp. **(****Figure 25****).**

In the abovementioned examples of preparing analyte eTPC-x and/or eTPC-t populations from eTPC, the eTPCS system is used to provide known analyte TCR chains in a defined manner to prepare discrete populations of analyte eTPC expressing defined TCRsp. Such a process may be repeated many times to build libraries of eTCP-x and/or eTCP-t to provide to the combined eAPC:eTPC system in operation of the device. An alternative approach is to take pooled libraries of analyte eTPC-t, wherein each eTPC-t integrates a single random pair of TCR ORF from the original vector pool, and thus each eTPC-t expresses a single random TCRsp, but the collectively the pool encodes multiple species of TCRsp represented in the original vector pool. The same shotgun principle can be applied to create pools of eTPC-x. This is particularly useful when analysing large libraries of candidate TCRsp against analyte antigens.

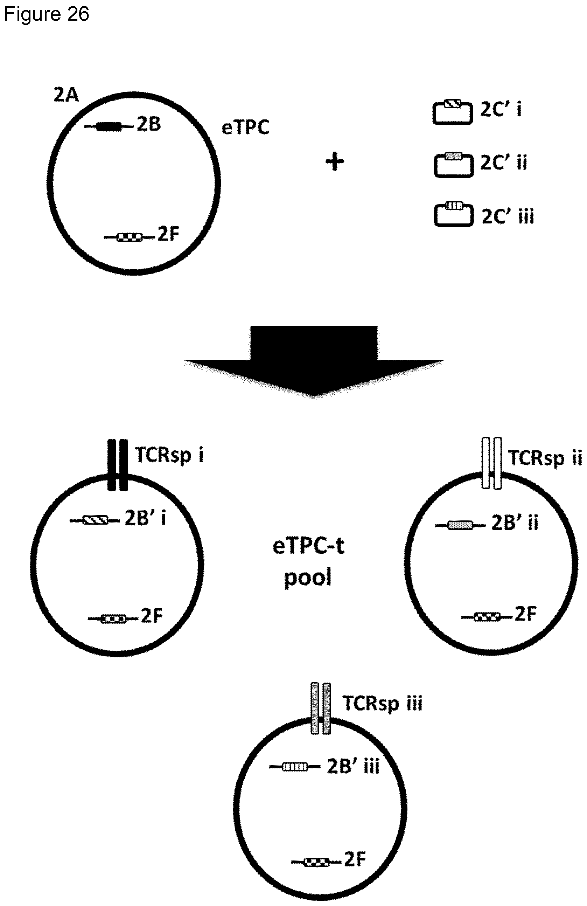

An eTPCS comprising one integration couple may be used to prepare an eTPC-t pool from component 2A in one step, by providing component 2C' combined with a library of multiple ORF encoding a pool analyte of analyte TCR pairs, such that a pair is integrated to site B, to create B', within each cell and wherein each eTPC-t integrates a single random pair of TCR ORF from the original vector pool, and thus each eTPC-t expresses a single random TCRsp, but the collectively the pool encodes multiple species of TCRsp represented in the original vector pool. The resulting pool of cells contains a collection of cells that collectively express multiple analyte TCRsp **(****Figure 26****).**

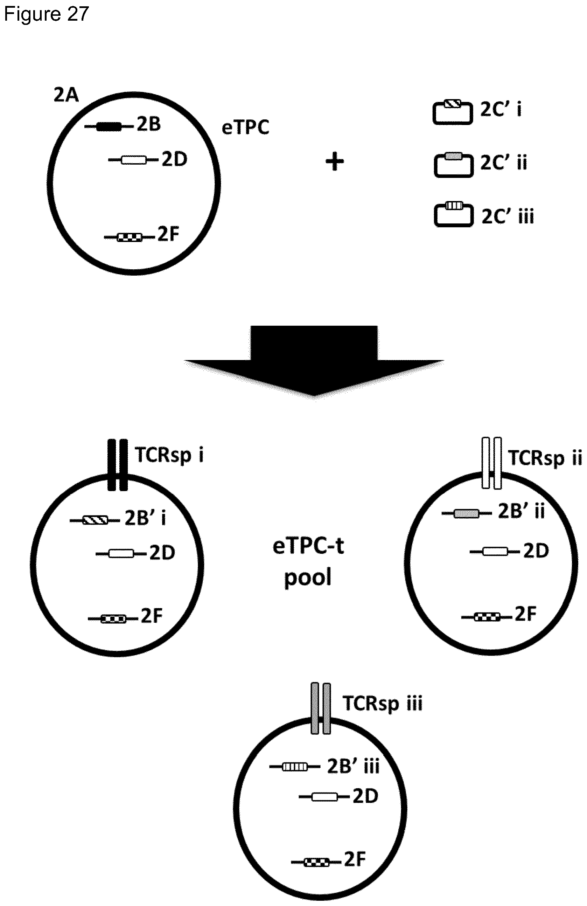

An eTPCS comprising two integration couples may be used to prepare an eTPC-t pool from component 2A in one step, by providing component 2C' combined with a library of multiple ORF encoding a pool of analyte TCR pairs, such that a pair is integrated to site B, to create B', within each cell and wherein each eTPC integrates a single random pair of TCR ORF from the original vector pool, and thus each eTPC-t expresses a single random TCRsp, but the collectively the pool encodes multiple species of TCRsp represented in the original vector pool. The resulting pool of cells contains a collection of cells that collectively express multiple analyte TCRsp. The second integration couple 1D/1E remains unmodified **(****Figure 27****).**

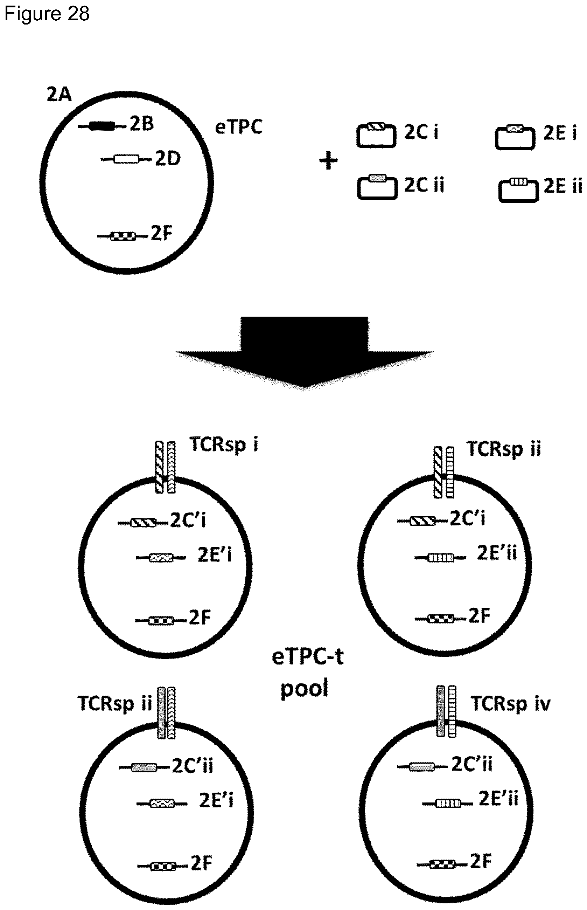

An eTPCS comprising two integration couples may be used to prepare an eTPC-t pool from component 2A in one step, by providing component 2C' combined with a library of multiple ORF encoding a pool of single analyte TCR chains such that each eTPC integrates a single randomised TCR chain ORF encoded in component 2C' to site 2B, to create 2B'. Simultaneously, providing component 2E' combined with a library of multiple ORF encoding a pool of single analyte TCR chains complementary to first library provided in 2C', such that each eTPC-t integrates a single randomised complementary TCR chain ORF encoded in component 2E' into site 2D, to create 2D'. Each resulting cell in the eTPC-t pool has a single pair of randomised complementary TCR chain ORF, such that each cell in the pool expresses a randomised TCRsp. Such a pooled library would contain all possible combinations of provided complementary TCR chains from the sets proceed in components 2C' and 2E' **(****Figure 28****).**

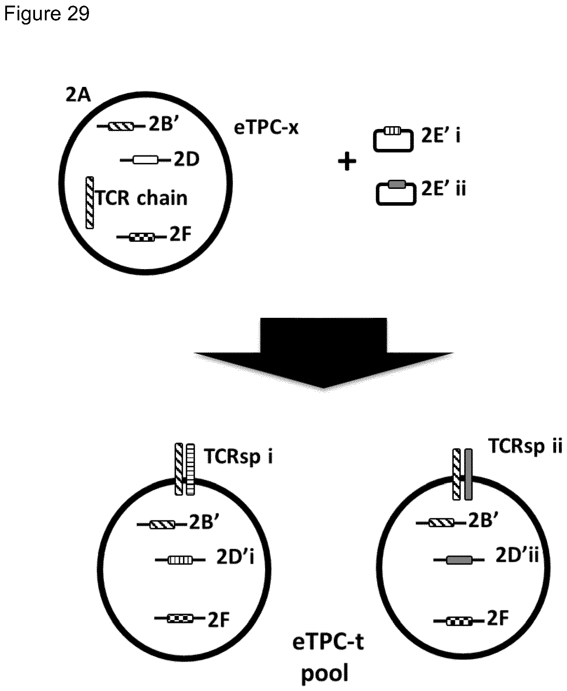

In the present context, an eTPCS comprising two integration couples may be used to prepare an eTPC-t pool from a previously obtained e-TPC-x in one step, wherein the site 2B has been converted to 2B' and contains the single analyte TCR chain. An eTPC-t is prepared by providing component 2E' combined with a library of multiple ORF encoding a pool of single analyte TCR chains complementary to the already integrated chain, such that each such that each eTPC-t integrates a single randomised TCR chain ORF of the provided 2E' library in to site 2D, to create 2D'. Each resulting cell in the eTPC-t pool has the analyte TCR chain provided by the starting eTPC-x, and a selection of each single complementary analyte TCR chain, such that each cell in the pool expresses a random pair of ORF as an TCRsp. Such an approach is used when analysing the effect of varying a single chain against a fixed chain in a complementary TCR chain pair **(****Figure 29****).**

An eTPC-x may be prepared from an eTPC-t by providing either one of further integration vectors, **components 2Y or 2Z,** which encode markers of integration or no ORF. Combination of component 2Y or 2Z to an eTPC-t would exchange either of the sites to obtain a single TCR chain expressing eTPC-x.

A preferred genetic integration vector, **component 2Y and component 2Z,** for conversion of eTPC-t to eTPC-x would comprise the same integration vector requirements as component 2C and 2E above, though not encoding any TCR chain ORF, and preferably encoding a marker of integration.

### Contacting analyte eAPC and eTPC

The present invention relates to the provision of an engineered two-part cellular device. The two parts of the device, eAPCS and eTPCS, are used to assemble analyte eAPC and analyte eTPC, respectively. These analyte cell populations are then assembled into a combined eAPC:eTPC system from which the device outputs may be obtained. The eAPC:eTPC system is comprised of a selection of one or more of analyte eAPC populations, and one or more eTPC populations **(****Figure 1****)** that are prepared as described above **(****Figures 4 to 29****).** The eAPC:eTPC system is provided in a format that permits physical contact between the analyte eAPC and analyte eTPC populations, wherein such contact is permissive of complex formation between one ore more analyte antigen and TCRsp of one or more analyte eTPC-t, wherein the analyte antigen is any of the following
i. aAPX and/or
ii. aAM and/or
iii. aAPX:aAM and/or
iv. CM and/or
v. aAPX:CM
presented by the analyte eAPC, with an analyte TCRsp presented by the analyte eTPC, such that complex formation may lead to stabilisation of such a complex and wherein the induction of signalling within the analyte eAPC and/or the analyte eTPC may be reported and measured.

A contact between an analyte eAPC and analyte eTPC is performed in a permissive cell culture system, wherein said system comprises cell culture media that is permissive to function of both eAPC and eTPC cells.

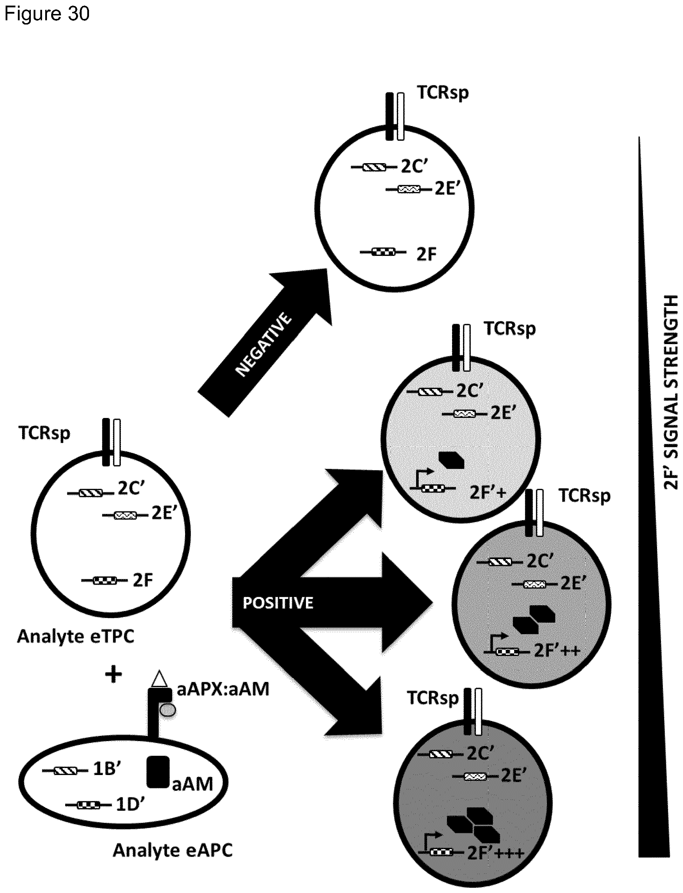

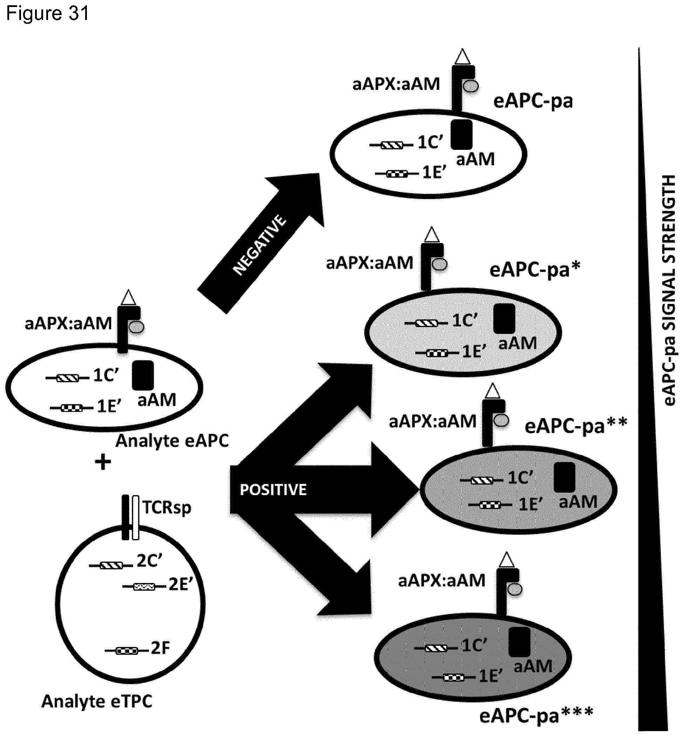

An analyte eTPC obtained from the combined eAPC:eTPC system is used for characterisation of a signal response of the analyte eTPC, expressing analyte **TCRsp,** to an **analyte antigen** presented by the analyte eAPC, wherein such a signal response may be either binary or graduated, and may be measured as intrinsic to the eTPC **(****Figure 30****)** or intrinsic to the eAPC **(****Figure 31****).**

The method for selecting one or more analyte eTPC from an input analyte eTPC or a library of analyte eTPC, from the combined eAPC:eTPC system, to obtain one or more analyte eTPC wherein the expressed TCRsp binds to one or more analyte antigen presented by the analyte eAPC comprises
i. Combining one or more analyte eTPC with one or more analyte eAPC resulting in a contact between an analyte TCRsp with an analyte antigen and at least one of
ii. Measuring a formation, if any, of a complex between one or more analyte TCRsp with one or more analyte antigen and/or
iii. Measuring a signal response by the analyte eTPC, if any, induced by the formation of a complex between one or more analyte TCRsp with one or more analyte antigen and/or
iv. Measuring a signal response by the analyte eAPC, if any, induced by the formation of a complex between one or more analyte TCRsp with one or more analyte antigen and
v. Selecting one or more analyte eTPC based on step b, c and/or d wherein the selection is made by a positive and/or negative measurement
wherein i, iii and v comprise the preferred arrangement.

An analyte eAPC obtained from the combined eAPC:eTPC system is used for characterisation of a signal response of the analyte eAPC, expressing analyte **antigen,** to an **TCRsp** presented by the analyte eTPC, wherein such a signal response may be either binary or graduated, and may be measured as intrinsic to the eTPC **(****Figure 30****)** or intrinsic to the eAPC **(****Figure 31****).**

The method for selecting one or more analyte eAPC from an input analyte eAPC or a library of analyte eAPC, from the combined eAPC:eTPC system, to obtain one or more analyte eAPC wherein the expressed analyte antigen binds to one or more analyte TCRsp presented by the analyte eTPC comprises
i. Combining one or more analyte eAPC with one or more analyte eTPC, resulting in a contact between an analyte antigen presented by the analyte eAPC with analyte TCRsp of one or more analyte eTPC and
ii. Measuring a formation, if any, of a complex between one or more analyte antigen with one or more analyte TCRsp and/or
iii. Measuring a signal response in the one or more analyte eTPC, if any, induced by the formation of a complex between the analyte TCRsp with the analyte antigen and/or
iv. Measuring a signal response, if any, by the analyte eAPC induced by the formation of a complex between one or more analyte TCRsp with one or more analyte antigen and
v. Selecting one or more analyte eAPC from step b, c and/or d wherein the selection is made by a positive and/or negative measurement
wherein i, iii and v comprise the preferred arrangement.

Selection of eTPC or eAPC may be from an eAPC:eTPC system comprising binary culture system wherein a single prepared analyte eAPC population and single prepared analyte eTPC population are provided, and the responding eAPC or eTPC are selected on the basis of a binary or graduated response within the eTPC **(****Figure 30****)** and/or the eAPC **(****Figure 31****).**

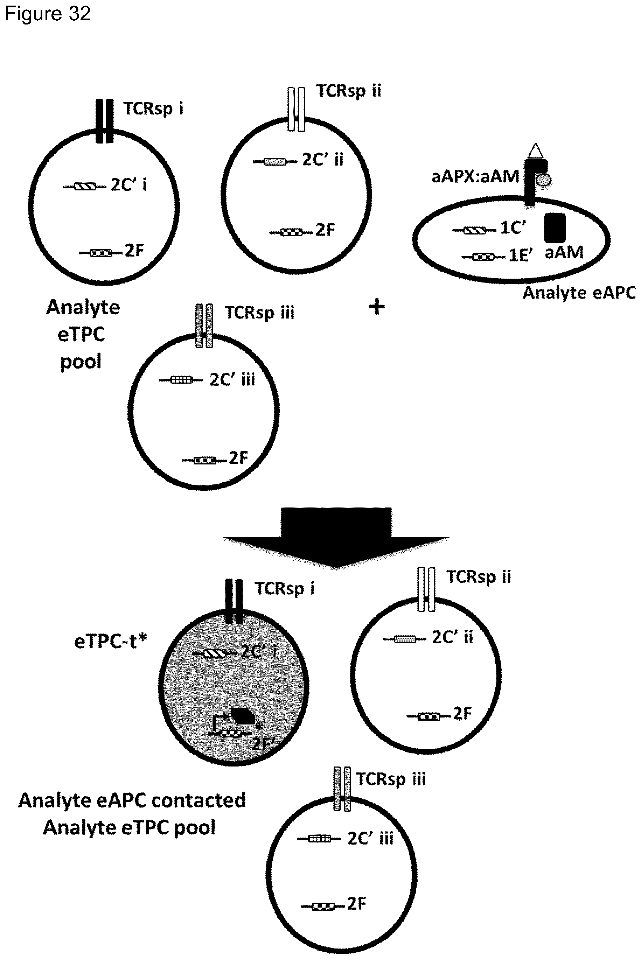

An eAPC:eTPC system may comprise an input of prepared single analyte eAPC and a prepared pooled library of eTPC **(****Figure 32****).**

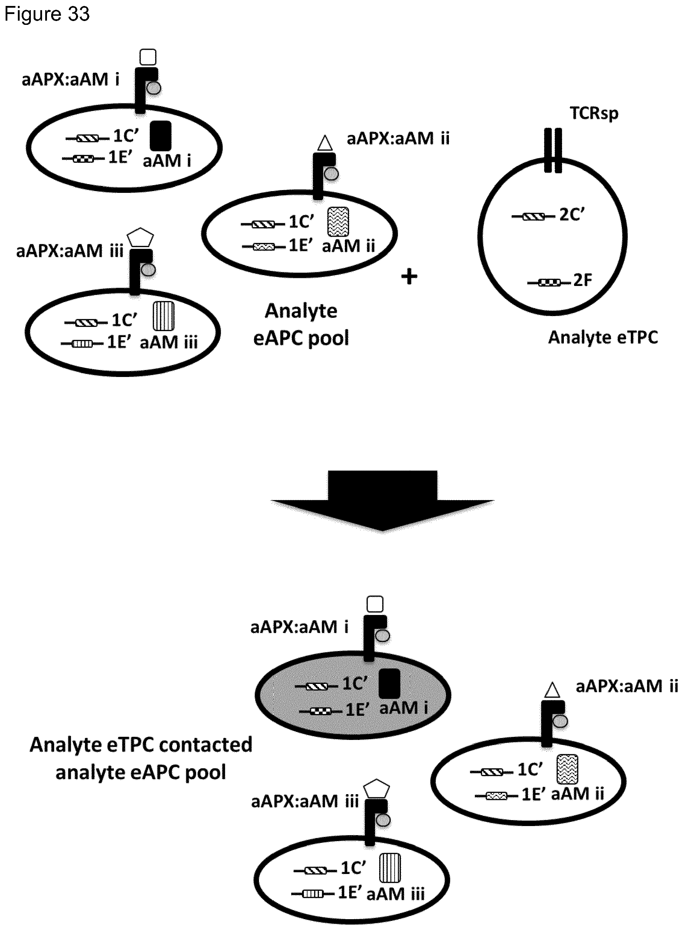

An eAPC:eTPC system may comprise an input of prepared single analyte eTPC and a prepared pooled library of eAPC **(****Figure 33****).**

Within the combined eATP:eTPC system, measuring a signal response in the one or more analyte eTPC or one or more eAPC, if any, which may be induced by the formation of a complex between the analyte TCRsp with the analyte antigen is critical to selection of primary device outputs, wherein the primary device outputs are single cells or pools of cells of one or more of the following
i. eAPC-p
ii. eAPC-a
iii. eAPC-pa
iv. eTPC-t
wherein the selection of cells may be made on the presence or absence of a reported signal response in either, or both, of the contacted analyte eAPC or analyte eTPC cells.

In the present context, a method for selecting analyte eTPC and/or analyte eAPC from the combined eAPC:eTPC system on the basis of a reported signal response within the eTPC comprises
i. Determining a native signalling response and/or
ii. Determining a synthetic signalling response, if the eTPC contains component 2F, and if the eAPC contains an equivalent synthetic reporter element.

An induced native or synthetic signal response that is intrinsic to eAPC and/or eTPC is measured by detecting an increase or decrease in one or more of the following
i. a secreted biomolecule
ii. a secreted chemical
iii. an intracellular biomolecule
iv. an intracellular chemical
v. a surface expressed biomolecule
vi. a cytotoxic action of the analyte eTPC upon the analyte **eAPC**
vii. a paracrine action of the analyte eTPC upon the analyte **eAPC** such that a signal response is induced in the analyte eAPC and is determined by detecting an increase or decrease any of a to e
viii. a proliferation of the analyte eTPC
ix. an immunological synapse between the analyte eTPC and the analyte eAPC
wherein said detected signal responses are compared to the non-induced signal response state intrinsic to analyte eAPC and/or analyte eTPC prior to assemble of the combined eAPC:eTPC system and/or a parallel assembled combined system wherein analyte eAPC and/or analyte eTPC may present control analyte antigen and/or analyte TCR species that are known not to the induce a signal response within the combined eAPC:eTPC system in use.

### Obtaining primary device outputs from the eAPC:eTPC system

The present invention relates to the provision of an engineered two-part cellular device. The two parts of the device, eAPCS and eTPCS, are used to assemble analyte eAPC and analyte eTPC, respectively. These analyte cell populations are then assembled into a combined eAPC:eTPC system from which the device outputs may be obtained. The eAPC:eTPC system is comprised of a selection of one or more of analyte eAPC populations, and one or more eTPC populations **(****Figure 1****)** that are prepared as described above **(****Figures 4 to 29****).** The eAPC:eTPC system is provided in a format that permits physical contact between the analyte eAPC and analyte eTPC populations, wherein such contact is permissive of complex formation between one ore more analyte antigen and TCRsp of one or more analyte eTPC-t, wherein the analyte antigen is any of the following
i. aAPX and/or
ii. aAM and/or
iii. aAPX:aAM and/or
iv. CM and/or
v. aAPX:CM
presented by the analyte eAPC, with an analyte TCRsp presented by the analyte eTPC, such that complex formation may lead to stabilisation of such a complex and wherein the induction of signalling within the analyte eAPC and/or the analyte eTPC may be reported and measured.

The modes of induced signal response reporting are described above, and it is these reported responses that are required to be measured in obtaining the primary output of the two-part device.

Primary outputs from the device are selected cell populations, which have or have not responded to the analyte presented by the reciprocal cell provided in the eAPC:eTPC system. That is, such a primary output may be represented as a single cell, or a pool of cells, that have been selected on the presence or absence of a reported response within the combined eAPC:eTPC system **(****Figure 1** **step v).** A response within an analyte eAPC is only provoked by engagement of a cognate TCR presented by a contacting eTPC. A response within an analyte eTPC is only provoked by engagement of a cognate antigen presented by a contacting eAPC **(****figure 1** **step iv).**

A selection of analyte eAPC and/or analyte eTPC from the combined eAPC:eTPC system may be made on the basis of a response in the contacting cell. That is, an analyte eAPC may be selected on that basis of a reported response, or lack thereof, in the contacting analyte eTPC. Conversely, an analyte eTPC may be selected on that basis of a reported response, or lack thereof, in the contacting analyte eAPC.

In the present context, primary eAPC outputs from the device are selected cells, wherein selection is made based on the presence or absence of a reported signal response, and these cells may comprise one or more of
i. an eAPC-p
ii. an eAPC-a
iii. an eAPC-pa
wherein the selected cells may comprise a single cell, a pool of cells of the same identity, a pool of cells of different identities **(****Figure 1** **step v).**

Primary eTPC outputs from the device are selected cells, wherein selection is made based on the presence or absence of a reported signal response, and these cells comprise eTPC-t, wherein selected cells may comprise a single cell, a pool of cells of the same identity, a pool of cells of different identities **(****Figure 1** **step v).**

The reported signals in the analyte eAPC and/or analyte eTPC in a combined eAPC:eTPC system is used to select analyte cell populations to provide the primary outputs.

A primary output of eAPC and/or eTPC types may be achieved in a an instance wherein the combined eAPC:eTPC system is of binary culture nature (e.g. **Figure 30** **and** **Figure 31****)** by selecting the desired analyte eAPC and/or analyte eTPC population from the binary system.

A primary output of eAPC and/or eTPC types may be achieved from an instance wherein the combined eAPC:eTPC system is of fixed eAPC and pooled library analyte eTPC nature (e.g. **Figure 32****),** or from an instance wherein the combined eAPC:eTPC system is of fixed eTPC and pooled library analyte eAPC nature (e.g. **Figure 33****)** by selecting the desired analyte eAPC and/or analyte eTPC population from the combined culture system.

There are several distinct modes in which the primary outputs may be obtained, wherein each mode entails a step of cell sorting. Cell sorting may be achieved through fluorescence-activated cell sorting (FACS) and/or magnetic-activated cell sorting (MACS) and/or distinct affinity-activated cell sorting methods.

Primary output eAPC and/or eTPC cells may be obtained by single cell sorting to obtain a single cell and/or cell sorting to a pool to obtain a pool of cells.

Primary output eAPC and/or eTPC cells may be obtained by single cell sorting to obtain a single cell, and subsequent outgrowth of the single cells to obtain monoclonal pool of selected eAPC or eTPC cells.

Primary output eAPC and/or eTPC cells may be obtained by cell sorting to a pool to obtain a pool of cells, and subsequent outgrowth of the pool of cells to obtain a pool of selected eAPC and/or eTPC cells.

### Obtaining terminal device outputs from the eAPC:eTPC system

Subsequent to the above-described methods of obtaining primary outputs, wherein primary outputs are selected analyte eAPC and/or eTPC cells that are selected on the basis of a measured signal response, the terminal outputs of the two part cellular device may be obtained via further processing of the selected eAPCa and/or eTPC primary outputs.

Terminal outputs from the two-part cellular device are the identities of
i. aAPX and/or
ii. aAM and/or
iii. aAPX:aAM and/or
iv. CM and/or
v. aAPX:CM an/or
vi. TCRsp
presented by the analyte eAPC (i to v) or analyte eTPC (vi), and obtained as primary outputs from the two-part device by their selection from the combined eAPC:eTPC system.

Within the two-part cellular device, it is often the case that analyte molecules that are presented by the analyte eAPC and analyte eTPC are genetically encoded. Therefore, to identify the analyte molecules presented by the analyte eAPC or eTPC, genetic sequencing of the prepared analyte molecules from the eAPC or eTPC may be performed.

eAPC may be processed such that genetic sequence is obtained for component 1B' and/or component 1D' of the sorted and/or expanded eAPC-p, eAPC-a or eAPC-pa cells to determine the identity of
i. aAPX and/or
ii. aAM and/or
iii. aAPX:aAM
wherein the obtained identities represent terminal outputs from the two-part cellular device.

eAPC may be processed such that genetic sequence is obtained for the genome of the sorted and/or expanded eAPC-p, eAPC-a or eAPC-pa cells to determine the identity of
i. aAPX and/or
ii. aAM and/or
iii. aAPX:aAM
iv. CM and/or
v. aAPX:CM
wherein the obtained identities represent terminal outputs from the two-part cellular device.

eTPC may be processed such that genetic sequence is obtained for component 2B' and/or component 2D' of the sorted and/or expanded eTPC-t cells to determine the identity of TCRsp, wherein the obtained identity of TCRsp represents a terminal output from the two-part cellular device.

eTPC may be processed such that genetic sequence is obtained for the genome of the sorted and/or expanded eTPC-t cells to determine the identity of TCRsp, wherein the obtained identify of TCRsp represents a terminal output from the two-part cellular device.

Genetic sequencing can be achieved by a range of modes, and from arrange genetic material sources, with and without specific processing.

In the present context, the sequencing step may be preceded by
i. Extracting of genomic DNA and/or
ii. Extracting of components 1B' and/or 1D' and/or 2B' and/or 2D' RNA transcript and/or
iii. Amplifying by a PCR and/or a RT-PCR of the DNA and/or RNA transcript of component 1B' and/or 1D' and/or 2B' and/or 2D'.

The sequencing step may be destructive to the eAPC or eTPC, or pool thereof, obtained as primary outputs from the two-part device.

If it is desirable to obtain primary outputs from the two-part device wherein the sequencing step has been destructive to the primary output eAPC or eTPC, the sequence information obtained as terminal output of the two-part device may be used to prepare equivalent output eAPC and eTPC as analyte eAPC or analyte eTPC through use of the eAPCS and eTPCS, respectively.

In the above-described scenarios of genetically encoded analyte molecules, the terminal outputs of the two-part device may be obtained by obtaining sequence information from component 1B' and/or 1D' and/or 2B' and/or 2D', and/or from the cell genome. However, in some embodiments the antigen information will not be genetically encoded. Post-transnationally modified antigens, antigens provided to the combined eAPC:eTPC system through non-genetic means, antigens that are emergent from an induced or modified state of the analyte eAPC proteome or metabolite, and CM intrinsic to the eAPC:eTPC system, may not reasonably be identified through genetic means.

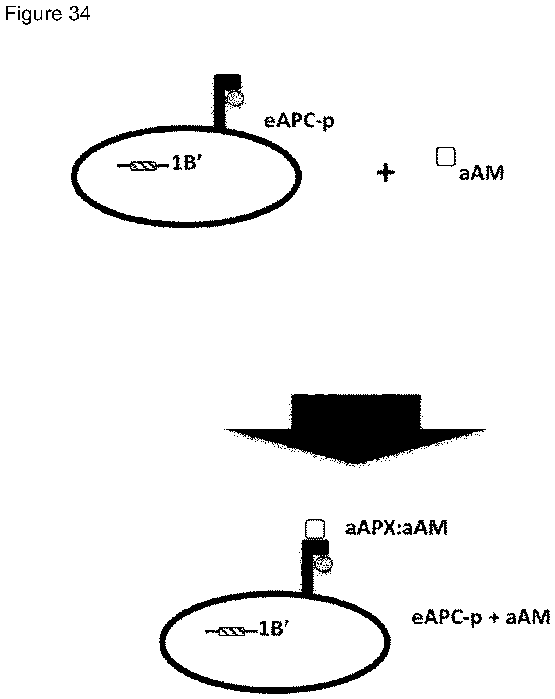

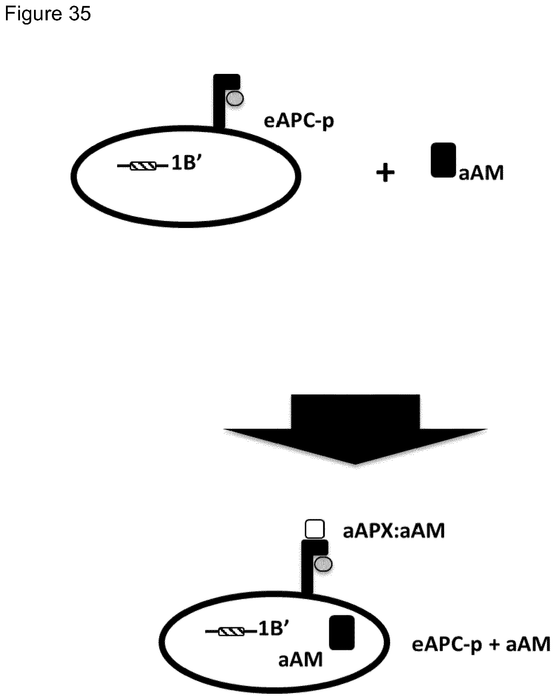

In the important case of aAM that may be provided to the eACP:eTPC system by non-genetic means, there are two distinct modes through which an eAPC-p or eAPC-pa may present a provided aAM as an aAPX:aAM complex. In the first scenario the aAM is provided in a form that may directly bind to the aAPX and forms an aAPX:aAM complex at the cells surface **(****Figure 34****).** An example of such an aAM would be a peptide antigen for an HLA complex. In the second scenario, the aAM is provided is in a form that may be taken up by the analyte eAPC and processed such that it is loaded as cargo in the aAPX and forms an aAPX:aAM complex at the cells surface **(****Figure 35****)**. In the present context, a method to select and identify an **aAM** cargo or a **CM** cargo, wherein the cargo is a metabolite and/or a peptide, that is loaded in an **aAPX** of an e**APC** selected and obtained by as a a primary output of the two-part device, comprises
i. isolating an **aAPX:aAM** or an **aAPX:CM** or the **cargo aM** or the **cargo CM** and
ii. identifying the loaded cargo
wherein the identified loaded cargo represent terminal outputs of the two-part device.

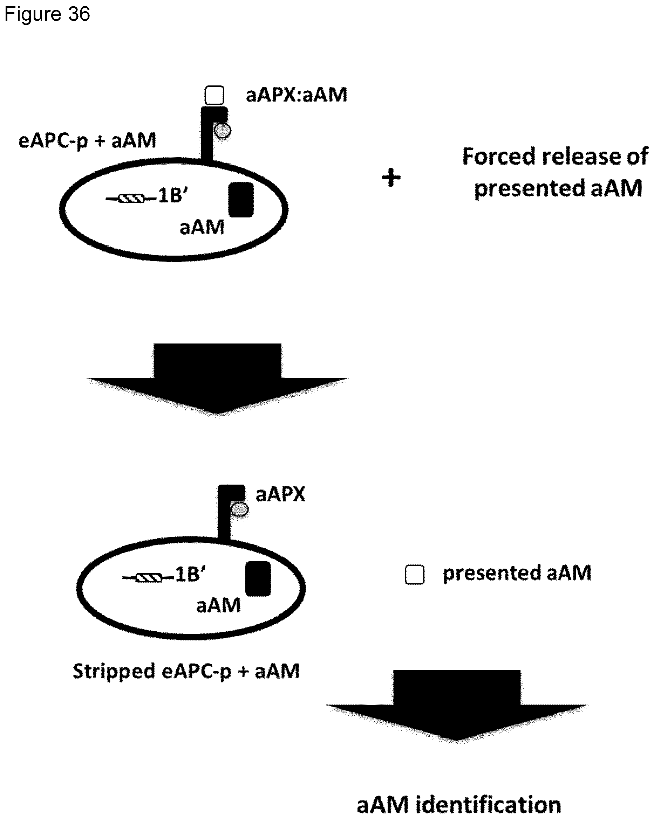

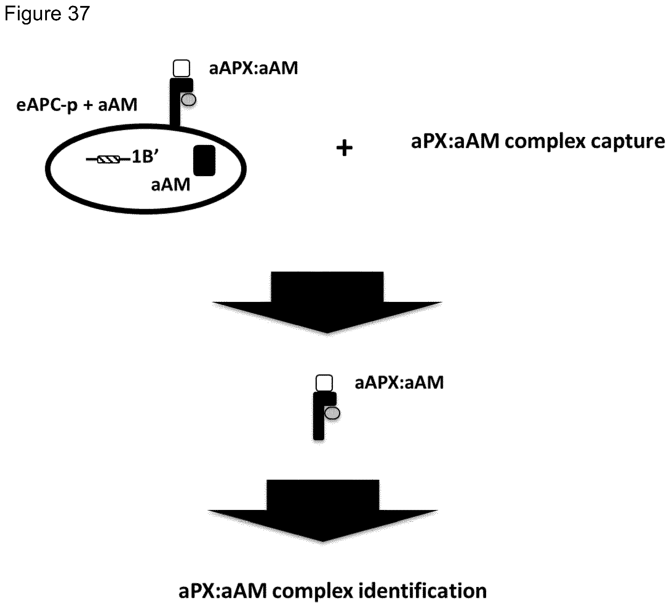

There are generally two modes through which a cargo molecule may be identified from a selected eAPC. First, a forced release of the cargo from the aAPX:aAM or aAPX:CM results in isolation of the aAM or CM that is available for subsequent identification **(Figure 36).** An example of this would be acid-washing of the eAPC to liberate peptide aAM from HLA complexes. Secondly, the capture of the aAPX:aAM or aAPX:CM, for example, by liberation of the complex and immunoaffinity isolation methods, results in isolation of the aAPX:aAM or aAPX:CM compelxes, such that aAM or CM can be identified **(****Figure 37****)**.

Methods for identifying isolated aAM and/or CM directly, or from the isolated **aAPX:aAM** or an **aAPX:CM** complexes, can comprise
i. Mass-spectrometry analysis
ii. Peptide sequencing analysis
wherein the ontain aAM and/or CM identities are terminal outputs from the two-part cellular device.

### Legends to figures

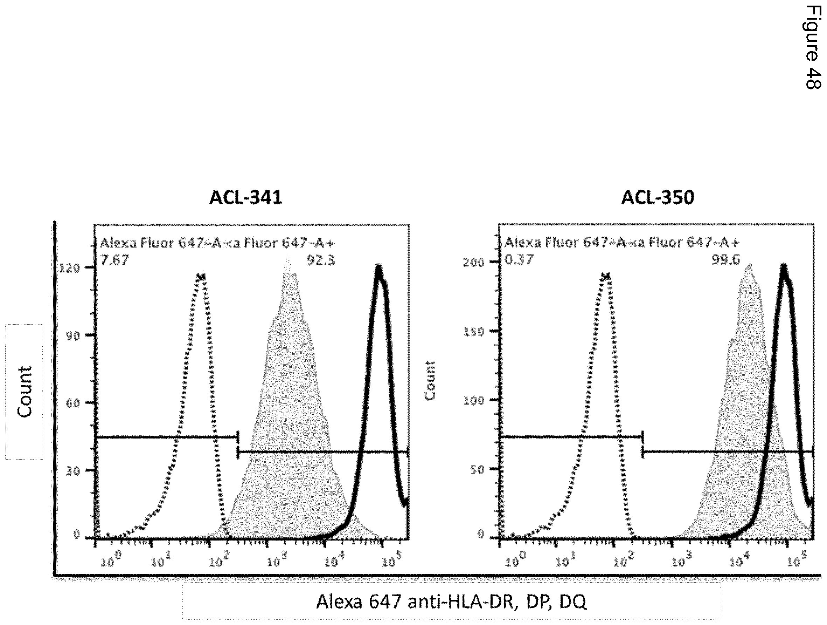

The invention is illustrated in the following non-limiting figures.
**Figure 1** - **Operation of the two-part device comprised of eAPC and eTPC systems**
   The two-part engineered cellular device is comprised of two multicomponent cell systems, the **eAPCS** and the **eTPCS,** which are contacted as a combined **eAPC:eTPC system.** Operation of the overall device comprises two phases, the preparation phase, and the analytical phase. In one aspect **of Phase 1,** the **eAPCS** system is used to prepare cells expressing analyte antigen-presenting complex **(aAPX),** and/or analyte antigenic molecule **(aAM)** at the cell surface (**step i**). An **eAPC** presenting **aAPX** alone is termed **eAPC-p.** An **eAPC** presenting **aAM** alone is termed **eAPC-a.** An **eAPC** presenting an **aAM** presented as cargo in an **aAPX** is termed an **eAPC-pa.** These analytes are collectively referred to as the analyte antigen. In a separate aspect of **Phase 1,** the **eTPCS** system is used to prepare cells expressing analyte TCR chain pairs **(TCRsp)** at the cell surface **(step ii).** An **eTPC** presenting a **TCRsp** at the cell surface is termed an **eTPC-t. Phase 2** of the overall system is the contacting of the analyte-bearing cells prepared in **Phase 1,** to form the combined **eAPC:eTPC system (step iii).** Contacted analyte **eAPC** present analyte antigens to the **analyte eTPC.** Within the combined **eAPC:eTPC system,** the responsiveness of the **analyte TCR chain pair** towards the provided **analyte antigen** is determined by readout of a contact-dependent **analyte eAPC and/or eTPC response,** as denoted by * and the shaded box to represent an altered signal state of these reporting analyte cells **(step iv).** As an outcome of the **eAPC:eTPC system** specific **eAPC** and/or **eTPC-t** can be selected based on their response and/or their ability to drive a response in the other contacting analyte cell. It is thus selected single cells, or populations of cells, of the type, **eAPC-p, eAPC-a, eAPC-pa** and/or **eTPC-t** that are the **primary outputs** of the device operation **(step v).** By obtaining the analyte cells from **step v,** the presented analyte **aAPX, aAM, aAPX:aAM, CM, aAPX:CM** and/or **TCRsp** may be identified from these cells as the **terminal output** of the device operation **(step vi).**
**Figure 2** - **Description of the components of a single integration couple eAPCS.**
   An example of an **eAPCS** comprising three components. The first component **1A** is the **eAPC** line itself with all required engineered features of that cell. The **eAPC 1A** contains one further component **1B,** which is a genomic integration site for integration of **aAPX** and/or **aAM.** One additional component, **1C** represents a genetic donor vector for site-directed integration of ORFs into sites **1B,** wherein the arrow indicates coupled specificity. The paired integration site / donor vector couple may be formatted to integrate a single ORF or a pair of ORFs to introduce **aAPX** and/or **aAM** expression.
**Figure 3** - **Description of the components of a dual integration couple eAPCS.**
   An example of an **eAPCS** comprising five components. The first component **1A** is the **eAPC** line itself with all required engineered features of that cell. The **eAPC 1A** contains two further components, **1B** and **1D,** which are genomic integration sites for integration of **aAPX** and/or **aAM.** Two additional components, **1C and 1E,** represent genetic donor vectors for site-directed integration of ORFs into sites **1B** and **1D,** respectively, wherein arrows indicate paired specificity. Each paired integration site / donor vector couple may be formatted to integrate a single ORF or a pair of ORFs to introduce **aAPX** and/or **aAM** expression.
**Figure 4** - **Compilation of different analyte antigen presenting eAPC**
   The **eAPCS** system begins with the **eAPC** and uses a donor vector(s) to create cells expressing analyte antigen-presenting complex **(aAPX),** and/or analyte antigenic molecule **(aAM)** at the cell surface. An **eAPC** presenting **aAPX** alone is termed **eAPC-p,** and may be created by introduction of **aAPX** encoding ORF(s) to the **eAPC (step i).** An **eAPC** expressing **aAM** alone is termed **eAPC-a,** wherein **aAM** may be expressed at the cell surface and available for TCR engagement, or require processing and loading as cargo into an **aAPX** as the **aAPX:aAM** complex. An **eAPC 1A** may be created by introduction of **aAM** encoding ORF(s) to the **eAPC (step ii).** An **eAPC** presenting an **aAM** as cargo in an **aAPX** is termed an **eAPC-pa.** An **eAPC-pa** be produced either; introduction of **aAM** and **aAPX** encoding ORFs to an **eAPC** simultaneously **(step iii);** introduction of **aAM** encoding ORF(s) to an **eAPC-p (step iv);** introduction **aAPX** encoding ORF(s) to an **eAPC-a (step v).**
**Figure 5** - **Operation of the genetic donor vector and genomic receiver site integration couple**
   A genetic donor vector and genomic receiver site form an integration couple, wherein one or more ORFs encoded within the genetic donor vector can integrated specifically to its coupled genomic receiver site. **Step 1** in operation of the integration couple is to introduce one or more target ORFs to the donor vector. The initial donor vector is denoted **X,** and is modified to a primed donor vector **X',** by introduction of target ORF(s). **Step 2** entails combination of the primed donor vector, **X',** with a cell harbouring a genomic receiver site, Y. Introduction of the ORF encoded by the primed donor vector into the receiver site results in the creation of a cell harbouring an integrated site, **Y'.**
**Figure 6** - **Example of compilation of an eAPC-p in one step with one integration couple**
   **eAPC 1A** contains genomic receiver site **1B.** Primed genetic donor vector **1C'** is coupled to **1B** and encodes an **aAPX.** When the **1A eAPC** is combined with the **1C'** donor vector. The resulting cell has the ORF of **1C'** exchanged to the **1B** genomic receiver site to create site **1B'** and introduce **aAPX** expression. This results in expression of the **aAPX** on the cell surface and creation of an **eAPC-p.**
**Figure 7** - **Example of compilation of an eAPC-p in one step with one integration couple and one unused integration site**
   **eAPC 1A** contains genomic receiver sites **1B** and **1D.** Primed genetic donor vector **1C'** is coupled to **1B** and encodes an **aAPX.** When the **1A eAPC** is combined with the **1C'** donor vector. The resulting cell has the ORF of **1C'** exchanged to the **1B** genomic receiver site to create site **1B'** and introduce **aAPX** expression. This results in expression of the **aAPX** on the cell surface and creation of an **eAPC-p.** Genomic receiver site **1D** remains unused.
**Figure 8** - **Example of compilation of an eAPC-a in one step with one integration couple**
   **eAPC 1A** contains genomic receiver site **1B.** Primed genetic donor vector **1C'** is coupled to **1B** and encodes an **aAM.** When the **1A eAPC** is combined with the **1C'** donor vector. The resulting cell has the ORF of **1C'** exchanged to the **1B** genomic receiver site to create site **1B'** and introduce **aAM** expression. This results in one of two forms of **eAPC-a,** expressing **aAM** at the cell surface or intracellularly.
**Figure 9** - **Example of compilation of an eAPC-a in one step with one integration couple and one unused integration site**
   **eAPC 1A** contains genomic receiver sites **1B** and **1D.** Primed genetic donor vector **1C'** is coupled to **1B** and encodes an **aAM.** When the **1A** eAPC is combined with the **1C'** donor vector. The resulting cell has the ORF of **1C'** exchanged to the **1B** genomic receiver site to create site **1B'** and introduce **aAM** expression. This results in one of two forms of **eAPC-a,** expressing **aAM** at the cell surface or intracellularly. Genomic receiver site **1D** remains unused.
**Figure 10** - **Example of compilation of an eAPC-pa in one step with one integration couple**
   **eAPC 1A** contains genomic receiver site **1B.** Genetic donor vector **1C'** is coupled to **1B.** Donor vector **1C'** encodes an **aAPX** as well as an **aAM.**
   The **1A eAPC** is combined with donor vectors **1C'.** The resulting cell has the ORFs **1C'** exchanged to the **1B** genomic receiver site to create site **1B'** and deliver an ORF for an **aAPX** and an **aAM.** This results in expression of the **aAPX** on the cell surface, **aAM** intracellularly, and thus loading of the **aAM** as cargo in the **aAPX** in formation of the **aAPX:aAM** complex at the cell surface.
**Figure 11** - **Example of compilation of an eAPC-pa in one step with one integration couple and one unused integration site**
   **eAPC 1A** contains distinct genomic receiver sites **1B** and **1D.** Genetic donor vector **1C'** is coupled to **1B.** Donor vector **1C'** encodes an **aAPX** as well as an **aAM.** The **1A** eAPC is combined with donor vectors **1C'.** The resulting cell has the ORFs **1C'** exchanged to the **1B** genomic receiver site to create site **1B'** and deliver an ORF for an **aAPX** and an **aAM.** Genomic receiver site **1D** remains unused. This results in expression of the **aAPX** on the cell surface, **aAM** intracellularly, and thus loading of the **aAM** as cargo in the **aAPX** in formation of the **aAPX:aAM** complex at the cell surface. This creates an **eAPC-pa** cell line. Genomic receiver site **1D** remains unused.
**Figure 12** - **Example of compilation of an eAPC-pa in one step with two integration couples**
   **eAPC 1A** contains distinct genomic receiver sites **1B** and **1D.** Distinct genetic donor vectors **1C'** and **1E'** are independently coupled to **1B** and **1D,** respectively. Donor vector **1C'** encodes an **aAPX** and donor vector 1E' encodes an **aAM.** The **1A eAPC** is combined with donor vectors **1C'** and **1E'** simultaneously. The resulting cell has the ORF **1C'** exchanged to the **1B** genomic receiver site to create site **1B'** and deliver an ORF for an **aAPX.** Simultaneously, the ORF of **1E'** exchanged to the **1D** genomic receiver site to create site **1D'** and deliver an ORF for an **aAM.** This results in expression of the **aAPX** on the cell surface, **aAM** intracellularly, and thus loading of the **aAM** as cargo in the **aAPX** in formation of the **aAPX:aAM** complex at the cell surface. This creates an **eAPC-pa** cell line.
**Figure 13** - **Example of compilation of an eAPC-pa in two steps with two integration couples via eAPC-p**
   **eAPC 1A** contains distinct genomic receiver sites **1B** and **1D.** Distinct genetic donor vectors **1C'** and **1E'** are independently coupled to **1B** and **1D,** respectively. Donor vector **1C'** encodes an **aAPX** and donor vector **1E'** encodes an **aAM.** In **STEP1** the **1A eAPC** is combined with the **1C'** donor vector. The resulting cell has insert **1C'** exchanged to the **1B** genomic receiver site to create site **1B'** and deliver an ORF for an **aAPX.** This results in expression of the **aAPX** on the cell surface and creation of an **eAPC-p.** Genomic receiver site **1D** remains unused. In **STEP2** the **eAPC-p** created in STEP1 is combine with the **1E'** donor vector. The resulting cell has insert **1E'** exchanged to the **1D** genomic receiver site to create site **1D'** and deliver an ORF for an **aAM.** This results in expression of the **aAM** on the cell surface as cargo of the expressed **aAPX,** and creation of an **eAPC-pa.**
**Figure 14** - **Example of compilation of an eAPC-pa in two steps with two integration couples via eAPC-a**
   eAPC **1A** contains distinct genomic receiver sites **1B** and **1D.** Distinct genetic donor vectors **1C'** and **1E'** are independently coupled to **1B** and **1D,** respectively. Donor vector **1C'** encodes an **aAM** and donor vector **1E'** encodes an **aAPX.** In **STEP1** the **1A eAPC** is combined with the **1C'** donor vector. The resulting cell has insert **1C'** exchanged to the **1B** genomic receiver site to create site **1B'** and deliver an ORF for an **aAM.** This results in expression of the **aAM** on the cell surface and creation of an **eAPC-a.** Genomic receiver site **1D** remains unused. In **STEP2** the **eAPC-a** created in STEP1 is combine with the **1E'** donor vector. The resulting cell has insert **1E'** exchanged to the **1D** genomic receiver site to create site **1D'** and deliver an ORF for an **aAPX.** This results in expression of the **aAPX** on the cell surface with the **aAM** as cargo and creation of an **eAPC-pa.**
**Figure 15** - **Shotgun compilation of an eAPC-pa pool from an eAPC-p**
   The **eAPC-p** contains the exchanged genomic receiver site **1B'** expressing an **aAPX** and the distinct genomic receiver site **1D.** The pool of genetic donor vectors **1E' i-iii** are coupled to **1D.** Donor vectors **1E' i-iii** each encode a single **aAM** gene. The **eAPC-p** is combined with donor vectors **1E' i, 1E' ii, 1E' iii** simultaneously. The resulting cell pool has either of inserts **1E' i-iii** exchanged to the **1D** genomic receiver site in multiple independent instances to create sites **1D' i-iii** each delivering a single ORF for an **aAM** gene. The resulting **eAPC-pa** cell pool comprises a mixed population of three distinct cell cohorts each expressing a discrete combination of **1B'** presenting as **aAPX:aAM** either of the **aAM** genes contained in the initial vector library.
**Figure 16** - **Shotgun compilation of an eAPC-pa pool from an eAPC-a**
   **eAPC-a** contains the exchanged genomic receiver site **1B'** expressing an **aAM** and the distinct genomic receiver site **1D.** The pool of genetic donor vectors **1E'** i-iii are coupled to **1D.** Donor vectors **1E' i-iii** each encode a single **aAPX** gene. The **eAPC-a** is combined with donor vectors **1E' i, 1E' ii, 1E' iii** simultaneously. The resulting cell pool has either of inserts **1E' i-iii** exchanged to the **1D** genomic receiver site in multiple independent instances to create sites **1D' i-iii** each delivering a single ORF for an **aAPX** gene. The resulting **eAPC-pa** cell pool comprises a mixed population of three distinct cell cohorts each expressing a discrete combination of the **aAM** encoded in **1B'** and either of the **aAPX** genes contained in the initial vector library.
**Figure 17** - **Shotgun compilation of pooled eAPC-pa libraries from eAPC containing combinatorial paring of aAM and aAPX genes**
   **eAPC 1A** contains distinct genomic receiver sites **1B** and **1D.** Distinct genetic donor vectors **1C'** and **1E'** are coupled to **1B** and **1 D,** respectively. Donor vectors **1C'** i and **1C' ii** each encode a single **aAM** gene, and donor vectors **1E' i** and **1E' ii** each encode a single **aAPX** gene. The **eAPC 1A** is combined with donor vectors **1C' i, 1C' ii, 1E' i** and **1E' ii** simultaneously. The resulting cell pool has insert **1C' i** or **1C' ii** exchanged to the **1B** genomic receiver site multiple independent instances to create sites **1B' i** and **1B' ii,** each delivering a single ORF for an **aAM.** The resulting cell pool further has insert **1E i** or **1E ii** exchanged to the **1D** genomic receiver site multiple independent instances to create sites **1E' i** and **1E' ii,** each delivering a single ORF for an **APX** gene. The resulting **eAPC-pa** cell pool comprises a mixed population of four distinct cell cohorts each expressing a discrete randomised **aAPX:aAM** pair at the surface comprised of one of each gene contained in the initial vector library.
**Figure 18** - **Description of the components of a single integration couple eTPCS** An example of an **eAPCS** comprising four components. The first component **2A** is the **eTPC** line itself with all required engineered features of that cell. The **eTPC 2A** contains a second component, **2B,** which is a genomic integration site for integration of a pair of complementary analyte TCR chain ORFs. A third component included in the **eTPC, 2A,** is a synthetic reporter construct that is induced upon TCR ligation, **2F.** One additional independent component, **2C,** represents a genetic donor vectors for site-directed integration of ORFs into site **2B,** where arrow indicates coupled specificity.
**Figure 19** - **Description of the components of a dual integration couple eTPCS**
   An example of an **eAPCS** comprising six components. The first component **2A** is the **eTPC** line itself with all required engineered features of that cell. The **eTPC 2A** contains three further components, two of which are **2B** and **2D,** which are genomic integration sites for integration of an analyte TCR chain pair. A third component included in the **eTPC, 2A,** is a synthetic reporter construct that is induced upon TCR ligation, **2F.** Two additional independent components, **2C** and **2E,** represent genetic donor vectors for site-directed integration of ORFs into sites **2B** and **2D,** respectively, where arrows indicate coupled specificity. Each paired integration site / donor vector couple may be formatted to integrate a single ORF or a pair of ORFs to introduce analyte TCR chain pair expression by different means.
**Figure 20** - **Compilation of different analyte TCRsp presenting eTPC**
   The **eTPCS** begins with the **eTPC** and uses a donor vector(s) to create cells expressing analyte **TCRsp,** or single analyte TCR chains. An **eTPC** presenting **TCRsp** is termed **eTPC-t,** and may be created by introduction of two **complimentary TCR chain** encoding ORFs to the **eTPC (step i).** An **eTPC** expressing a single **analyte TCR chain** alone is termed an **eTPC-x,** wherein **a,** and may be created by introduction of a single **TCR chain** encoding ORF(s) to the **eTPC (step ii).** A **eTPC-t** may alternatively be created from an eTPCx, wherein a second **complimentary TCR chain** encoding ORF is introduced to an existing **eTPC-x (step iii).**
**Figure 21** - **Compilation of a eTPC system with a one-step and one-vector format eTPC 2A** contains distinct genomic receiver site. The genetic donor vectors **2C** is coupled to **2D.** Donor vector **2C** encodes a **TCR chain pair.** The **eTPC 2A** further contains a **TCR signal response element 2F.** The **eTPC 2A** is combined with donor vector **2C.** The resulting cell has insert **2C** exchanged to the **2B** genomic receiver site to create site **2C'** and deliver the two ORFs for a **TCR chain pair.** This cell is capable of presenting a **TCRsp** at the surface, and is thus designated a **eTPC-t.**
**Figure 22** - **Compilation of a eTPC-t in one step with one vector and an unused receiver site**
   **eTPC 2A** contains distinct genomic receiver sites **2B** and **2D.** The genetic donor vectors **2C'** is coupled to **2D.** Donor vector **2C'** encodes a **TCR chain pair.** The **eTPC 2A** further contains a **TCR signal response element 2F.** The **eTPC 2A** is combined with donor vector **2C'.** The resulting cell has insert **2C'** exchanged to the **2B** genomic receiver site to create site **2B'** and deliver the two ORFs for a **TCR chain pair.** Genomic receiver site **2D** remains unused. This cell is capable of presenting a **TCRsp** at the surface, and thus designated a **eTPC-t.**
**Figure 23** - **Compilation of a eTPC-t in one step with two vectors and two integration couples**
   **eTPC 2A** contains distinct genomic receiver sites **2B** and **2D.** The **eTPC 2A** further contains a **TCR signal response element 2F.** Distinct genetic donor vectors **2C'** and **2E'** are independently coupled to **2B** and **2D,** respectively. Donor vector **2C'** encodes a single **TCR chain,** and donor vector **2E'** encodes a second reciprocal **TCR chain.** The **eTPC 2A** is combined with donor vectors **2C'** and **2E'.** The resulting cell has insert **2C** exchanged to the **2B** genomic receiver site to create site **2B'** and deliver an ORF for a first **TCR chain.** In addition, the resulting cell line has insert **2E'** exchanged to the **2D** genomic receiver site to create site **2D'** and deliver an ORF for a second **TCR chain.** This cell is capable of presenting a **TCRsp** at the surface, and is thus designated a **eTPC-t.**
**Figure 24** - **Compilation of a eTPC-x in one step with one vector and an unused receiver site**
   **eTPC 2A** contains distinct genomic receiver sites **2B** and **2D.** The genetic donor vectors **2C'** is coupled to **2D.** Donor vector **2C'** encodes a single **TCR chain.** The **eTPC 2A** further contains a **TCR signal response element 2F.** The **eTPC 2A** is combined with donor vector **2C'.** The resulting cell has insert **2C'** exchanged to the **2B** genomic receiver site to create site **2B'** and deliver a single TCR chain ORF. Genomic receiver site **2D** remains unused. This cell expresses only a **single TCR chain** and is thus designated a **eTPC-x.**
**Figure 25** - **Compilation of a eTPC-t in two steps with two vectors**
   **eTPC 2A** contains distinct genomic receiver sites **2B** and **2D.** The **eTPC 2A** further contains a **TCR signal response element 2F.** Distinct genetic donor vectors **2C'** and **2E'** are independently coupled to **2B** and **2D,** respectively. Donor vector **2C'** encodes a single **TCR chain,** and donor vector **2E'** encodes a second reciprocal **TCR chain.** In **STEP 1** a **eTPC 2A** is combined with donor vector **2C'.** The resulting cell has insert **2C'** exchanged to the **2B** genomic receiver site to create site **2B'** and deliver an ORF for a first **TCR chain.** This cell expresses only a **single TCR chain** and is thus designated a **eTPC-x.** Genomic receiver site **2D** remains unused. In **STEP 2,** the **eTPC-x** is combined with donor vector **2E'.** The resulting cell has insert **2E'** exchanged to the **2D** genomic receiver site to create site **2D'** and deliver an ORF for a second complementary **TCR chain.** This cell is capable of presenting a **TCRsp** at the surface, and is thus designated a **eTPC-t.**
**Figure 26** - **Shotgun compilation of an eTPC-t pool from an eTPC with paired analyte TCR chains in single replicate vector to express discrete TCR chain pairs from a selected TCR chain pair library**
   **eTPC 2A** contains a genomic receiver site **2B.** The genetic donor vectors **2C'** is coupled to **2B.** Donor vector **2C' i, 2C' ii** and **2C' iii** encode a distinct **TCR chain pair** and constitutes a mixed vector library of discrete **TCR chain pairs.** The **eTPC 2A** further contains a **TCR signal response element 2F.** The **eTPC 2A** is combined with donor vectors **2C' i, 2C' ii** and **2C' iii** simultaneously. The resulting cell pool has insert **2C** exchanged to the **2B** genomic receiver site multiple independent instances to create site **2B' i, 2B' ii** and **2B' iii** delivering two ORFs for each discrete **TCR chain pair** contained in the initial vector library. This **eTPC-t** cell pool comprises a mixed population of three distinct cell clones each expressing a distinct **TCR chain pairs,** denoted **TCRsp i, ii** and **iii,** forming an **eTPC-t** pooled library.
**Figure 27** - **Shotgun compilation of an eTPC-t pool from an eTPC with paired analyte TCR chains in single replicate vector to express discrete TCR chain pairs from a selected TCR chain pair library and an unused receiver site**
   **eTPC 2A** contains distinct genomic receiver sites **2B** and **2D.** The genetic donor vectors **2C'** is coupled to **2B.** Donor vector **2C' i, 2C' ii** and **2C' iii** encode a distinct **TCR chain pair** and constitutes a mixed vector library of discrete **TCR chain pairs.** The **eTPC 2A** further contains a **TCR signal response element 2F.** The **eTPC 2A** is combined with donor vectors **2C' i, 2C' ii** and **2C' iii** simultaneously. The resulting cell pool has insert **2C** exchanged to the **2B** genomic receiver site multiple independent instances to create site **2B' i, 2B' ii** and **2B' iii** delivering two ORFs for each discrete **TCR chain pair** contained in the initial vector library. This **eTPC-t** cell pool comprises a mixed population of three distinct cell clones each expressing a distinct **TCR chain pairs,** denoted **TCRsp i, ii** and **iii,** forming an **eTPC-t** pooled library. Genomic receiver site **2D** remains unused.
**Figure 28** - **Shotgun compilation of an eTPC-t pool from an eTPC with two vectors to express random combinations of paired TCR chain pairs from a selected single TCR chain library**
   **eTPC 2A** contains distinct genomic receiver sites **2B** and **2D.** Distinct genetic donor vectors **2C'** and **2E'** are independently coupled to **2B** and **2D,** respectively. Donor vectors **2C'** i and **2C' ii** each encode a single **TCR chain,** and donor vectors **2E' i** and **2E' ii** each encode a reciprocal single **TCR chain.** The **eTPC 2A** further contains a **TCR signal response element 2F.** The **eTPC 2A** is combined with donor vectors **2C' i, 2C' ii, 2E'** i and **2E' ii** simultaneously. The resulting cell pool has insert **2C' i** or **2C' ii** exchanged to the **2B** genomic receiver site multiple independent instances to create sites **2B'** i and **2B' ii,** each delivering a single ORF for a **TCR chain.** The resulting cell pool further has insert **2E** i or **2E ii** exchanged to the **2D** genomic receiver site multiple independent instances to create sites **2E' i** and **2E' ii,** each delivering a single ORF for a **TCR chain** reciprocal to those at sites **2C'i** and **2C'ii.** The resulting **eTPC-t** cell pool comprises a mixed population of four distinct cell cohorts each expressing a discrete randomised **TCRsp** at the surface comprised of one of each reciprocal **TCR chain** contained in the initial vector library.
**Figure 29** - **Shotgun compilation of an eTPC-t pool from an eTPC-x with unpaired analyte TCR chains to express random combinations of paired TCR chain pairs from a selected single TCR chain library**
   **eTPC-x** contains the exchanged genomic receiver site **2B'** expressing a single **TCR chain** and the distinct genomic receiver site **2D.** Distinct genetic donor vectors **2E' i** and **2E' ii** are coupled to **2D,** respectively. Donor vectors **2E' i** and **2E' ii** each encode a single **TCR chain.** The **eTPC-x** further contains a **TCR signal response element 2F.** The **eTPC-x** is combined with donor vectors **2E' i** and **2E' ii** simultaneously. The resulting cell pool has insert **2E' i** or **2E' ii** exchanged to the **2D** genomic receiver site multiple independent instances to create sites **2E i** and **2E'ii,** each delivering a single ORF for a **TCR chain.** The resulting **eTPC-t** cell pool comprises a mixed population of 2 distinct cell cohorts expressing a discrete **TCRsp** at the surface comprised of the **TCR chain** expressed from **2B'** paired with one of each **TCR chain** contained in the initial vector library.
**Figure 30** - **Operation of a combined eAPC:eTPC system showing possible eTPC-t-output states**
   The analyte **eAPC** contains sites **1C'** and **1E'** integrated with one ORF each to encode one **aAPX** and one **aAM,** with the **aAM** loaded as cargo in **aAPX** at the cell surface. The analyte **eTPC** contains sites **2C'** and **2E'** each integrated with one ORF encoding a reciprocal **TCRsp** at the surface. The **eTPC-t** further contains a **TCR signal response element 2F.** When **eTPC-t and eAPC-pa** populations are contacted, four **eTPC-t** response states can be achieved, one negative and three positive. The negative state is the resting state of the **eTPC-t,** with no signal strength at the **2F** element, denoting failure of the **eAPC aAPX:aAM** complex to stimulate the **eTPC-t** presented chain pair. Three positive states show increasing signal strength from the **2F.** States **2F'+, 2F'++** and **2F'+++** denote low, medium and high signal strength, respectively. The gene product **of 2F** denoted as hexagons accumulates to report signal strength of each cell state, as denoted by darker shading of the cells. This indicates a graded response of analyte **TCRsp** expressed by **eTPC-t** population towards analyte **aAPX:aAM** presented by the **eAPC-pa.**
**Figure 31** - **Operation of a combined eAPC:eTPC system showing possible eAPC-pa output states**
   The analyte **eAPC-pa** contains sites **1C'** and **1E'** integrated with one ORF each to encode one **aAPX** and one **aAM,** with the **aAM** loaded as cargo in **aAPX** at the cell surface. The analyte **eTPC** contains sites **2C'** and **2E'** each integrated with one ORF encoding a reciprocal **TCRsp** at the surface. The **eTPC-t** further contains a **TCR signal response element 2F.** When analyte **eTPC** and **eAPC-pa** populations are contacted, four **eAPC** response states can be achieved, one negative and three positive. The negative state is the resting state of the analyte **eAPC,** denoting failure of the **TCRsp** chain pair to stimulate the **aAPX:aAM** complex presented by the analyte **eAPC.** Three positive states show increasing signal strength from the contacted **aAPX:aAM.** The reported signal strength of each cell state, is denoted by *, ** and **, and also denoted by darker shading of the cells. This indicates a graded response of analyte **aAPX:aAM** towards the analyte **TCRsp** chain pair.
**Figure 32** - **Combined operation of the two-part analyte APC:eTPC system to identify TCR chain pairs reactive with analyte aAPX:aAM from a library of eTPC-t expressing discrete analyte TCR chain pairs**
   The **eTPC-t** pool contains cells harboring sites **2C' i, ii** or **ii,** wherein each integrated with two ORFs encoding a reciprocal **TCR chain pair,** and thus each cell cohort in the population expresses a discrete **TCRsp** at the surface. The **eTPC-t** further contains a **TCR signal response element 2F.** The analyte **eAPC** contain sites **1C'** and **1E'** integrated with a distinct set of ORF to encode one **aAPX** and one **aAM,** with the **aAM** loaded as cargo in **aAPX** at the cell surface. In the present example, only the **TRC chain pair** expressed from **2C'** i is specific for the **aAPX:aAM** presented by the **analyte APC,** such that when **eTPC-t** pool and **analyte APC** population are contacted, only the cell cohort of the **eTPC-t** that bears **2C' i** reports **TCRsp** engagement through state **2F'.**
**Figure 33** - **Combined operation of the two-part eAPC:eTPC system to identify aAM reactive with analyte eTPC from a library of analyte eAPC expressing discrete analyte aAPX:aAM complexes.**
   The analyte **eAPC** contain sites **1C'** and **1E'** integrated with a distinct set of ORF each to encode one **aAPX** and one **aAM,** with the **aAM** loaded as cargo in **aAPX** at the cell surface. The analyte **eTPC** contain the exchanged genomic receiver site **2C'** expressing a **TCRsp** at the surface. It further contains a **TCR signal response element 2F.** In the present example, only the complex **aAPX:aAM i** is specific for the **TCR** presented by the analyte **eTPC,** such that when analyte **eAPC pool and** analyte **eTPC** population are contacted, only the cell cohort expressing **aAM i** express a distinct signal *.
**Figure 34** - **Generation of a eAPC-p + aAM from a eAPC-p and the addition of a soluble, presentable antigen aAM**
   **eAPC-p** contains the exchanged genomic receiver site **1B'** expressing an **aAPX.** A soluble, directly presentable antigen **aAM** is combined with the **eAPC-p.** This results in the formation of the **aAPX:aAM** complex on the cell surface and the generation of a **eAPC-p + aAM.**
**Figure 35** - **Generation of an eAPC-p + aAM from an eAPC-p and soluble aAM**
   **eAPC-p** contains the exchanged genomic receiver site **1B'** expressing an **aAPX.A** soluble antigen aAM is combined with the **eAPC-p,** this results in expression of the **aAPX** on the cell surface, the presence of **aAM** intracellularly, and thus loading of the **aAM** as cargo in the **aAPX** in formation of the **aAPX:aAM** complex on the cell surface and the generation of a **eAPC-p + aAM.**
**Figure 36** - **Identification of the aAM presented by an eAPC-p + aAM through forced release of the aAM**
   **eAPC-p + aAM** contains the exchanged genomic receiver site **1B'** expressing an **aAPX** as well as internalized **aAM** that is presented on the surface as **aAPX:aAM** complex.The **aAM** is released from the **aAPX:aAM** surface complex through incubation and the released **aAM** available for identification.
**Figure 37** - **Identification of the aAM presented by an eAPC-p + aAM through capture of the aAPX:aAM complex**
   **eAPC-p + aAM** contains the exchanged genomic receiver site **1B'** expressing an **aAPX** as well as internalized aAM that is presented on the surface as **aAPX:aAM** complex.The **aAPX:aAM** surface complex is captured for identification of loaded **aAM.**

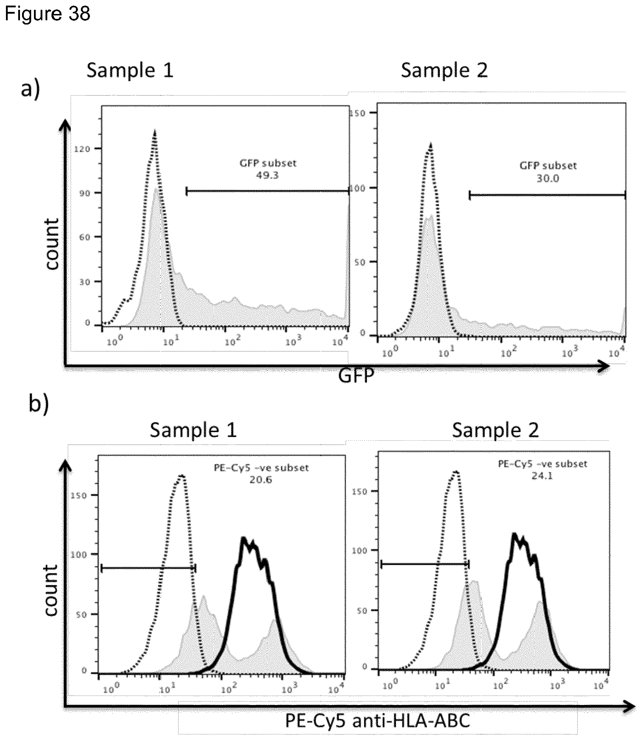

**Figure 38** - **Selection of cells with targeted mutagenesis of the HLA-A, HLA-B and HLA-C loci in HEK239 cell line**
   **a)** GFP fluorescence signal in two independent cell populations 48 hours after transfection with plasmids encoding Cas9-P2A-GFP and gRNAs targeting the HLA-A, HLA-B and HLA-C loci (grey histogram) compared to HEK293 control cells (dashed lined histogram). Cells that had a GFP signal within the GFP subset gate were sorted as a polyclonal population. **b)** Cell surface HLA-ABC signal observed on the two sorted polyclonal populations when labelled with a PE-Cy5 anti-HLA-ABC conjugated antibody (grey histogram). Single cells that showed a low PE-Cy5 anti-HLA-ABC signal and were displayed within the sort gate were sorted to establish monoclones. Non-labelled HEK293 cells (dashed line histogram) and PE-Cy5 anti-HLA-ABC labelled HEK293 cells (full black lined histogram) served as controls.

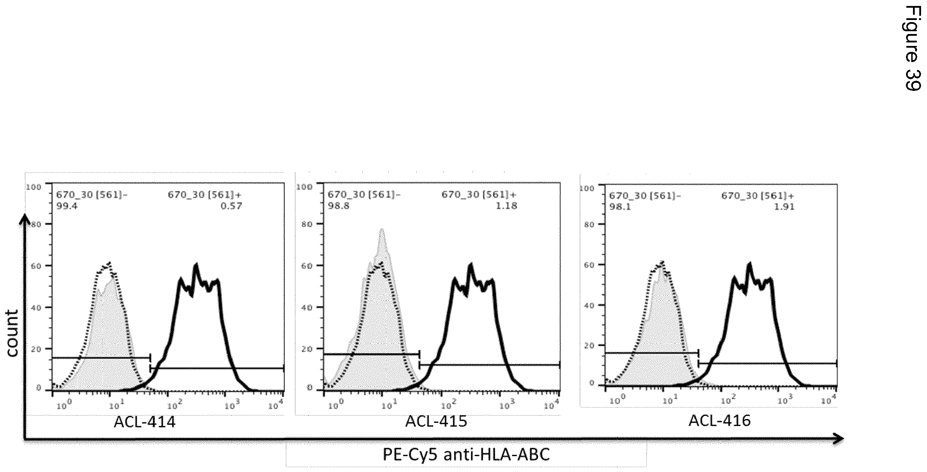

**Figure 39** - **Phenotypic analysis of HLA-ABC^{null} monoclones:** Monoclone populations were stained with the PE-Cy5 anti-HLA-ABC conjugated antibody, and were analysed by flow cytometry (grey histogram). Non-labelled HEK293 cells (dashed lined histogram) and PE-Cy5 anti-HLA-ABC labelled HEK293 cells (full black lined histogram) served as controls. All three monoclone lines showed a fluorescent signal matching to non-labelled controls demonstrating that each line lacked HLA-ABC surface expression.

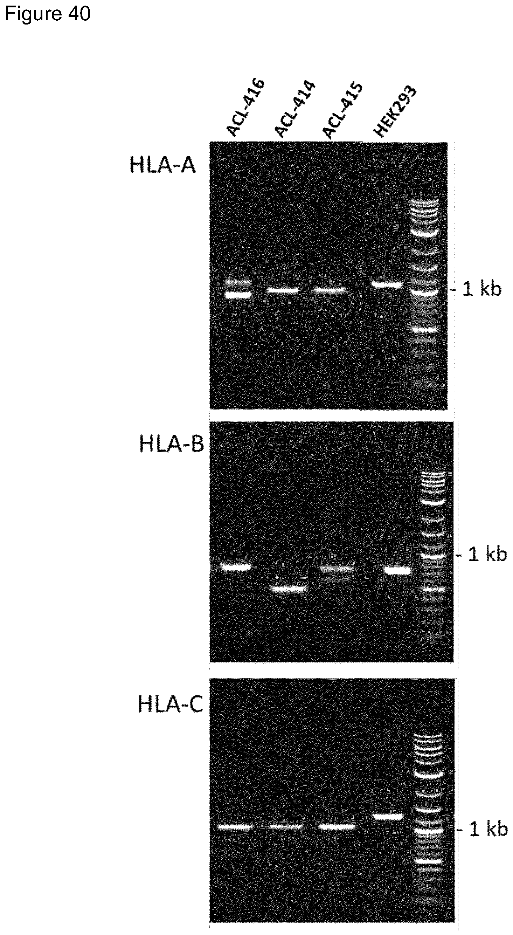

**Figure 40** - **Genetic characterization of a selection of monoclones lacking surface HLA-ABC expression demonstrating a genomic deletion in the targeted HLA alleles.** PCR amplicons were generated with primers that spanned the gRNA genomic target sites of a specific HLA alleles and their size determined by electrophoresis. The expected size of the wild type HLA-A amplicon is 1067 bp, HLA-B amplicon is 717 bp and HLA-C amplicon is 1221 bp.

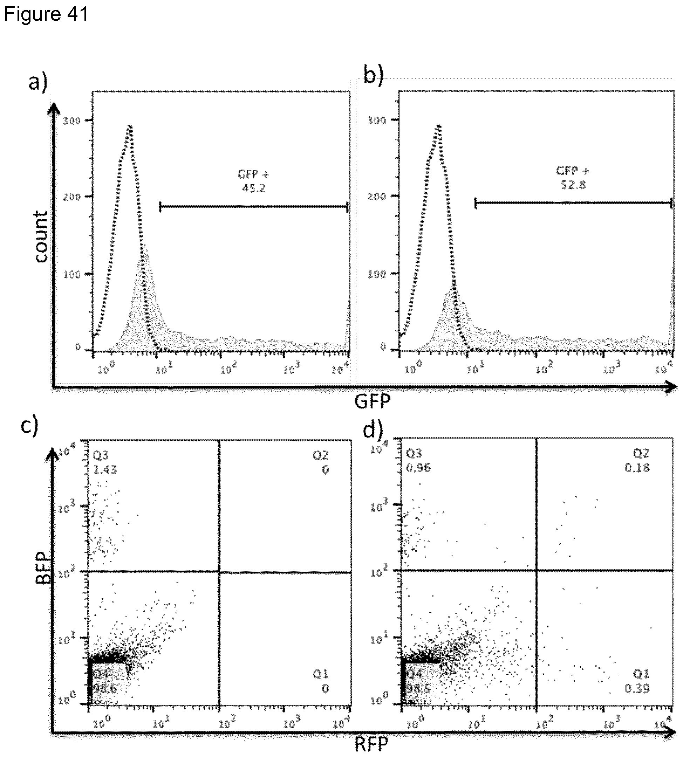

**Figure 41** - **Selection of cells with targeted genomic integration of synthetic Component B with or without synthetic Component D**
   **a)** GFP fluorescence signal 48 hours after transfection with plasmids encoding Cas9-P2A-GFP, gRNAs targeting the AAVS1 locus and component B genetic elements flanked by AAVS1 left and right homology arms (grey histogram). HEK293 cells server as a GFP negative control (dashed line histogram). Cells that had a GFP signal within the GFP+ gate were sorted as a polyclonal population. **b)** GFP fluorescence signal 48 hours after transfection with plasmids encoding Cas9-P2A-GFP, gRNAs targeting the AAVS1 locus and component B and D, both flanked by AAVS1 left and right homology arms (grey histogram). HEK293 cells server as a GFP negative control (dashed line histogram). Cells that had a GFP signal within the GFP+ gate were sorted as a polyclonal population **c)** Maintained BFP but no detectable RFP signal observed in the D1 sorted polyclonal population. Single cells that showed high BFP signal in quadrant Q3 were sorted to establish eAPC containing synthetic component B monoclones. **d)** Maintained BFP and RFP signal observed in the D2 sorted polyclonal population. Single cells that showed high BFP and RFP signals in quadrant Q2 were sorted to establish eAPC monoclones containing synthetic component B and synthetic component D.

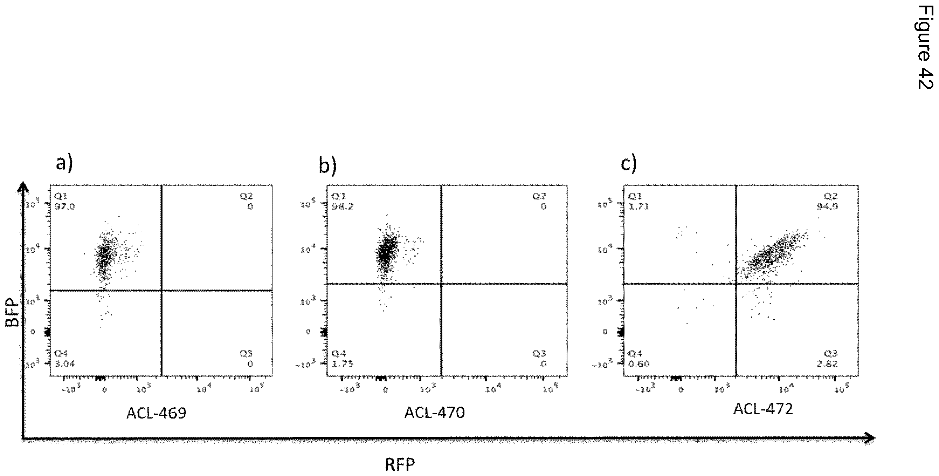

**Figure 42** - **Phenotypic analysis of eAPC monoclones**
   **a and b)** Monoclone populations that display maintained BFP expression suggest the integration of synthetic component B. **c)** Monoclone populations that display maintained BFP and RFP expression suggest the integration of both synthetic component B and synthetic component D.

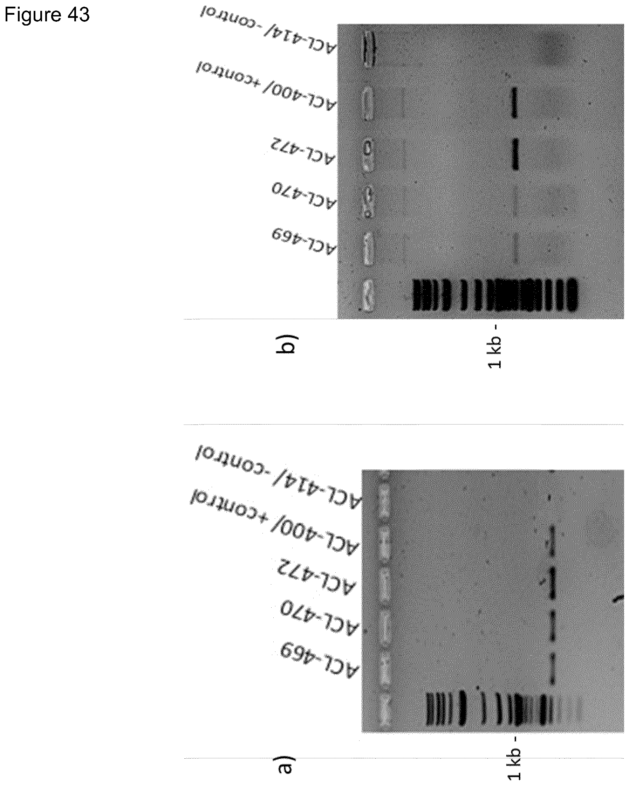

**Figure 43** - **Genetic characterization of a selection of monoclones for integration of Component B or Component B and D in the AAVS1 locus.**
   **a)** PCR amplicons were generated with primers that prime within component B and/or D and size determined by electrophoresis. The expected size of a positive amplicon is 380bp indicating stable integration of component B and/or D. **b)** PCR amplicons were generated with primers that prime on AAVS1 genomic sequence distal to region encoded by the homologous arms and the SV40 pA terminator encoded by component B and/or D and size determined by electrophoresis. The expected size of a positive amplicon is 660 bp indicating integration of component B and/or D occurred in the AAVS1 site.

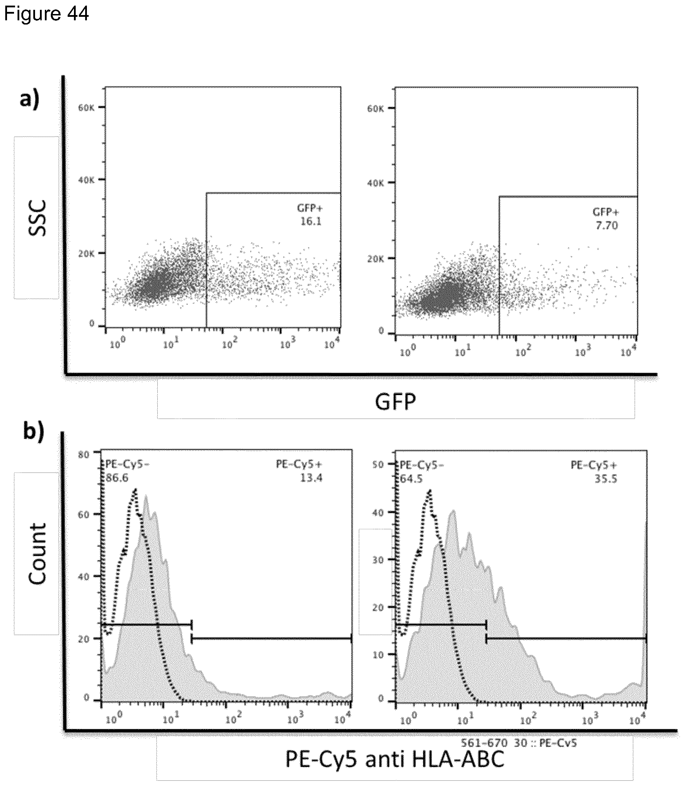

**Figure 44** - **Selection of cells with targeted genomic integration of component C' into component B**
   **a)** GFP fluorescence signal 48 hours after transfection with plasmids encoding Cas9-P2A-GFP, gRNAs targeting the AAVS1 locus and component C'^{HLA-A*24:02} (left panel) or component C'^{HLA-B*-07:02} (right panel). Cells that had a GFP signal within the GFP+ gate were sorted as a polyclonal population ACL-303 or ACL-305.
   **b)** Analyte HLA cell surface expression observed on the two sorted polyclonal populations when labelled with a PE-Cy5 anti-HLA-ABC conjugated antibody (grey histogram). Single cells that showed a high PE-Cy5 anti-HLA-ABC signal and were displayed within the right sort gate were sorted to establish monoclones. Signal detected from PE-Cy5 anti-HLA-ABC labelled ACL-128, the HLA-ABC^{null} and HLA-DR,DP,DQ^{null} eAPC cell line (dashed line histogram) served as controls.

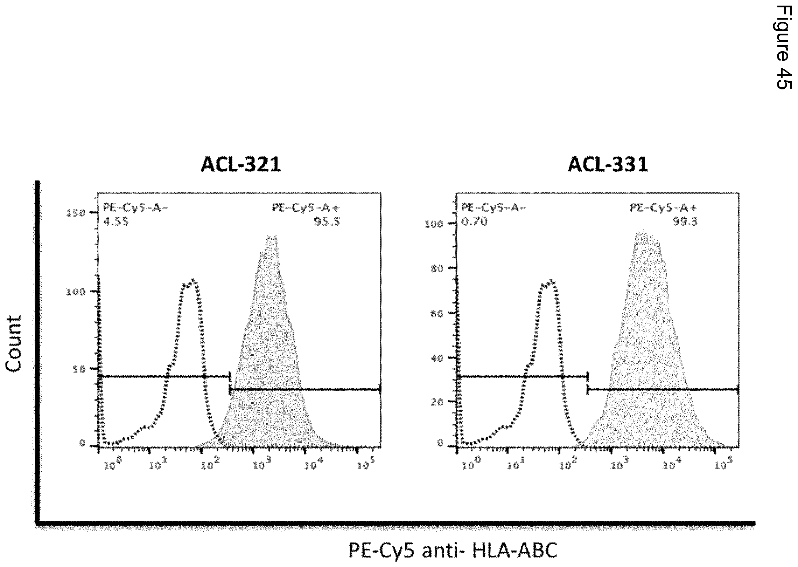

**Figure 45** - **Phenotypic analysis of eAPC-p monoclones expressing analyte HLA class I protein on the cell surface**
   Monoclone populations were stained with the PE-Cy5 anti-HLA-ABC conjugate antibody, and were analysed by flow cytometry (grey histogram). ACL-128, the HLA-ABC^{null} and HLA-DR,DP,DQ^{null} eAPC cell line (dashed line histogram) served as controls. ACL-321 and ACL-331 monoclone cell lines showed a stronger fluorescent signal compared to the HLA-ABC^{null} and HLA-DR,DP,DQ^{null} eAPC cell line control, demonstrating that each line expresses their analyte aAPX, HLA-A*24:02 or HLA-B*-07:02 ORF, respectively, and therefore are eAPC-p cell lines.

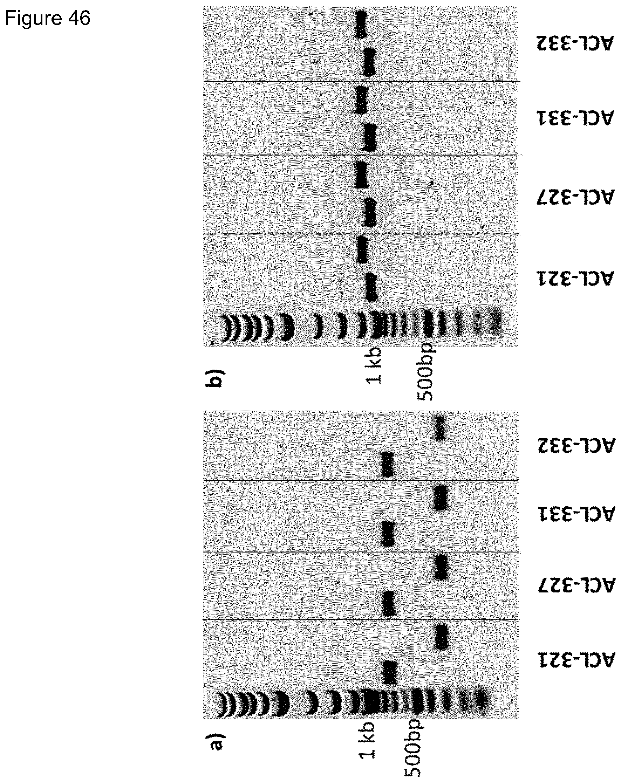

**Figure 46** - **Genetic characterization of a selection of monoclones demonstrating that their genomes integrated component C', and that the integration occurred in the AAVS1 genomic receiver site, generating component B'**
   **a)** PCR amplicons confirm the presence of HLA insert, a band of 810 bp indicated correct CMV promoter amplicon and 380 bp is the amplicon generated from SV40pA terminator. **b)** PCR amplicons were generated with two set of primers that primed on AAVS1 genomic sequence distal to region encoded by the homologous arms and a primer that is unique to the SV40 pA terminator linked to the analyte HLA ORF. The expected size of a positive amplicon 1 kb and 1.1 kb indicate generation of component B'.

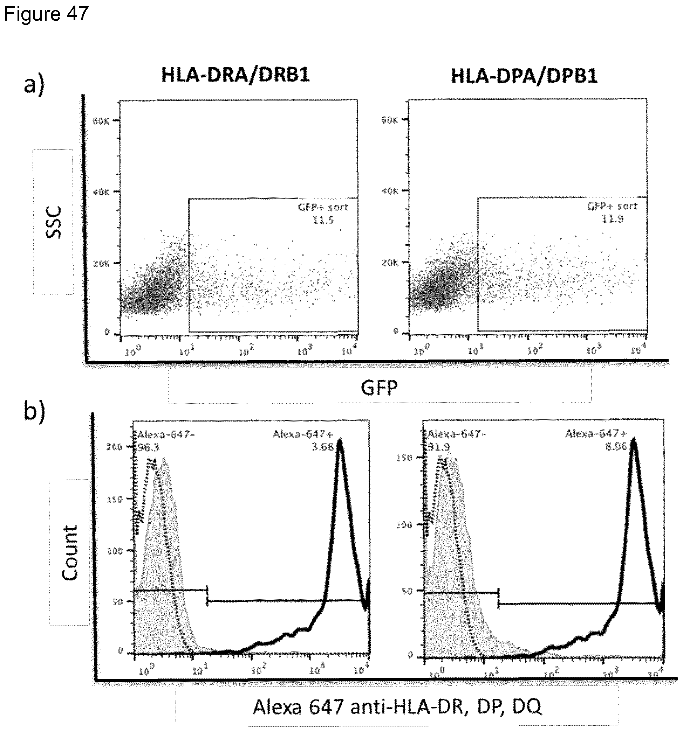

**Figure 47** - **Selection of cells with targeted genomic integration of component C' into component B**
   **a)** GFP fluorescence signal 48 hours after transfection with plasmids encoding Cas9-P2A-GFP, gRNAs targeting the AAVS1 locus and component C'^{HLA-DRA*01:01/HLA-DRB1*01:01} (left panel) or component C'^{HLA-DPA1*01:03/HLA-DPB1*04:01} (right panel). Cells that had a GFP signal within the GFP+ gate were sorted as a polyclonal population.
   **b)** Analyte HLA cell surface expression observed on the two sorted polyclonal populations when labelled with an Alexa 647 anti-HLA-DR,DP,DQ conjugated antibody (grey histogram). Single cells that showed a high Alexa 647 anti-HLA-ABC signal and were displayed within the right sort gate were sorted to establish monoclones. Signal detected from Alexa 647 anti-HLA-ABC labelled ACL-128 (HLA-ABC^{null} and HLA-DR,DP,DQ^{null} eAPC cell line) (dashed line histogram) and ARH wild type cell line (full black lined histogram) served as controls
**Figure 48** - **Phenotypic analysis of eAPC-p monoclones expressing analyte HLA class II protein on the cell surface**
   Monoclone populations were stained with a Alexa 647 anti-HLA-DR,DP,DQ conjugated antibody, and analysed by flow cytometry (grey histogram). ACL-128 (HLA-ABC^{null} and HLA-DR,DP,DQ^{null} eAPC cell line) (dashed line histogram) and ARH wild type cell line (full black lined histogram) served as controls. ACL-341 and ACL-350 monoclone cell lines showed a stronger fluorescent signal compared to the HLA-ABC^{null} and HLA-DR,DP,DQ^{null} eAPC cell line control, demonstrating that each line expressed their analyte aAPX, HLA-DRA*01:01/HLA-DRB1*01:01 or HLA-DPA1*01:03/HLA-DPB1*04:01, respectively, and therefore are eAPC-p cell lines.

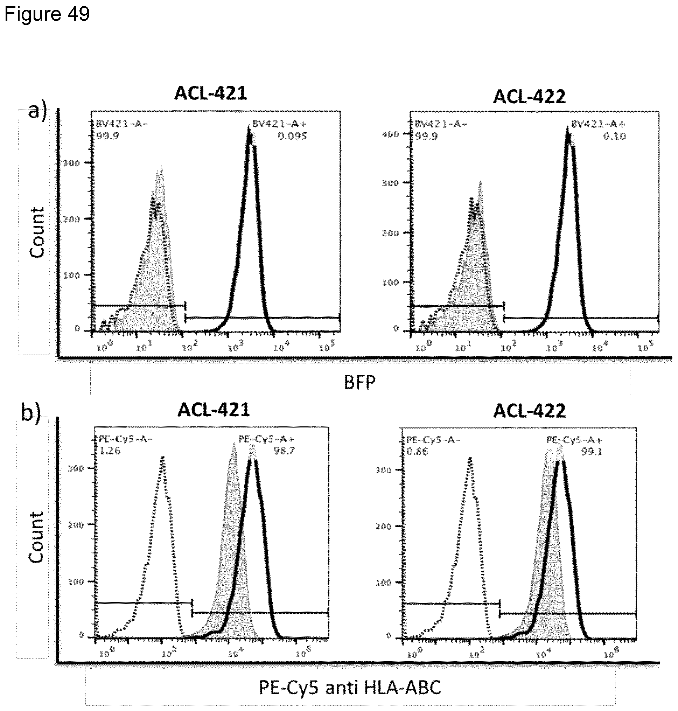

**Figure 49** - **eAPC-p monoclones generated by RMCE integration of analyte HLA class I protein**
   **a)** eAPC-p monoclone populations ACL-421 and ACL-422 lost BFP fluorescence (grey histogram). Their parent eAPC cell line ACL-385 (full black line histogram) and the BFP negative ARH wild type cell line (dash line histogram) served as a control
   **b)** eAPC-p monoclone populations ACL-421 and ACL-422 gained HLA-A*02:01 expression when stained with the PE-Cy5 anti-HLA-ABC conjugate antibody (grey histogram). Their parent ACL-385 HLA-ABC^{null} and HLA-DR,DP,DQ^{null} eAPC cell line (dash line histogram) and ARH wild type cell line (full black line histogram) served as negative and positive PE-Cy5 anti-HLA-ABC labeling control, respectively.
   These results strongly indicated a successful RMCE occurred between the BFP ORF and HLA-A*02:01 ORF in both ACL-421 and ACL-422 cell lines.

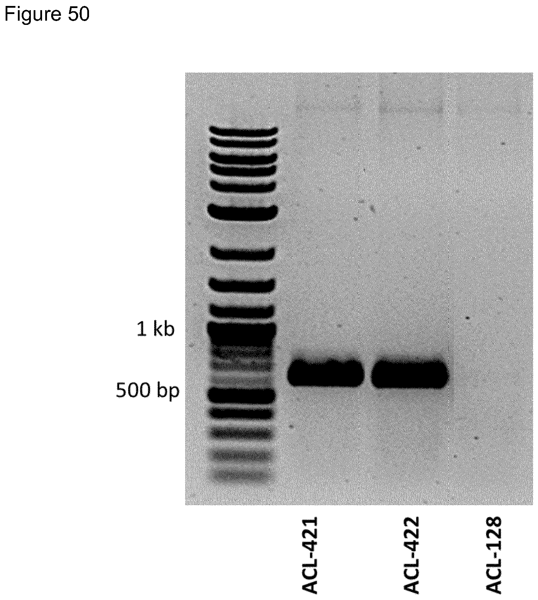

**Figure 50** - **Genetic characterization of a selection of monoclones confirmed HLA-A*02:01 integration by RMCE**
   An amplicon of 630 bp indicated presence of HLA-A2 in monclones ACL-421 and 422 but not in the control line, ACL-128.

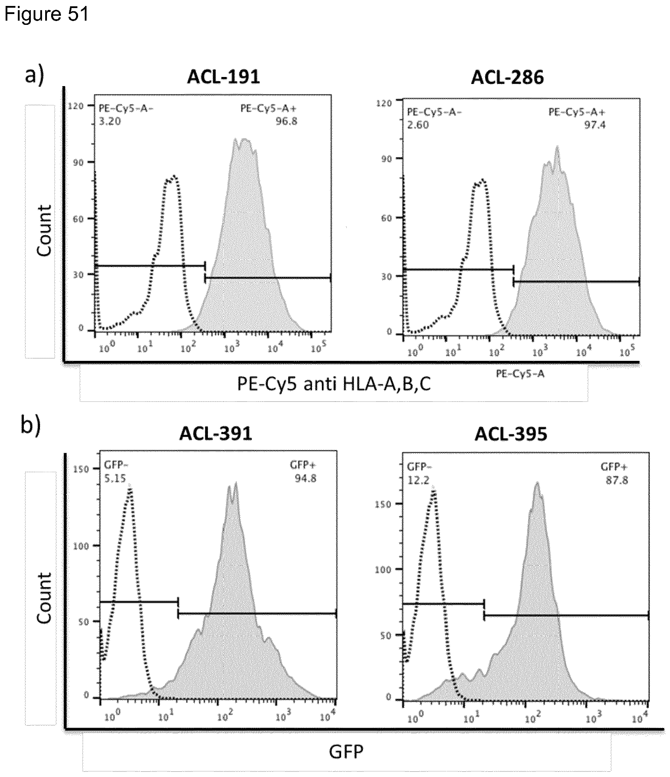

**Figure 51** - **Phenotypic analysis of eAPC-pa monoclones expressing analyte HLA class I protein on the cell surface and aAM**
   **a)** eAPC-p Monoclone populations were stained with the PE-Cy5 anti-HLA-ABC conjugated antibody, and were analysed by flow cytometry (grey histogram). ACL-128, the HLA-ABC^{null} and HLA-DR,DP,DQ^{null} eAPC cell line (dashed line histogram) served as control. ACL-321 and ACL-331 monoclone cell lines showed stronger fluorescent signal compared to controls demonstrating that each line expressed their analyte aAPX, HLA-A*02:01 or HLA-B*35:01 ORF, respectively, and therefore were eAPC-p cell lines.
   **b)** eAPC-pa Monoclone populations were assessed for GFP fluorescence by flow cytometry (grey histogram). ACL-128, the HLA-ABC^{null} and HLA-DR,DP,DQ^{null} eAPC cell line (dashed line histogram) served as control. ACL-391 and ACL-395 monoclone cell lines showed a stronger fluorescent signal compared to controls demonstrating that each line expresses analyte aAM selection marker and therefore inferred aAM expression, in a cell line which also expressing HLA-LA-A*02:01 or HLA-B*35:01 ORF, respectively. Therefore ACL-391 and ACL-395 were eAPC-pa lines.

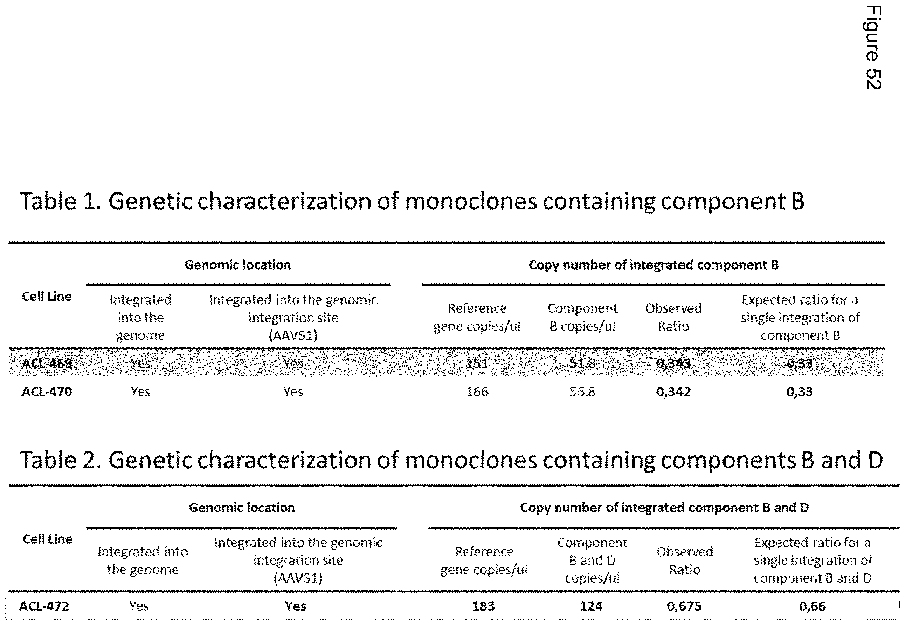

**Figure 52** - **Genetic characterisation of monoclones containing components 1D and/or 1B**
   Two tables are presented summarising the genetic characterisation of eAPC generated to contain Component 1B, or Component 1B and 1D, respectively.

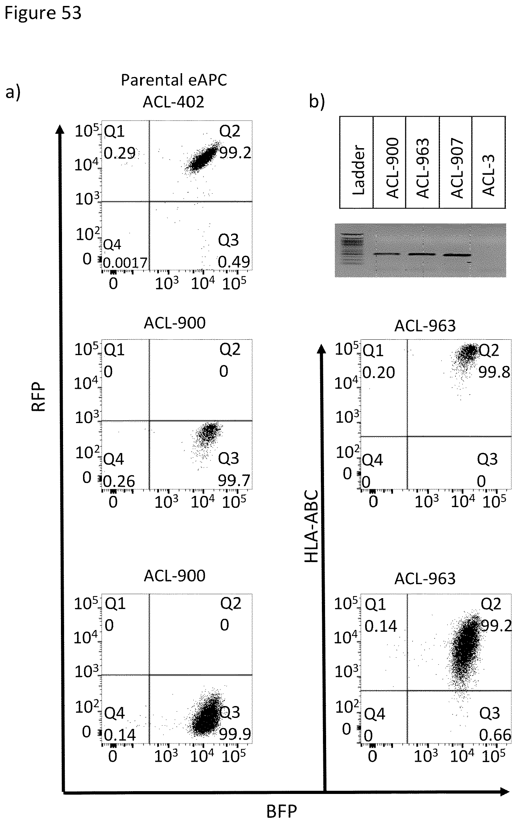

**Figure 53** **- An eACP-p constructed in one step wherein Component 1C' encoded a single HLAI ORF.**
   An eAPC-p was created through RMCE by electroporation of the cell line ACL-402 with the plasmid that encodes expression of the Tyr-recombinase, Flp (V4.1.8), together with one **Component 1C'** plasmid encoding an aAPX, selected from either HLA-A*02:01 (V4.H.5 or HLA-A*24:02 (V4.H.6). At 10 days post electroporation, individual cells positive for HLAI surface expression and diminished fluorescent protein signal, RFP, encoded by **Component 1B** selection marker, were sorted. Resulting monoclonal eAPC-p lines were analysed by flow cytometry in parallel with the parental eAPC line, and two examples are presented **a)** Individual outgrown monoclone lines (ACL-900 and ACL-963) were analysed by flow cytometry for loss of RFP, presence of BFP and gain of HLA-ABC (aAPX). Left-hand plots display BFP vs RFP, the parental cell has both BFP and RFP (Q2, top plot, 99.2%), whereas ACL-900 (Q3, middle plot, 99.7%) and ACL-963 (Q3, bottom plot, 99.9%) both lack RFP signal, indicating integration couple between Component 1B/1C' has occured. Right-hand plots display BFP vs HLA-ABC (aAPX), wherein both ACL-900 (Q2, top plot, 99.2%) and ACL-963 (Q2, bottom plot, 99.2%) show strong signal for HLA-ABC (aAPX), further reinforcing that 1B/1C' integration. Critically, both ACL-900 and ACL-963 have strong BFP signal, indicating that Component 1D remains open and isolated from the Component 1B/1C' integration couple. **b)** To further characterize ACL-900 and ACL-963, and a third eAPC-p not presented in a) ACL-907, genomic DNA was extracted and PCR conducted using primers that target adjacent and internal of Component 1B' (Table 5, 8.B.3, 15.H.2), thereby selectively amplifying only successful integration couple events. Comparison is made to an unmodified parental line, ACL-3 wherein the Component 1B is lacking. Amplicon products specific for Component 1B' were produced for all three eAPC-p monoclones whereas no product was detected in the ACL-3 reaction, confirming the specific integration couple event between Component 1B and Component 1C' had occurred.

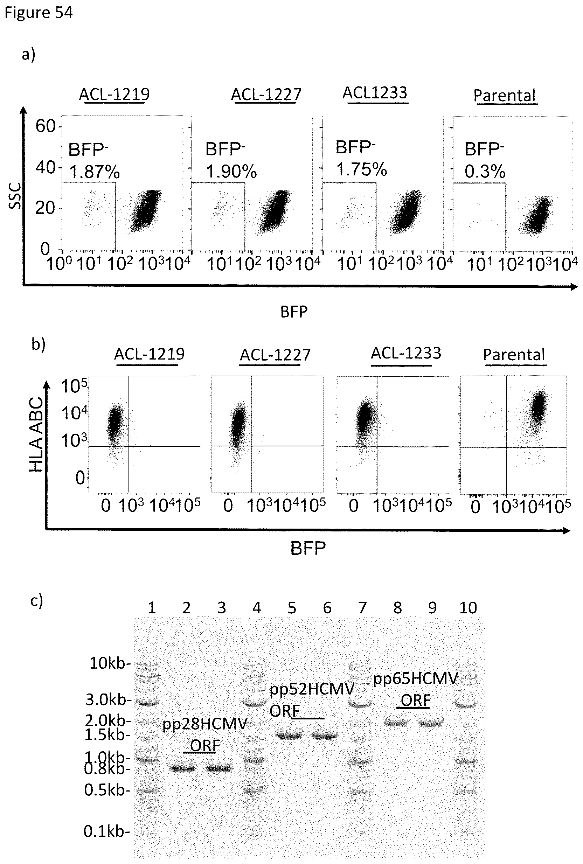

**Figure 54** - **An eAPC-pa constructed from eAPC-p in one step, wherein Component 1D' encodes a single analyte antigen molecule (aAM) ORF.**
   Multiple eAPC-pa were constructed from a parental eAPC-p (ACL-905) in parallel, wherein the genomic receiver site, **Component 1D,** is targeted for integration by a primed genetic donor vector, **Component 1E',** comprising of a single ORF that encodes an aAM. The eAPC-p (ACL-900, example 8) was independently combined with a vector encoding expression of the RMCE recombinase enzyme (Flp, V4.1.8) and each **Component 1E'** of either V9.E.6, V9.E.7, or V9.E.8 by electroporation. At 10 days post electroporation, individual eAPC-pa were selected and single cell sorted (monoclones) based on diminished signal of the selection marker of integration BFP, encoded by **Component 1**D. Resulting monoclonal eAPC-pa lines were analysed by flow cytometry in parallel with the parental eAPC line, and three examples are presented. In addition, resulting monoclones were also genetically characterized to confirm the integration couple event. a) Monoclones for eAPC-pa, ACL-1219, ACL-1227 and ACL-1233, were analysed and selected by flow cytometry for loss of BFP signal and retention of the HLA-ABC signal. Plots of BFP vs SSC are displayed with a BFP- gate. An increase in the number of BFP- events compared to parental eAPC-p is observed, indicating that an integration couple between Component 1D/1E' has occurred. Single cells from the BFP- gate were selected, sorted and outgrown. **b)** Selected monoclones of ACL-1219, ACL-1227, ACL-1233 were analysed by flow cytometry to confirm loss of BFP and retention of HLA-ABC signals. Plots of BFP vs HLA-ABC are presented, wherein all three monoclones can be observed having lost the BFP signal in comparison to parental eAPC-p (right most plot), indicating a successful integration couple event. **c)** To demonstrate that the monoclones contained the correct fragment size for aAM ORF, a polymerase chain reaction was conducted, utlising primers targeting the aAM ORF and representative agarose gel is presented. Results from two monoclones representing each aAM ORF are shown. Lane 1: 2_log DNA marker, Lanes 2-3: pp28 ORF (expected size 0.8kb), Lane 4: 2_log DNA marker, Lanes 5-6: pp52 ORF (expected size 1.5kb), Lane 7: 2_log DNA marker, Lanes 8-9: pp65 ORF (expected size 1.9kb), Lane 10: 2_log DNA marker. All monoclones analysed had the expected amplicon size for the respective aAM, further indicating the integration couple had occurred.

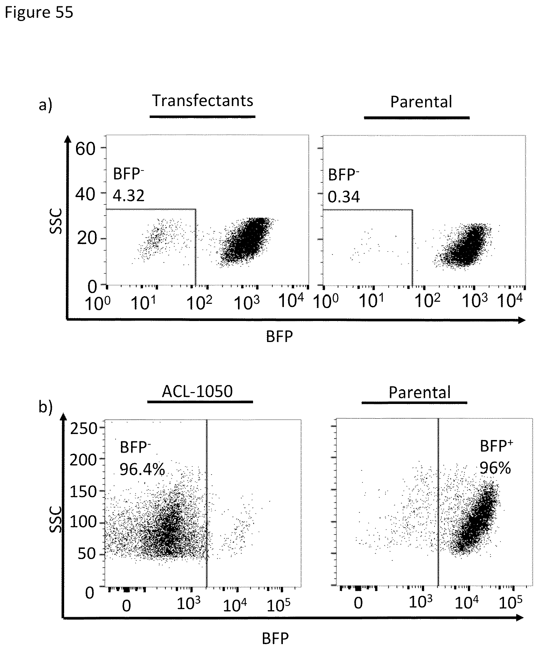

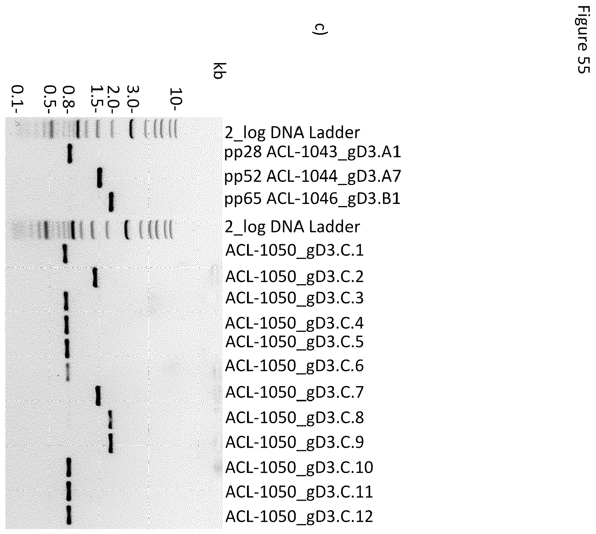

**Figure 55** - **Shotgun integration of multiple antigens into eAPC-p to create a pooled eAPC-pa library in a single step**
   A pooled library of eAPC-pa were generated from a pool of primed **Component 1E** vectors **(Component 1E')** collectively encoding multiple aAM ORF (HCMVpp28, HCMVpp52 and HCMVpp65) by integration in a single step into the parental eAPC-p, wherein each individual cell integrates a single random analyte antigen ORF derived from the original pool of vectors, at **Component 1D',** such that each generated eAPC-pa expresses a single random aAM, but collectively the pooled library of eAPC-pa represents all of aAM ORF encoded in the original pooled library of vectors. The library of eAPC-pa was generated by electroporation by combing the eAPC-p (ACL-905, aAPX: HLA-A*02:01) with a pooled vector library comprised of individual vectors encoding an ORF for one of HCMVpp28, HCMVpp52 or HCMVpp65 (V9.E.6, V9.E.7, and V9.E.8), and being mixed at a molecular ratio of 1:1:1. Resulting eAPC-pa populations were analysed and selected by flow cytometry, in parallel with the parental eAPC-p line. **a)** At 10 days post electroporation putative eAPC-pa cells (Transfectants) were analysed and selected by flow cytometry, compared in parallel with the parental line (ACL-905). Plots display BFP vs SSC, gated for BFP- populations, wherein an increase in BFP- cells are observed in the BFP- gate compared to the parental line. Bulk cells were sorted form the transfectants based on BFP- gate, denoted ACL-1050. **b)** After outgrowth, ACL-1050 cells were analysed by flow cytometry for loss of BFP. Plots displayed are BFP vs SSC, wherein ACL-1050 has been enriched to 96.4% BFP- compared to parental line -4% BFP-. Subsequently, single cells were sorted from the BFP- pollution of ACL-1050. **c)** To demonstrate that the polyclone ACL-1050 was comprised of a mixture of HCMVpp28, HCMVpp52 and HCMVpp65 encoding cells, 12 monoclones were selected at random, outgrown and were used for genetic characterisation. Cells were characterised by PCR utilising primers targeted to the aAM ORF **(Component 1D'),** to amplify and detect integrated aAM. All 12 monoclones screened by PCR have detectable amplicons and are of the expected size for one of pp28 (0.8kb), pp52 (1.5kb) or pp65 (1.9kb). In addition, all 3 aAMs were represented across the 12 monoclones. In comparison, amplicons from three discrete monoclones, wherein in the aAM was known, were amplified in parallel as controls; all three controls produced the correct sized amplicons of pp28 (0.8kb), pp52 (1.5kb) and pp65 (1.9kb). Thus, it is confirmed that the pool is comprised of eAPC-pa wherein each cell has a single randomly selected aAM form the original pool of three vectors.

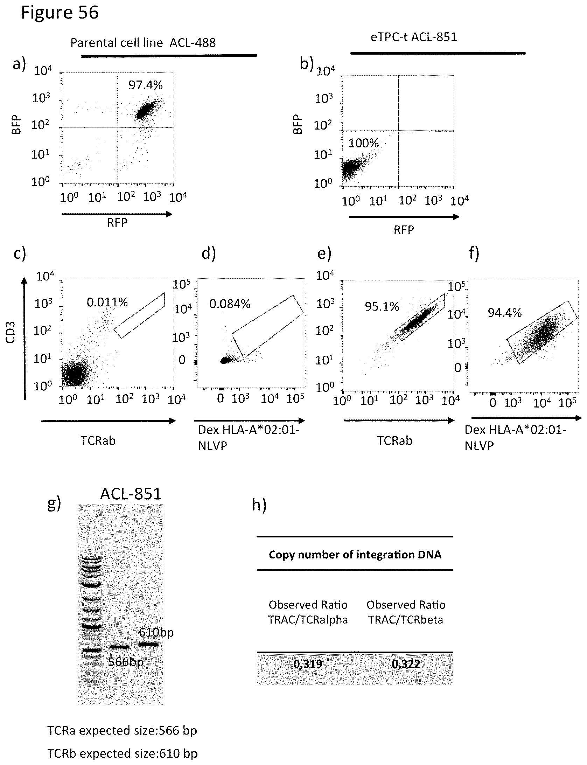

**Figure 56** - **Demonstration of eTPC-t generation from parental eTPC**
   A model TCR alpha/beta pair (JG9-TCR), which has a known specificity for a HCMV antigen presented in HLA-A*02:01 was selected for integration to an eTPC parental line. The JG9-TCR-alpha ORF was cloned in a Component 2E' context, and JG9-TCR-beta in a 2C' context. An eTPC-t was created through RMCE by transfection of Component 2C' and 2E' plasmids and a construct encoding flp recombinase into the eTPC line ACL-488, which harbours two genomic integration sites, 2B and 2D, encoding reporters BFP and RFP, respectively. 10 days after transfection, individual cells diminished for the BFP and RFP signals, encoded by **Components 2B** and 2**D** selection markers, were sorted as single cells. Resulting monoclonal eTPC-t ACL-851 were analysed in parallel with the parental eTPC, and a single example presented. a) and b) Parental eTPC cell line ACL-488 and an example monoclonal was analysed by flow cytometry for BFP and RFP signals. The plot displays live single cells as BFP versus RFP, showing the eTPC cell line is positive for selection markers present in Component 2B and 2D (a), and resulting monoclone has lost these markers as expected for integration couple events between 2B/2C and 2D/2E (b). Percentage values represent the percentage of double positive cells in a) and double negative cells in b). c) to f) eTPC ACL-488 and monoclone eTPC-t ACL-851 were stained with antibodies for CD3 and TCR alpha/beta (TCRab) and HLA multimer reagent specific for the JG9-TCR (Dex HLA-A*02:01-NLVP) and analysed by flow cytometry and gated for live single cells. The parental eTPC line showed no positive staining for CD3 or TCR on the cell surface (c), and was also negative for staining with HLA multimer reagent (d). In contrast, the resulting monoclone showed positive staining for both CD3 and TCR on the cell surface (e) and showed positive staining with the multimer reagent specific for the expressed JG9-TCR. Percentage values represent the percentage of CD3/TCRab double positive cells in c) and e), and CD3/HLA-multimer double positive cells in d) and f). g) Genomic DNA was prepared from monoclonal eTPC-t ACL-851 and subjected to PCR with primers specific for the JG9-TCR-alpha chain encoded by Component 2D', or the JG9-TCR-beta chain encoded by Component 2B'. PCR products were resolved by agarose gel and observed as expected band size. h) Genomic DNA was prepared from monoclonal eTPC-t ACL-851 and subjected to digital drop PCR with primers and probes specific for the JG9-TCR-alpha chain encoded by Component 2D', or the JG9-TCR-beta chain encoded by Component 2B'. A reference amplicon primer/probe pair for an intron of the TCR alpha constant (TRAC) was included. The table presents ratios of reference to TCR alpha and TCR beta. A ratio of close to 0.33 indicates that a single copy of each TCR alpha and beta chain is present in the eTPC-t line ACL-851, which is a triploid line.

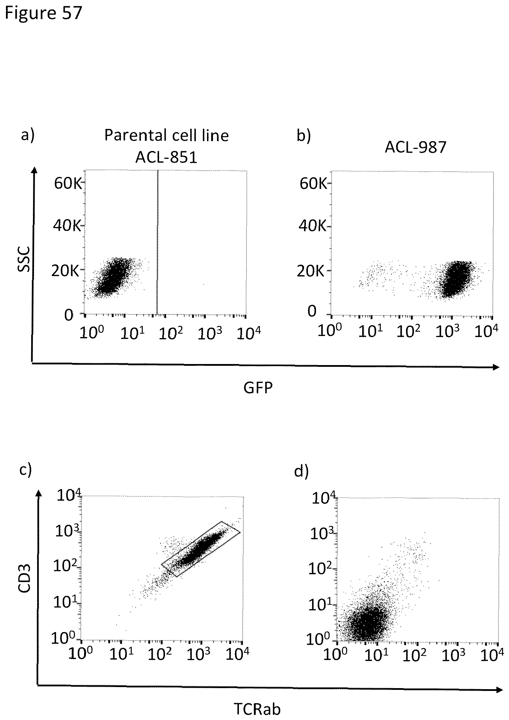

**Figure 57** - **Demonstration of eTPC-x reversion from eTPC-t**
   A parental eTPC-t cell line ACL-851, expressing a TCR alpha and beta chain at site 2D' and 2B', respectively was reverted to a eTPC-x line by exchanging Component 2D' with a donor vector encoding GFP (Component 2Z). **Component 2Z** contained recombinase heterospecific F14/F15 sites flanking the GFP ORF, and was thus compatible with **Component 2D'.** eTPC-t line ACL-851 was transfected with Component 2Z along with a construct encoding flp recombinase. 7 days after transfection, individual cells positive for GFP signals were sorted and grown as monoclones. Resulting monoclonal eTPC-x lines were analysed by flow cytometry in parallel with the parental eTPC-t, and a single example presented. a) and b) The monolcone eTPC-x (ACL-987) derived from parental eTPC-t ACL-851 was analysed by flow cytometry for GFP expression along with the parental line. Plots display SSC versus GFP parameters of gated live single cells. The parental cell line has no GFP expression (a), while the monoclone ACL-987 has gained GFP as expected (b), indicating exchange of the TCR alpha ORF for a GPF ORF. c) and d) The monolcone eTPC-x ACL-987 derived from parental ACL-851 along with the parental eTPC-t ACL-851 were stained with antibodies for CD3 and TCRab and analysed by flow cytometry. Plots display CD3 versus TCRab parameters gated on live single cells. The parental cell showed positive staining for both CD3 and TCRab (c), while the derived monoclone showed negative staining for both (d); confirming loss of TCR alpha ORF in the derived eTPC-x line.

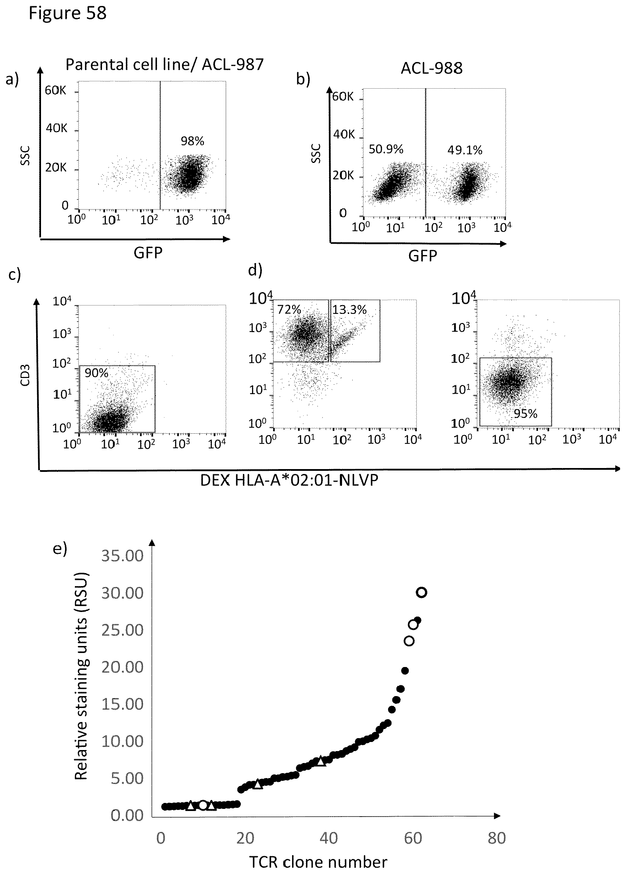

**Figure 58** - **Demonstration of shotgun integration into eTPC-x to create pool of eTPC-t**
   An eTPC-t pool was created from an eTPC-x parental line expressing a single TCR beta chain in Component 2B'. The eTPC-x line expressed GFP as the reporter at available site 2D. A pool of 64 variant TCR alpha chains, including the parental chain, were constructed. The parental TCR chain pair represents the JG9-TCR with known specificity for a HCMV antigen presented in HLA-A*02:01. The Component 2E pool was transfected into the parental eTPC-x ACL-987 along with a construct encoding flp recombinase. A polyclonal line was selected by sorting for GFP positive cells 10 days after transfection. The resulting ACL-988 polyclonal eTPC-t was subsequently sorted on the basis of negative staining for GFP and positive or negative staining for HLA multimer reagent (DEX HLA-A*02:01-NLVP). Recovered single cells were sequenced to identify the encoded TCR-alpha chains and compared to a parallel analysis of each of the TCR-alpha chain variants paired with the native TCR-beta chain in terms of staining with an HLA multimer reagent specific for the parental TCR chain pair. a) and b) Parental eTPC-x ACL-987 line and resulting polyclone eTPC-t ACL-988 line were analysed by flow cytometry for GFP expression. Plots display SSC versus GFP parameters of live single cells. Parental cell line shows positive signal for GFP, indicating intact Component 2D (a). Derived polyclonal line shows half positive and half negative for GFP (b), indicating that half of the cells in the polyclonal population have potentially exchanged the GFP ORF at D for TCR alpha ORF to form Component 2D', c) and d) Parental eTPC-x ACL-987 line and resulting polyclone eTPC-t ACL-988 line were stained with and CD3 antibody and HLA multimer with specificity for the parental JG9-TCR (DEX HLA-A*02:01-NLVP), and analysed by flow cytometry. Plots display CD3 versus HLA multimer parameters of live single cells. The parental cell line is negative for both CD3 and HLA multimer staining (c). The left hand panel of d) displays gated GFP-negative events, and the right hand GFP-positive events. Only GFP-negative events, where the Component 2D is converted to D', shows CD3 positive staining, of which a subset shows positive staining for HLA multimer. Single cells from the gated HLA multimer negative and positive gate were sorted and the integrated ORF at Component 2D' sequenced to determine identity of TCR alpha ORF. e) All 64 JG9-TCR-alpha variants were cloned into an expression construct that permitted each to be independently transfected to parental eTPC-x (ACL-987). Relative staining units (RSU) against the HLA-A*02:01-NLVP tetramer reagent was determined for each. RSU is calculated as the ratio of the mean fluorescence intensity (MFI) of HLA-A*02:01-NLVP tetramer signal for the CD3 positive population over the CD3 negative population, and is indicative of the binding strength of each TCR chain pair variant to the HLA multimer reagent. Each point plotted in Figure e) represents the observed RSU for each 64 variants. Open circles correlate to the sequenced cells recovered from the GFP-negative/HLA multimer-positive gate. Open triangles correlate to the sequenced cells recovered from the GFP-negative/HLA multimer-negative gate.

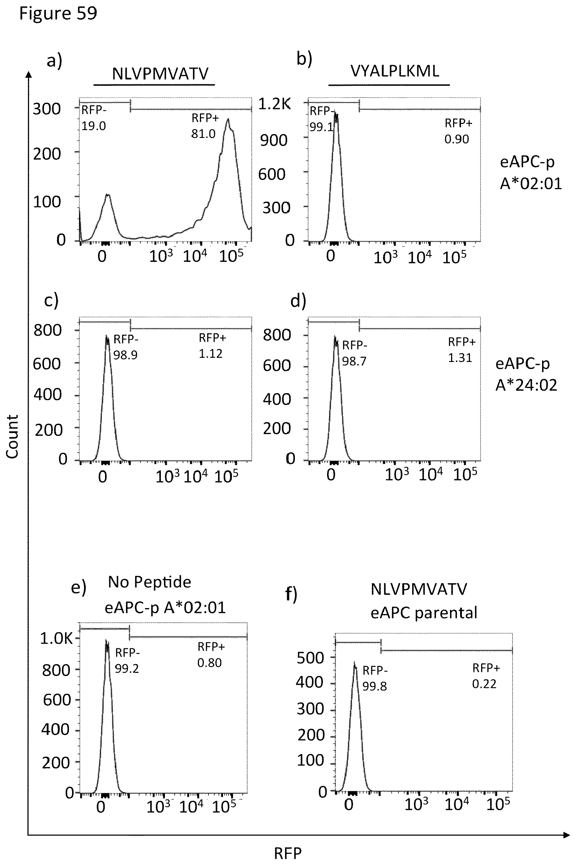

**Figure 59** - **Functional demonstration of Component 2F in eAPC:eTPC system using eAPC-p and exogenous aAM**
   The eTPC-t cell line carrying a Component 2F (ACL-1277), wherein the TCR chains at Component 2B' and 2D' encode a TCR pair that is specific for HMCV antigenic peptide NLVPMVATV presented in HLA-A*02:01. The Component 2F reporter was RFP. This eTPC-t was contacted for 24 hours with various eAPC lines of differing -p characteristics in the presence and absence of model peptide antigens, and the contact cultures analysed by flow cytometry. Flow cytometry histogram plots show event counts against RFP signal of viable single T-cells identified by antibody staining for a specific surface marker that was not presented by the eAPC. a) and b) eAPC-p cells expressing only HLA-A*02:01 (ACL-209) were pulsed with NLVPMVATV (a) or VYALPLKML (b) peptides and subsequently co-cultured with eTPC-t for 24 hrs. c) and d) eAPC-p cells expressing only HLA-A*24:02 (ACL-963) were pulsed with NLVPMVATV (c) or VYALPLKML (d) peptides and subsequently co-cultured with eTPC-t for 24 hrs. e) eAPC-p cells expressing only HLA-A*02:01 (ACL-209) were left without peptide pulsing and subsequently co-cultured with eTPC-t for 24 hrs. f) eAPC parental cells that express no HLA on the cell surface (ACL-128) were pulsed with NLVPMVATV and subsequently co-cultured with eTPC-t for 24 hrs. RFP signal was significantly increased in the eTPC-t ACL-1277 only in the presence of HLA-A*02:01 expressing eAPC-p pulsed with NLVPMVATV, representing the known target of the expressed TCR. Histogram gates and values reflect percentage of events in the RFP positive and RFP negative gates. This indicates the specific response of Component 2F to engagement of eTPC-t expressed TCRsp with cognate HLA/antigen (aAPX:aAM).

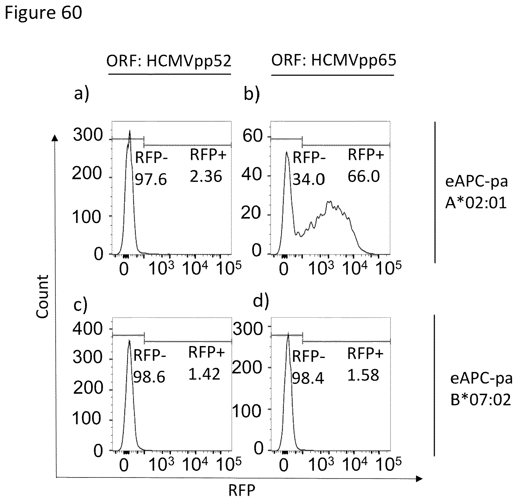

**Figure 60** - **Functional demonstration of Component 2F in eAPC:eTPC system using eAPC-pa with integrated aAM**
   The eTPC-t cell line carrying a Component 2F (ACL-1150), wherein the TCR chains at Component 2B' and 2D' encode a TCR pair that is specific for HMCV antigenic peptide NLVPMVATV presented in HLA-A*02:01. The Component 2F reporter was RFP. This eTPC-t was contacted for 24 hours with various eAPC lines of differing -pa characteristics in the absence of exogenous antigen.a) eAPC-pa line (ACL-1044) expressing HLA-A*02:01 and the full-length ORF for HCMV protein pp52. pp52 does not contain antigenic sequences recognised by the JG9-TCR. b) eAPC-pa line (ACL-1046) expressing HLA-A*02:01 and the full-length ORF for HCMV protein pp65. pp65 contains antigenic sequence recognised by the JG9-TCR, when presented in HLA-A*02:01. c) eAPC-pa line (ACL-1045) expressing HLA-B*07:02 and the full-length ORF for HCMV protein pp52. pp52 does not contain antigenic sequences recognised by the JG9-TCR. d) eAPC-pa line (ACL-1048) expressing HLA-B*07:02 and the full-length ORF for HCMV protein pp65. pp65 contains antigenic sequence recognised by the JG9-TCR, when presented in HLA-A*02:01. After independent co-culture of each eAPC-pa line with the eTPC-t for 48 hrs, RFP expression in the eTPC-t was determined by flow cytometry. RFP signal was significantly increased in the eTPC-t ACL-1150 only when contacted with eAPC-pa ACL-1046. This was the only eAPC-pa with both the recognised antigenic peptide sequence encoded by the integrated aAM ORF, and the correct HLA restriction. Histogram gates and values reflect percentage of events in the RFP positive and RFP negative gates. This indicates the specific response of Component 2F to engagement of eTPC-t expressed TCRsp with cognate HLA/antigen (aAPX:aAM).

### Materials and methods

All cell lines used in this application are either on the ARH or HEK293 background. They are denoted by ACL followed by a number. A summary of the cell lines used in this application is presented as Table 1.

### Transfection of cells

One day prior to transfection/ electroporation, cells were seeded at a density of 1.2-1.4 ×10⁶ cells/60mm dish in 90% DMEM + 2mML-glutamine + 10% HI-FBS (Life Technologies). The following day, cells with 65% confluency were transfected with a total amount of 5ug DNA and jetPEI ^{®} (Polyplus transfection reagent, Life Technologies) at a N/P ratio of 6. Stock solutions of DNA and jetPEI ^{®} were diluted in sterile 1M NaCl and 150mM NaCl respectively. The final volume of each solution was equivalent to 50% of the total mix volume. The PEI solution was then added to the diluted DNA and the mixture was incubated at room temperature for 15min. Finally, the DNA/PEI mixtures were added to the 60-mm dishes, being careful not to disrupt the cell film. The cells were incubated for 48 hours at (37 °C, 5% CO2, 95% relative humidity) prior to DNA delivery marker expression analysis. The medium was replaced before transfection.

### Fluorescence activated cell sorting (FACS)

Single cell sorting or polyclone sorting was achieved through standard cell sorting methodologies using a *BDInflux* instrument. Briefly, ACL cells were harvested with TrypLE^{™} Express Trypsin (ThermoFisher Scientific) and resuspended in a suitable volume of DPBS 1X (Life Technologies) prior to cell sorting, in DMEM 1X medium containing 20% HI-FBS and Anti-Anti 100X (Life Technologies). Table 2 summarises the antibodies and multimers used in this application for FACS.

| **Table 2: BD Influx filters** | | |
|---|---|---|
| **Protein** | **Fluorochrome** | **Filter** |
| Cas9/GFP | GFP | 488-530/40 |
| HLA-A, B, C | PE-Cy5 | 561-670/30 |
| BFP | BFP | 405-460/50 |
| RFP | RFP | 561-585/29 |
| TCRab (R63) | APC | 640-670/30 |
| CD3 (R78) | APC-H7 | 640-750LP |
| CD3 (R71) | APC | 640-760/30 |
| DEX HLA-A*02:01-NLVP | PE | 561-585/29 |

| **Table 1**: Table of ACL cell lines, components and if applicable ORF integrated at Component 1B/1B' or 2B/2B' and Component 1D/1D' or 2D/2D' | | | | |
|---|---|---|---|---|
| **ID** | **Components** | **Gene of interest (B or B')** | **Gene of interest (D or D')** | **Designation** |
| ACL-3 | None | NA | NA | - |
| ACL-128 | None | NA | NA | - |
| ACL-191 | 1B', 1D | HLA-A*02:01 | | eAPC-p |
| ACL-209 | 1B', 1D | HLA-A*02:01 | | eAPC-p |
| ACL-341 | 1B', 1D | HLA-DRB1*01.01 | | eAPC-p |
| ACL-390 | 1B', 1D' | HLA-A*02:01 | pp65 ORF | eAPC-pa |
| ACL-402 | 1B, 1D | RFP | BFP | eAPC |
| ACL-488 | 1B, 1D | BFP | RFP | |
| ACL-851 | 1B', 1D' | JG9-TCR-beta | JG9-TCR-alpha | eTPC-t |
| ACL-900 | 1B', 1D | HLA-A*02:01 | BFP | eAPC-p |
| ACL-905 | 1B', 1D | HLA-A*02:01 | BFP | eAPC-p |
| ACL-963 | 1B', 1D | HLA-A*24:02 | BFP | eAPC-p |
| ACL-987 | 1B', 1D | JG9-TCR-beta | GFP | eTPC-x |
| ACL-988 | 1B', 1D' | JG9-TCR-beta | JG9-TCR-alpha 64x variants | eTPC-t (pool) |
| ACL-1050 | 1B', 1D' | HLA-A*02:01 | pp28, pp52, pp65 | eAPC-pa (pool) |
| ACL-1043 | 1B', 1D' | HLA-A*02:01 | pp28 ORF | eAPC-pa |
| ACL-1044 | 1B', 1D' | HLA-A*02:01 | pp52 ORF | eAPC-pa |
| ACL-1045 | 1B', 1D' | HLA-B*07:02 | pp52 ORF | eAPC-pa |
| ACL-1046 | 1B', 1D' | HLA-A*02:01 | pp65 ORF | eAPC-pa |
| ACL-1048 | 1B', 1D' | HLA-B*07:02 | pp65 ORF | eAPC-pa |
| ACL-1063 | 2B, 2D, 2F | Selection marker 1 | Selection marker 2 | eTPC with response element |
| ACL-1150 | 2B', 2D', 2F | TCR-alpha | TCR-beta | eTPC-t, with response element |
| ACL-1219 | 1B', 1D' | HLA-A*02:01 | pp28 ORF | eAPC-pa |
| ACL-1227 | 1B', 1D' | HLA-A*02:01 | pp52 ORF | eAPC-pa |
| ACL-1233 | 1B', 1D' | HLA-A*02:01 | pp65 ORF | eAPC-pa |
| ACL-1277 | 2B', 2D', F | TCR-alpha | TCR-beta | eAPC-t, with response element |

### Flp-mediated integration of HLA-A*02:01 sequences in eAPC cell line

eAPC cells were electroporated with vectors encoding Flp, DNA encoding a marker to track delivery (vector encoding GFP) and a vector containing HLA-A*02:01. The HLA-A*02:01 sequence also encoded a linker and 3xMyc- tag at the 3'end. The electroporation conditions used were 258 V, 12.5 ms, 2 pulses, 1 pulse interval. Ratio between each integrating vector and the Flp-vector was 1:3. Cells electroporated with only GFP-vector and no electroporated cells were used as controls respectively in order to set the gates for GFP sort after two days. On the following day (2 days after electroporation), cells were analyzed and sorted based on GFP expression. Cells were sorted using the BD Influx Cell Sorter.

At 3 days after electroporation, a sort based on GFP-expression was performed in order to enrich for electroporated cells. 7-8 days after electroporation, the cells were harvested and surface stained for HLA-ABC expression. BFP+ve RFP-ve HLA+ve cells were single cell sorted for monoclonal.

To genotype the cells, 100 ng of DNA was used as template to run a PCR reaction to check if integrations had occurred at the expected integration site. A forward primer targeting the integration cassettes (Pan_HLA_GT_F1) and a reverse primer (SV40pA_GT_R1) targeting just outside the integration site was used and the PCR product was run on a 1% agarose gel.

### Flp-mediated integration of HCMV ORF sequences in eAPC-p cell line

eAPC-p cells were electroporated with vectors encoding Flp, DNA encoding a marker to track delivery (vector encoding GFP) and vectors containing HCMV pp28, pp52 or pp65 aAM-ORF. The HCMV-ORF sequences also encoded a linker and 3xMyc- tag at the 3'end. The electroporation conditions used were 258 V, 12.5 ms, 2 pulses, 1 pulse interval.

Ratio between each integrating vector and the Flp-vector was 1:3. Cells electroporated with only GFP-vector and no electroporated cells were used as controls respectively in order to set the gates for GFP sort after two days. On the following day (2 days after electroporation), cells were analyzed and sorted based on GFP expression. Cells were sorted using the BD Influx Cell Sorter.

### Flp-mediated shotgun integration of 3 HCMV ORF sequences in eAPC-p cell line

eAPC-p cells were electroporated with vectors encoding Flp, DNA encoding a marker to track delivery (vector encoding GFP) and vectors containing HCMV pp28, pp52 or pp65 aAM-ORF. The HCMV-ORF sequences also encoded a linker and 3xMyc- tag at the 3'end. The electroporation conditions used were 258 V, 12.5 ms, 2 pulses, 1 pulse interval. For the shotgun integration, the vectors containing HCMV-ORFs were pooled in a ratio 1:1:1 and the mixture was electroporated into the eAPC-p cell. The resulting eAPC-pa cells were polyclonal. Individual monoclone cells were sorted and genetically characterized to demonstrate that the polyclone was made up of cells containing all three HCMV-ORFs.

### Genetic characterization of the monoclones

### PCR reactions to assess the RMCE-integration of the HCMV ORFs into Component D

Primers used to assess integration of the HCMV ORF annealed to the linker (forward primer 10.D.1) and EF1aplha promoter (reverse primer 15.H.4). Expected size was 0.8kb for pp28, 1.5kb for pp52, 1.9kb for pp65. PCR products were run on a 1% Agarose gel in 1XTAE buffer, using the PowerPac Basic (Bio-Rad), stained with 10,000 dilution of sybersafe and analyzed with Fusion SL (Vilber Lourmat).

| **Table 3: PCR reagents for assess integration of the aAM ORF** | |
|---|---|
| Reaction Component | Volume per reaction |
| 5xPhusion buffer | 4 ul |
| DNTPs | 0,2 ul |
| Phusion DNA polymerase | 0,15 ul |
| 10.D.1 | 0,5 ul |
| 15.H.4 | 0,5 ul |
| H20 | up to 20 ul |
| DNA (1 00ng) | 1 ul (100 ng/ul) |
| DMSO 3% | 0.6 ul |

| **Table 4: PCR cycle conditions** | | |
|---|---|---|
| **Step** | **Temperature** | **Time** |
| Initial Denaturation | 98°C | 30 sec |
| 30 cycles | 98°C | 10 sec |
| | 60°C | 10 sec |
| | 72°C | 15 sec |
| Final extension | 72°C | 10 min |

### RMCE between a paired integration couple

For RMCE integration, cells were transfected with 0.6 µg of DNA vectors encoding FLP, (V4.L8), 2 µg of Component C/Y, 2 µg of Component E/Z, 0.4 µg of DNA encoding a marker to track DNA delivery. 2 days after transfection cell positive for the DNA delivery marker, either GFP or RFP positive, were sorted by FACS. 4-10 days after transfection, individual cells displaying diminished fluorescent protein signal, encoded by **Components D** and **B** selection markers were sorted by FACS. The exception being for generating ACL-987 where individual cells displaying GFP positivity were sorted by FACS.

### Transient expression of TCR chain pairs to characterization of their RSU

For transient expression, cells were transfected with DNA vectors encoding FLP, (V4.I.8), JG9-TCR-alpha variant (VP.7751.RC1.A1 to VP.7751.RC1.H8), JG9-TCR-beta WT chain (V3.C.5), and DNA vector vehicle (V1.C.2) . 2 days after transfection, all cells were stained with HLA-A*02:01-NLVP tetramer and anti-CD3 antibodies. RSU were calculated as the ratio of the mean fluorescence intensity (MFI) of HLA-A*02:01-NLVP tetramer signal for the CD3 positive population over the CD3 negative population, and was indicative of the binding strength of each TCR chain pair variant.

### HLA multimer staining

Cells were stained with HLA-multimer reagent on ice for 10 mins, then with CD3 and/or TCRab antibodies. Detection of specific cell fluorescent properties by the *BDInflux* instrument are defined in table 6.

Sorting of single cells for monoclonal generation, the cells displaying the phenotype interest were deposited into 96-well plates, containing 200 ul of growth medium. One to two plates were sorted per sample. Polyclonal cell sorts were directed into FACS tubes, containing media, using the Two-way sorting setting in the cell sorter Influx^{™} (BD Biosciences).

Single cells sorts for molecular characterization of their JG9-TCR-alpha variant were sorted to PCR plate pre-loaded with 5 µL of nuclease-free water. Specimens were snap-frozen until subsequent processing.

### Genomic DNA extraction for genetic characterization

DNA was extracted from 5×10⁶ cells using the QIAamp DNA Minikit (Qiagen). DNA was stored in 1xTE (10mM Tris pH8.0 and 0.1mM EDTA).

| **Table 5: Vectors** | |
|---|---|
| **ID** | **Name** |
| V1.A.4 | pcDNA3.1_GFP |
| V1.A.6 | pcDNA3.1_RFP |
| V1.C.2 | pMA-SV40pA |
| V3.C.5 | pMA-CS-JG9-TCRbeta |
| V4.H9 | pMA-F14-TurboGFP-F15 |
| V7.A.3 | pMA-F14-TCR-JG9⊐-F15 |
| V7.A.4 | pMA-FRT-TCR-JG9⊐-F3 |
| V8.F.8 | F14-TCRaF15 CDR3degen.64mix |
| V4.I.8 | CMVpro-Flp-sv40pA-V2 |
| VP.7751.RC | 64 individual vectors, each encode a different |
| 1-A1 to H8 | member of JG9-TRA CDR3 64 variants set |
| V4.H.5 | pMA_F14_HLA-A*02:01-6xHis_F15 |
| V4.H.6 | pMA_F14_HLA-A*24:02-6xHis_F15 |
| V4.H.7 | pMA_F14_HLA-B*07:02-6xHis_F15 |
| V4.H.8 | pMA_F14_HLA-B*35:01-6xHis_F15 |
| V9.E.6 | FRT_HCMVpp28-3xMYC_F3 |
| V9.E.7 | FRT_HCMVpp52-3xMYC_F3 |
| V9.E.8 | FRT_HCMVpp52-3xMYC_F3 |
| V1.A.8 | SpCas9-2A-GFP |
| V2.A.1 | HLA-A-sg-sp-opti1 |
| V2.A.7 | HLA-B-sg-sp-3 |
| V2.B.3 | HLA-C-sg-sp-4 |
| V2.I.10 | HLA-A-ex2-3_sg-sp-opti_1 |
| V2.J.1 | HLA-A-ex2-3_sg-sp-opti_2 |
| V2.J.6 | AAVSI_sg-sp-opti_3 |

### PCR reactions to assess the RMCE- integration of the TRA-ORF and TRB-ORF into component 2B or 2D.

Primers used to assess integration of the TCR-alpha, annealed to the TRAC segment (forward primer 1.F.7) and the sv40pA terminator (Reverse primer 15.H.2) that is a pre-existing part of the genomic receiving sites. Expected size 566bp. Primers used to assess integration of the TCR-beta, annealed to the TRBC segment (forward primer 1.F.9) and the sv40pA terminator (Reverse primer 15.H.2) that is a pre-existing part of the genomic receiving sites. Expected size 610bp. PCR products were run on a 1% Agarose gel in 1XTAE buffer, using the PowerPac Basic (Bio-Rad), stained with 10,000 dilution of sybersafe and analyzed with Fusion SL (Vilber Lourmat).

| **Table 6: PCR reagents for assess integration of ORF encoding TCR-alpha and TCR-beta** | |
|---|---|
| **Reaction Component (TCR-alpha)** | **Volume per reaction** |
| 5xPhusion buffer | 4 ul |
| DNTPs | 0,2 ul |
| Phusion DNA polymerase | 0,15 ul |
| 1.F.7: TRAC-GT-F1 | 0,5 ul |
| 15.H.2: sv40pA-GT-R1 | 0,5 ul |
| H20 | up to 20 ul |
| DNA (100ng) | 1 ul (100 ng/ul) |

| **Reaction Component (TCR Beta)** | **Volume per reaction** |
|---|---|
| 5xPhusion buffer | 4 ul |
| DNTPs | 0,2 ul |
| Phusion DNA polymerase | 0,15 ul |
| 1.F.9: TRBC2-GT-F1 | 0,5 ul |
| 15.H.2: sv40pA-GT-R1 | 0,5 ul |
| H20 | up to 20 ul |
| DNA (100 ng) | 1 ul (100 ng/ul) |

| **Table 7: PCR cycle conditions** | | |
|---|---|---|
| **Step** | **Temperature** | **Time** |
| Initial Denaturation | 98°C | 30 sec |
| 30 cycles | 98°C | 10 sec |
| | 60°C | 10 sec |
| | 72°C | 15 sec |
| Final extension | 72°C | 10 min |

### ddPCR reactions to assess the copy number of TRA-ORF and TRB-ORF in the genome after DNA delivery

DNA of selected ACL-851 monoclones was analysed by using specific primers and probed targeting the TCR ORF C segment (TRAC) of interest. Primers and probe used to assess TRA-ORF copy number, annealed to the TRAC segment (forward primer 1.F.7, Reverse primer 1.F.8 and probe 1.G.1). Primers and probe used to assess TRB-ORF copy number, annealed to the TRB-C segment (forward primer 1.F.9, Reverse primer 1.F.10 and probe 1.G.2)

In all cases, a reference gene (TRAC) was simultaneously screened to chromosome determine copy numbers, using primers 10.A.9 and 10.A.10 together with the fluorescent probe 10.B.6 conjugated with HEX. Integration copy number considered that HEK293 cells are triploid for reference gene (TRAC). Prior to Droplet Digital PCR, DNA was digested with Mfel (NEB) to separate tandem integrations. The reaction setup and cycling conditions were followed according to the protocol for ddPCR^{™} Supermix for Probes (No dUTP) (Bio-Rad), using the QX200^{™} Droplet Reader and Droplet Generator and the C1000 Touch^{™} deep-well Thermal cycler (Bio-Rad). Data was acquired using the QuantaSoft^{™} Software, using Ch1 to detect FAM and Ch2 for HEX.

| **Table 8: ddPCR conditions** | | |
|---|---|---|
| **Step** | **Temperature** | **Time** |
| Initial Denaturation 40 cycles | 95°C | 10 min |
| | 98°C | 30 sec |
| | 60°C | 60 sec |
| Final extension | 72°C | 10 min |
| (Option) Cooling | 8°C | ∞ |

| **Table 9: ddPCR Primers and probes** | | |
|---|---|---|
| **ID** | **Name** | **Sequence** |
| 1.F.7 | TRAC-GT-F1 | ATGTGCAAACGCCTTCAAC |
| 1.F.8 | TRAC-GT-R1 | TTCGGAACCCAATCACTGAC |
| 1.G.1 | TRAC-probe-FAM | TTTCTCGACCAGCTTGACATCACAGG |
| 1.F.9 | TRBC2-GT-F1 | GCTGTCAAGTCCAGTTCTACG |
| 1.F.10 | TRBC2-GT-R1 | CTTGCTGGTAAGACTCGGAG |
| 1.G.2 | TRBC2-probe-FAM | CAAACCCGTCACCCAGATCGTCA |
| 10.A.9 | TRAC-TCRA-ex1-F1 | CTGATCCTCTTGTCCCACAGATA |
| 10.A.10 | TRAC-TCRA-ex1-F1 | GACTTGTCACTGGATTTAGAGTCTCT |
| 10.B.6 | TRAC-probe(HEX) | ATCCAGAACCCTGACCCTGCCG |
| 21.1.1 | HCMVpp65_GT_F2 | TCGACGCCCAAAAAGCAC |
| 21.1.2 | HCMVpp28_GT_F1 | TGCCTCCTTGCCCTTTG |
| 21.1.3 | HCMVpp52_GT_F1 | CGTCCCTAACACCAAGAAG |
| 20.H.10 | Myc-Tag_GT_R1 | AAGGTCCTCCTCAGAGATG |
| 20.H.9 | Linker-Myc_Probe_Fam | CTTTTGTTCTCCAGATCCAGATCCACC |

### Sequencing of TCR alpha and beta chains from single T-cells

Individual FACS-sorted eTPC-t-cells were subjected to a two-step amplification process that entails a V-region specific primer collection for each TRA and TRB, followed by paired nested PCR reactions that create TRA and TRB amplicons for sequence analysis. This procedure is described previously (Han et. al. Nat Biotechnol. 2014 32(7): 684-692). The following materials were used in the described procedures:

| **Table 10: Single cell RT-PCR and nested PCR reagents** | | |
|---|---|---|
| **Product** | **Supplier** | **Supplier Number** |
| 2x Reaction Mix | Thermo Scientific | 12574035 |
| 5X Phusion HF Buffer | Thermo Fisher Scientific | F-549S |
| dNTPs | Thermo Fisher Scientific | 10297018 |
| Nuclease free water | Qiagen | 129114 |
| Phusion Hot Start II DNA Polymerase | Thermo Fisher Scientific | F-549S |
| SuperScript^{®} III One- Step RT-PCR System with Platinum^{®} Taq High Fidelity DNA Polymerase | Thermo Scientific | 12574035 |

### Functional demonstration of component F

eTPC-t and eAPC cells were routinely cultured in RPMI+10% heat-inactivated Fetal Calf Serum (complete media) between 0.2 × 10^6 - 1.5 × 10^6 cells/ml, at 37'C, 90% relative humidity and 5% CO2. Peptide NLVPMVATV were synthetized by Genescript, and received lyophilized. Peptide primary stocks were suspended in 10% DMSO and sorted at -80'C. Working stocks were prepared at the time of administration, at 50 µM in complete media (50x concentrated). The following eAPC-p presenting HLA-A*02:01 (ACL-900) or HLA-B*07:02 (ACL-906) or eAPC-pa with aAPX and exogenous aAM, HLA-A*02:01+HCMVpp52 (ACL-1044) or HLA-A*02:01+HCMVpp65 (ACL-1046) or HLA-B*07:02+HCMVpp52 (ACL-1045) or HLA-B*07:02+HCMVpp65 (ACL-1048), or parent eAPC (ACL-128) were used. Two different eTPC-t cell lines were used; the first eTPC-t, ACL-1277, (Component A) was engineered with two unique genomic receiver sites, utilizing native CD3 expression, and harboring a genomic two-component, synthetic response element (Component F, RFP reporter) (See Example 14). The second eTPC-t, ACL-1150, (Component A) was engineered with two unique genomic receiver sites, utilizing native CD3 expression, and harboring a genomic one-component, synthetic response element (Component F, RFP reporter) (See Example 15). Both eTPC-t were loaded with the TCR chain ORF at Component 2B' and 2E' encoding a TCR pair that is specific for HLA-peptide complex (HLA), HLA-A*02:01-NLVPMVATV.

### Antigen pulsing procedure

Actively growing cultures of eAPC cells (0.4-1.0 × 10^6 cells/ml) were suspended, sample taken and counted to determine cell concentration. Subsequently, 1 million cells were harvested, washed once with Dulbecco's phosphate buffered saline (DPBS, Gibco) followed by suspension in complete media with 1 µM of peptide or no peptide at a cell concentration between 1 to 2 × 10^6 cells/ml. Cells were incubated for 2 h in standard culturing conditions, in a 24-well culture plate. After 2 h the cells were harvested, pelleted by centrifugation (400 rcf, 3 min), followed by 3 × 10 ml washes with DPBS. Cells were subsequently suspended at 0.2 × 10^6 cells/ml in complete media.

### eTPC-t harvesting

Actively growing cultures of eTPC-t cells (0.4-1.0 × 10^6 cells/ml) were suspended, sample taken and counted to determine cell concentration. Cells were harvested, washed once with DPBS and then suspended at a concentration of 0.4×10^6 (for endogenous assays) or 0.6 × 10^6 cells/ml (or exogenous assays) in complete media.

### Contacting eTPC-t and eAPC in an e TPC:eAPC system with exogenous antigenic molecules

To each well of a 96-well round-bottom plate, 50 µl of complete media, 50 µl of eAPC , followed by 50 µl of eTPC-t were added. This equated to approximately 10,000 eAPC and 30,000 eTPC-t for a ratio of 1:3, at a total cell concentration of approximately 0.27 × 10^6 cells/ml. The cell mixture was then incubated for approximately 24 hours at standard culturing conditions.

### Contacting eTPC-t and eAPC in an eTPC:eAPC system with endogenous antigenic molecules

To each well of a 96-well round-bottom plate, 50 µl of complete media, 50 µl of eAPC , followed by 50 µl of eTPC-t were added. This equated to approximately 10,000 eAPC and 20,000 eTPC-t for a ratio of 1:2, at a total cell concentration of approximately 0.2 × 10^6 cells/ml. The cell mixture was then incubated for approximately 48 hours at standard culturing conditions.

### Staining and analysis

After 24 or 48 hours incubation, the cells were harvested, and transplanted into 0.75 ml V-bottom Micronic tubes, washed once with 500 µl DPBS and subsequently stained with Dead Cell Marker (DCM-APC-H7) as follows; to each well 25 µl of staining solution was added, cells suspended by mixing and then incubated for 15-20 min. The staining solution comprised of 0.5 µl DCM-APC-H7 per 100 µl staining solution. After incubation, cells were washed twice with 500 µl DPBS+2%FCS (Wash Buffer). Cells were then stained for surface markers unique to the eTPC-t; to each well 30 µl of staining solution was added, cells suspended by mixing and then incubated for 30-45 min. The staining solution comprised of 2.5 µl anti-myc-AF647 per 100 µl staining solution (clone 9E10, Santa Cruz Biotech). After incubation, cells were washed twice with 500 µl Wash buffer, suspended in 200 µl of Wash buffer and then analysed by FACS on a LSRFor-tessa (BD Biosciences).

### Examples

### Example 1: Deletion of an APX gene family by targeted mutagenesis

Herein describes how targeted mutagenesis of a family of antigen-presenting complex **(APX)** encoding genes was achieved to produce the first trait of an engineered antigen-presenting cell **(eAPC).** The said trait is the lack of surface expression of at least one member of the APX family.

In this example, the targeted APX comprised the three members of the major HLA class I family, HLA-A, HLA-B and HLA-C in the HEK293 cell line. HEK293 cells were derived from human embryonic kidney cells that showed endogenous surface expression of HLA-ABC. Cytogenetic analysis demonstrated that the cell line has a near triploid karyotype, therefore the HEK293 cells encoded three alleles of each HLA-A, HLA-B and HLA-C gene.

Targeted mutagenesis of the HLA-A, HLA-B and HLA-C genes was performed using an engineered CRISPR/Cas9 system, in which, Cas9 nuclease activity was targeted to the HLA-A, HLA-B and HLA-C loci by synthetic guide RNAs (gRNAs). 4 to 5 unique gRNAs were designed to target conserved nucleotide sequences for each HLA gene locus and the targeted sites were biased towards the start of the gene coding sequence as this was more likely to generate a null allele. The gRNAs efficiency to induce a mutation at their targeted loci was determined and the most efficient gRNAs were selected to generate the HLA-A, HLA-B and HLA-C null (HLA-ABC^{null}) HEK293 cell line.

Plasmid that encoded the optimal gRNAs targeting the HLA-A, HLA-B and HLA-C loci, together with a plasmid that encoded Cas9-P2A-GFP were transfected into HEK293 cells as described in the methods. Cells positive for Cas9-P2A-GFP plasmid uptake were FAC sorted based on GFP fluorescence, 2 days after transfection **(****figure 38a****).** The GFP sorted cells were further expanded for more than 5 days to allow sufficient time for gene editing events to occur, and in the case of a detrimental mutation, to lose of expression of the residual endogenous HLAI protein. After this growth period, the cells were stained with a pan-HLA-ABC antibody, resulting in the identification of cells with reduced expressed HLA-ABC on their surface **(****figure 38b****).** The absence of pan-HLA-ABC antibody staining implied that each HLA-A, HLA-B and HLA-C allele was mutated. Individual HLA-ABC negative cells were sorted and expanded to represent a collection of monoclones.

HLA-ABC^{null} monoclones were confirmed by lack of surface expression of HLA-ABC. It was demonstrated that a subset of monoclones lacked surface expression of HLA-ABC, of which three example monoclones, ACL-414, ACL-415 and ACL-416 are depicted in **figure 39****.** Further genetic characterization of the monoclones that lacked HLAI surface expression was performed by determining that the cell lines possessed an underlying genetic mutation in all alleles of the HLA-A, HLA-B and HLA-C genes **(****figure 40****).** Genetic characterization was performed by PCR with primers that spanned the gRNA genomic target sites, for detection of amplicon size changes and/or were used as a template for sequencing. **Figure 40** shows a selection of HLA-ABC^{null} monoclones that contained genetic deletion in the alleles of HLA-A, HLA-B and HLA-C genes detected by a shorter PCR amplicon compared to the amplicon size of the founding cell line (e.g. ACL-414).

In conclusion, the genetically modified HEK293 cell lines, including, ACL-414, ACL-415 and ACL-416, were demonstrated to lack surface expression of the HLA-ABC and therefore possessed the first trait of an engineered antigen-presenting cell **(eAPC).**

### Example 2: Generation of an eAPC containing Component 1B

Herein describes how **Component 1B** was stably integrated into the HLA-ABC^{null} monoclone line ACL-414 to produce the second trait of an **eAPC.** The said second trait contained at least one genomic receiver site for integration of at least one ORF, wherein the genomic receiver site was a synthetic construct designed for recombinase mediated cassette exchange (RMCE).

In this example, the genomic integration site, component 1B, comprised of selected genetic elements. Two unique heterospecific recombinase sites, FRT and F3, which flanked an ORF that encoded the selection marker, blue fluorescent protein (BFP). Encoded 5' of the FRT site, was an EF1a promoter and 3' of the F3 site was a SV40 polyadenylation signal terminator. The benefit of positioning the non-coding cis-regulatory elements on the outside of the heterospecific recombinase sites was so they are not required in the matched genetic donor vector, component 1C. Therefore, after cellular delivery of the genetic donor vector, no transient expression of the encoded ORF would be observed. This made the selection of successful RMCE more reliable as the cellular expression of the ORF from the genetic donor vector would mostly likely occur only after correct integration into component 1B as it now contained the appropriate cis-regulator elements (see example 6).

To promote the stable genomic integration of component 1B into the genomic safe harbour locus, AAVS1, a plasmid was constructed, wherein; the DNA elements of component 1B were flanked with AAVS1 left and right homology arms. Each arm comprised of >500 bp of sequence homologous to the AAVS1 genomic locus.

Stable integration of component 1B was achieved through the process of homology directed recombination (HDR) at the genomic safe harbour locus, AAVS1. The ACL-414 cell line was transfected with plasmid that encoded the optimal gRNAs targeting the AAVS1 locus, plasmid that encoded Cas9-P2A-GFP and the plasmid that encoded component 1B genetic elements flanked by AAVS1 left and right homology arms. Cells positive for Cas9-P2A-GFP plasmid uptake were FAC sorted based on GFP fluorescence, 2 days after transfection **(****figure 41a****).** The GFP sorted cells were further expanded for greater than 7 days allowing sufficient time for HDR to occur and to lose transient expression of the selection marker, BFP. After this growth period, the cells were analysed on a FACS machine and individual BFP positive cells were sorted and expanded to represent a collection of monoclones **(****figure 41c****).**

Individual monoclone lines were selected as an eAPC on the basis of their maintained BFP expression and for a single integration of component 1B into the desired AAVS1 genomic location. Cell lines ACL-469 and ACL-470 represented monoclones with maintained BFP expression **(****figure 42a** **and b).** Genetic characterization was performed on DNA extracted from monoclones ACL-469 and ACL-470 and demonstrated that their genomes integrated component 1B, and that component 1B has been integrated into the AAVS1 site **(****figure 43****).** Confirmation of genomic integration was determined by the detection of a PCR amplicon of the expected size that utilized primers specific for the Component 1B **(****figure 43a****).** Confirmation that component 1B integrated into the AAVS1 site was determined by the detection of a PCR amplicon of the expected size that utilized primers designed against the AAVS1 genomic sequence distal to the region encoded by the homologous arms and a primer that is unique to the SV40 pA terminator encoded by component 1B **(****Figure 43b****).** The copy-number of component 1B was determined by digital drop PCR, in which the number of component 1B and reference gene DNA molecules were measured and the ratio calculated **(table 1).** Monoclones ACL-469 and ACL-470 contained a ratio of 1 component 1B molecule to 3 reference gene molecules. When factored in that the founding HEK293 cell line has a near triploid karyotype, this demonstrated a single integration of component 1B in ACL-469 and ACL-470 cell lines.

In conclusion, the genetically modified ACL-469 and ACL-470 cell lines, were HLA-ABC^{null} and contained a single copy of a synthetic genomic receiver site designed for RMCE and therefore demonstrated the creation of a **eAPC** with a single synthetic integration receiver site.

### Example 3: Generation of an eAPC containing Component 1B and Component 1D

Herein describes how **Component 1B** and **Component 1D** were stably integrated into the HLA-ABC^{null} monoclone line ACL-414 to produce the second trait of an **eAPC.** The said second trait contains two genomic receiver sites for integration of at least one ORF, wherein the genomic receiver site was a synthetic construct designed for recombinase mediated cassette exchange (RMCE).

This example uses the same methods and components as described in example 2 but with the addition of a second genomic receiver site, Component 1D. Component 1D genetic elements comprised of two unique heterospecific recombinase sites, F14 and F15, which were different to component 1B. These sites flanked the ORF that encoded the selection marker, the red fluorescent protein (RFP). Encoded 5' of the F14 site was an EF1a promoter and 3' of the F15 site was a SV40 polyadenylation signal terminator. As in example 2, component 1D genetic elements were flanked with AAVS1 left and right homology arms, each comprised of >500 bp of sequence homologous to the AAVS1 genomic locus.

Component 1B and component 1D were integrated into the AAVS1 as described in example 2 but with the addition of the plasmid that encoded component 1D elements, to the transfection mix. Cells positive for Cas9-P2A-GFP plasmid uptake were FAC sorted based on GFP fluorescence, 2 days after transfection **(****figure 41b****).** The GFP sorted cells were further expanded for grater than 7 days, after which, the cells were analysed on a FACS machine and individual BFP and RFP positive cells were sorted and expanded to represents a collection of monoclones **(****figure 41d****).**

Individual monoclone lines were selected as an eAPC on the basis of their maintained BFP and RFP expression and for a single integration of component 1B and a single integration of component 1D into different AAVS1 alleles. Cell line ACL-472 was a representative monoclone with maintained BFP and RFP expression **(****figure 42c****).** As described in example 2, genetic characterization was performed on DNA extracted from monoclone ACL-472 and demonstrated that their genomes integrated component 1B and component 1D, and that both components integrated into the AAVS1 site **(****figure 43****).** The copy-number of both component 1B and D was determined by digital drop PCR, in which the number of component 1B, D and reference gene DNA molecules was measured and the ratio calculated. The monoclone ACL-472 contained a ratio of 2 component 1B and D molecules to 3 reference gene molecules **(Table 2).** When factored in that the founding HEK293 cell line has a near triploid karyotype, this demonstrated a single integration of component 1B and a single integration of component 1D into the ACL-472 cell line.

In conclusion, the genetically modified ACL-472 cell line, was HLA-ABC^{null} and contained a single copies of the synthetic genomic receiver site component 1B and component 1D, designed for RMCE and therefore demonstrated the creation of an **eAPC** with two unique synthetic integration receiver sites.

### Example 4: An eAPC-p constructed in one step with one integration couple wherein component 1C' encoded a single HLAI ORF

Herein describes how an eAPC-p was constructed in one step with one integration couple, wherein, the genomic receiver site, component 1B, is a native genomic site and the genetic donor vector, component 1C', comprised a single ORF that encoded one analyte antigen-presenting complex **(aAPX).**

In this example, the eAPC was a genetically modified ARH-77 cell line, designated ACL-128, wherein, two families of APX, major HLA class I family and HLA class II, were mutated. The founding cell line, ARH-77, is a B lymphoblast derived from a plasma cell leukemia that showed strong HLA-A,B,C and HLA-DR,DP,DQ cell surface expression. Cytogenetic analysis demonstrated that the founding ARH-77 cell line has a near diploid karyotype, but also displayed a chromosome 6p21 deletion, the region encoding the HLA locus. DNA sequencing of the ARH-77 locus confirmed that ARH-77 encoded only a single allele of HLA-A, HLA-B and HLA-C and HLA-DRA, HLA-DRB, HLA-DQA, HLA-DQB, HLA-DPA and HLA-DPB gene families.

The HLA-ABC^{null} and HLA-DR,DP,DQ^{null} cell line ACL-128, was generated by CRISPR/cas9 targeted mutagenesis with gRNA targeting the HLA-A, HLA-B and HLA-C and HLA-DRA, HLA-DRB, HLA-DQA, HLA-DQB, HLA-DPA and HLA-DPB gene families using the method described in Example 1. Surface labeling with a pan-anti-HLA-ABC or pan-anti-HLA-DR,DP,DQ confirmed that ACL-128 lacked surface expression of both APX families, **figure 44b** and **45** and **figure 47b****,** respectively.

In this example, the genomic receiver site, component 1B, was the native AAVS1 genomic site, and the targeted integration was achieved through HDR. The genetic donor vector, component 1C, was matched to component 1B, by component 1C encoding the AAVS1 left and right homology arms, each comprised of >500 bp of sequence homologous to the AAVS1 genomic locus. Between the AAVS1 left and right homology arms, the plasmid encoded a CMV promoter and a SV40 terminator. The aAPX of interest was cloned between the promoter and the terminator, generating component 1C'. In this example, component 1C' comprised a single ORF that encoded one aAPX, the HLA-A*24:02 or HLA-B*-07:02, denoted component 1C'^{HLA-A*24:02} and component 1C'^{HLA-B*-07:02} respectively.

The process to construct an eAPC-p was via HDR induced integration of component 1C' into component 1B to produce component 1B'. The cell line ACL-128 was electroporated with plasmids that encoded the optimal gRNAs targeting the AAVS1 loci, Cas9-P2A-GFP and component 1C'. Cells positive for Cas9-P2A-GFP plasmid uptake were FAC sorted based on GFP fluorescence, 2 days after electroporation **(****figure 44a****).** The GFP sorted cells were further expanded for grater than 7 days allowing sufficient time for HDR to occur and lose transient expression of the aAPX. After this growth period, the cells were stained with a pan-HLA-ABC antibody, resulting in the identification of cells that gained expression of an analyte HLA on their surface **(****figure 44b****).** The presence of pan-HLA-ABC antibody staining implied that the analyte HLA ORF encoded in component 1C' had integrated into the genome. Individual HLA-ABC positive stained cells were sorted and expanded to represent a collection of eAPC-p monoclones.

Individual monoclone lines were selected as an eAPC-p on the basis of their maintained analyte HLA surface expression and the integration of the analyte ORF into the genomic receiver site, creating component 1B'. Cell lines ACL-321 and ACL-331 were representative monoclones with maintained analyte HLA surface expression of HLA-A*24:02 or HLA-B*-07:02 respectively **(****figure 45****).** Genetic characterization was performed on DNA extracted from selected monoclones ACL-321, ACL-327, ACL-331 and ACL-332 and demonstrated that their genomes integrated component 1C', and that the integration occurred in the AAVS1 genomic receiver site, generating component 1B' **(****figure 46****).** Confirmation of genomic integration was determined by the detection of a PCR amplicon of the expected size using primers specific to the Component 1C' **(figure 46a).** Presence of component 1B' was confirmed by the detection of a PCR amplicon of the expected size using primers designed against the AAVS1 genomic sequence distal to region encoded by the homologous arms and a primer unique to the SV40 pA terminator linked to the analyte HLA ORF **(****Figure 46b****).**

In conclusion, the generation of the genetically modified ACL-321 and ACL-331 cell lines, which contained a copy of the aAPX HLA-A*24:02 or HLA-B*-07:02 ORF, respectively, within the genomic receiver site, component 1B', resulted in the said analyte aAPX to be the only major HLA class I member expressed on the cell surface. Therefore, this demonstrated the creation of two defined **eAPC-p** cell lines using the multicomponent system.

### Example 5: An eAPC-p constructed in one step with one integration couple, wherein component 1C' encoded a paired HLAII ORF

Herein describes how an eAPC-p was constructed in one step with one integration couple, wherein, the genomic receiver site, component 1B, was a native genomic site and the genetic donor vector, component 1C' comprised a single ORF that encoded two **aAPX** chains.

This example used eAPC, ACL-128, and component 1B, both of which are defined in example 4. However component 1C' comprised a single ORF that encoded an HLA-DRA*01:01 allele linked to an HLA-DRB1*01:01 allele by a viral self-cleaving peptide element, or HLA-DPA1*01:03 allele linked to an HLA-DPB1*04:01 allele by a viral self-cleaving peptide element, denoted component 1C'^{HLA-DRA*01:01/HLA-DRB1*01:01} and component 1C'^{HLA-DPA1*01:03/HLA-DPB1*04:01} respectively. The viral self-cleaving peptide element encoded a peptide sequence, that when transcribed resulted in self-cleavage of the synthesized peptide and produced two polypeptides defining each HLA chain.

Within example 4, described the process to construct an eAPC-p with the exception that identification of cells that gained expression of an analyte HLA on their surface were assed by cell surface labelling with a pan-anti-HLA-DR,DP,DQ antibody **(****figure 47****).** The presence of pan-anti-HLA-DR,DP,DQ antibody staining implied that the analyte HLA ORF encoded in component 1C' had integrated into the genome. Individual HLA-DR,DP,DQ positive stained cells were sorted and expanded to represent a collection of eAPC-p monoclones.

Individual monoclone lines were selected as an eAPC-p on the basis of their maintained analyte HLA surface expression and the integration of the analyte ORF into the genomic receiver site, creating component 1B' as described in example 4. Cell lines ACL-341 and ACL-350 were the representative monoclones with maintained analyte HLA surface expression of HLA-DRA*01:01/HLA-DRB1*01:01 or HLA-DPA1*01:03/HLA-DPB1*04:01 **(****figure 48****).**

In conclusion, the generation of the genetically modified ACL-341 and ACL-350 cell lines, which contained a copy of the aAPX HLA-DRA*01:01/HLA-DRB1*01:01 or HLA-DPA1*01:03/HLA-DPB1*04:01 ORF, respectively, within the genomic receiver site, component 1B', resulted in the said analyte aAPX to be the only major HLA class II member expressed on the cell surface. Therefore, this demonstrated the creation of two defined **eAPC-p** cell lines using the multicomponent system.

### Example 6: An eAPC-p constructed in one step with one integration couple wherein component 1B was a synthetic construct

Herein describes how an eAPC-p was constructed in one step with one integration couple, wherein, the genomic receiver site, component 1B, was a synthetic construct designed for RMCE genomic site and the genetic donor vector, component 1C' comprised a single ORF that encoded one **aAPX.**

In this example, the genomic integration site, component 1B, comprised of selected genetic elements. Two unique heterospecific recombinase sites, FRT and F3, which flanked the ORF that encoded the selection marker, blue fluorescent protein (BFP). Encoded 5' of the FRT site, was an EF1a promoter and 3' of the F3 site was a SV40 polyadenylation signal terminator. The genetic elements of component 1B, were integrated in the cell line ACL-128 by electroporation with the same plasmids as described in example 2. Individual monoclone lines were selected on the basis of their maintained BFP expression and were genetically charaterised to contain a single integration of component 1B into the desired AAVS1 genomic location as described in example 2 **(****figure 49a****).** The resulting eAPC cell line, ACL-385, was HLA-ABC^{null} and HLA-DR,DP,DQ^{null} and contained a single copy of a synthetic genomic receiver site, component 1B, designed for RMCE
The genetic donor vector, component 1C was matched to component 1B, as component 1C encoded the same heterospecific recombinase sites, FRT and F3. The aAPX ORF of interest, additionally encoded a kozak sequence just before the start codon, was cloned between the two heterospecific recombinase sites, and generated component 1C'. In this example, component 1C' comprised a single ORF that encoded one aAPX, the HLA-A*02:01, designated component 1C'^{FRT:HLA-A*02:01:F3}.

An eAPC-p was created through RMCE by electroporation of the cell line ACL-385 with plasmid that encoded the Tyr-recombinase, Flp, together with component 1C'^{FRT:HLA-A*02:01:F3}. 4-10 days after electroporation, individual cells positive for HLAI surface expression and negative/reduced for the fluorescent protein marker, BFP, encoded by component 1B selection marker, were sorted. Individual outgrown monoclone lines were selected on the basis of their maintained HLAI allele expression and loss of BFP florescence, which indicated that the expected RMCE occurred. To identify such monoclones, both phenotypic and genetic tests were performed. Firstly, all monoclone cell lines were screened for cell surface HLA-ABC expression and lack of BFP florescence **(****figure 49****).** Genomic DNA was extracted from such cell lines, e.g. ACL-421 and ACL-422, and the integration of component 1C' into component 1B that generated component 1B' was confirmed by the detection of a PCR product specific to component 1B' **(****figure 50****).**

In conclusion, the generation of the genetically modified ACL-421 and ACL-422 cell lines, which contained a copy of the aAPX HLA-A*02:01 ORF, respectively, within the synthetic genomic receiver site, component 1B', resulted in the said analyte aAPX to be the only major HLA class I member expressed on the cell surface. Therefore, this demonstrated the creation of two defined **eAPC-p** cell lines using the multicomponent system.

### Example 7: An eAPC-pa constructed in two steps with two integration couples

Herein describes how an eAPC-pa was constructed in two steps. Step 1, wherein the genomic receiver site, component 1B, was the native genomic site and the genetic donor vector, component 1C' comprised a single ORF that encoded one **aAPX.** Step 2 the genomic receiver site, component 1D, was a second native genomic site and the genetic donor vector, component 1E' comprised a single ORF that encoded one analyte antigen molecule **(aAM).**

In this example, step 1 was performed, wherein, the eAPC was ACL-128, the genomic receiver site, component 1B, was the mutated HLA-A allele genomic site, designated HLA-A^{null}, and the targeted integration was achieved through HDR. The genetic donor vector, component 1C was matched to component 1B, by the component 1C encoding the HLA-A^{null} left and right homology arms, each comprised of >500 bp of sequence homologous to the HLA-A^{null} genomic locus. Between the HLA-A^{null} left and right homology arms, the plasmid encoded a CMV promoter and SV40 terminator. The aAPX of interest was cloned between the promoter and terminator, generating component 1C'. In this example, component 1C' comprised a single ORF that encoded one aAPX, the HLA-A*02:01 or HLA-B*-35:01, denoted component 1C'^{HLA-A*02:01} component 1C'^{HLA-B*-35:01} respectively.

The integration of component 1C' into component 1B, and selection of monoclone eAPC-p cell lines was as described in example 4, with the exception that a gRNA targeting the HLA-A^{null} genomic locus was used to promote HDR integration of component 1C' into component 1B. Monoclone eAPC-p ACL-191 and ACL-286 expressed HLA-A*02:01 or HLA-B*-35:01 on the cell surface, respectively **(****figure 51a****).**

In this example, step 2 was performed, wherein, the genomic receiver site, component 1D, was the native AAVS1 genomic site, and the targeted integration was achieved through HDR. The genetic donor vector, component 1E was matched to component 1D, by the component 1E that encoded the AAVS1 left and right homology arms, each comprised of >500 bp of sequence homologous to the AAVS1 genomic locus. Between the AAVS1 left and right homology arms, the plasmid encoded a CMV promoter and SV40 terminator. The aAM of interest was cloned between the promoter and terminator, generating Component 1E'. In this example, component 1E' comprised a single ORF that encoded the selection marker, GFP, linked to the aAM ORF, encoding hCMV-pp65, denoted component 1E'^{GFP:2A:pp63}. The viral self-cleaving peptide element encoded a peptide sequence, that when transcribed resulted in self-cleavage of the synthesized peptide and produced two polypeptides, GFP and the intracellular hCMV-pp65 protein.

The integration of component 1E' into component 1D, was as described in example 4. Individual monoclone lines, ACL-391 and ACL-395, were selected as an eAPC-pa on the basis of their maintained selection marker GFP expression **(****figure 51b****).**

In conclusion, the genetically modified ACL-391 and ACL-395 cell lines, which contained a copy of the aAPX HLA-A*02:01 or HLA-B*-35:01 ORF, respectively, within the genomic receiver site, component 1B', and aAM ORF pp65 within the genomic receiver site component 1D' were generated. These genetic modifications resulted in the said aAPX to be the only major HLA class I member expressed on the cell surface of a cell that also expressed the said aAM. Therefore, this demonstrated the creation of two defined **eAPC-pa** cell lines using the multicomponent system.

### Example 8: An eACP-p constructed in one step wherein Component 1C' encoded a single HLAI ORF.

Herein describes the conversion of an eAPC to an eAPC-p in one step, via a single integration couple event, to integrate a single HLAI ORF encoding analyte antigen-presenting complex (aAPX), and wherein the eAPC contains two synthetic genomic receiver sites **Component 1B** and **Component 1D** designed for RMCE based genomic integration. The created eAPC-p has one genomic receiver site occupied by the HLAI ORF **(Component 1B'),** while the remaining **Component 1D** is available for an additional integration couple event.

This example used the eAPC generated in example 3 (ACL-402) containing **Components 1B** and **1D,** wherein **Component 1B** comprises two unique heterospecific recombinase sites, F14 and F15, which flank the ORF that encodes the selection marker, red fluorescent protein (RFP). Encoded 5' of the F14 site is an EF1a promoter and 3' of the F15 site is a SV40 polyadenylation signal terminator. **Component 1D** comprises of two unique heterospecific recombinase sites, FRT and F3, flanking the ORF that encodes the selection marker, blue fluorescent protein (BFP). Encoded 5' of the FRT site, is an EF1a promoter and 3' of the F15 site is a SV40 polyadenylation signal terminator.

This example utilizes a **Component 1C** genetic donor vector, comprising of heterospecific recombinase sites, F14 and F15 and thus is matched to **Component 1B.** Two independent **Component 1C'** were generated from **Component 1C,** wherein one vector (V4.H.5) comprises of a Kozak sequence, start codon and aAPX ORF encoding HLA-A*02:01 between the F14/F15 sites, and wherein the second vector (V4.H.6) comprises a Kozak sequence, start codon and aAPX ORF encoding HLA-A*24:02 between the F14/F15 sites.

The eAPC (ACL-402) was independently combined with vector encoding expression of the RMCE recombinase enzyme (Flp, V4.1.8) and each **Component 1C'** of either V4.H.5 or V4.H.6 by electroporation. Cells were cultured for 4-10 days, whereupon cells were selected and sorted based on loss of the selection marker of integration, RFP, and gain of HLAI on the surface of the cell. Subsequently, individual outgrown monoclone lines were characterized, confirmed and selected on the basis of the gain of HLAI surface expression and the loss of the RFP fluorescence, which indicated that the expected conversion of **Component 1B** to 1**B'** had occurred. Selected eAPC-p monoclones ACL-900 (V4.H.5, HLA-A*02:01) and ACL-963 (V4.H.6, HLA-A*24:02) are negative for RFP compared to the parental ACL-402 cell line and maintain HLAI surface expression (Figure 53a). Furthermore, both monoclones retain expression of the BFP selection marker of integration, indicating that **Component 1D** remains uncoupled and isolated from **Component 1B** integration couple events. To further characterize the eAPC-p monoclones, genomic DNA was extracted from the cells, and confirmation of the integration couple between **Component 1C'** and **Component 1B,** generating **Component 1B',** was conducted by detection of a PCR product specific to **Component 1B'** (Figure 53b). The primers were designed to target a region adjacent to the genomic receiver site (primer ID 8.B.3), and a region within the integration couple event (primer ID 15.H.2). Amplification occurred only in cases of specific integration, while no product was generated from the control (ACL-3) or from off-target recombination.

In summary, this example demonstrates two specific examples of conversion of an eAPC to an eAPC-p, using the multicomponent system, wherein two different aAPX are individually delivered **(Component 1C')** and integrated into a single genomic receiver site **(Component 1B)** by RMCE genomic integration method, subsequently creating a limited library comprising two discrete eAPC-p. Furthermore, it was demonstrated that second genomic receiver site **(Component 1D)** was insulated and unaffected by the **Component 1B/Component 1C'** integration couple.

### Example 9: An eAPC-pa constructed from eAPC-p in one step, wherein Component 1D' encodes a single analyte antigen molecule (aAM) ORF.

The present example describes how multiple eAPC-pa are constructed from a parental eAPC-p (described in example 8) in parallel, wherein the genomic receiver site, **Component 1D,** is targeted for integration by a primed genetic donor vector, **Component 1E',** comprising of a single ORF that encodes an aAM.

In the present example, the parental eAPC-p line used was ACL-900, which expresses a single aAPX (HLA-A*02:01) that is integrated at **Component 1B'** (described in example 8). The eAPC-p **Component 1D** remains open and comprises of two unique heterospecific recombinase sites, FRT and F3, which flank the ORF that encodes the selection marker, blue fluorescent protein (BFP). Encoded 5' of the FRT site, is an EF1a promoter, and 3' of the F15 site is a SV40 polyadenylation signal terminator. The genetic donor vector, **Component 1E** was used in this example and comprises of two heterospecific recombinase sites, F14 and F15, thus being matched to **Component 1D.** In this example, the **Component 1E** was further primed with one aAM ORF of interest selected from HCMVpp28 (V9.E.6), HCMVpp52 (V9.E.7), or HCMVpp65 (V9.E.8), which also each encode a C-terminal glycine-serine rich linker and c-myc tag. Furthermore, each **Component 1E'** further comprises of Kozak sequence and start codon immediately 5' of the aAM ORF. Thus, a small discrete library of **Component 1E'** was created, comprising of three vectors.

The eAPC-p (ACL-900) was independently combined with a vector encoding expression of the RMCE recombinase enzyme (Flp, V4.1.8) and each **Component 1E'** of either V9.E.6, V9.E.7, or V9.E.8 by electroporation. Cells were incubated for 4-10 days to allow for the integration couple to occur, whereupon, individual eAPC-pa were selected and single cell sorted (monoclones) based on diminished signal of the selection marker of integration BFP, encoded by **Component 1D** (Figure 54a). Subsequently, the individual outgrown monoclone eAPC-pa, ACL-1219 (pp28), ACL-1227 (pp52) and ACL-1233 (pp65), were characterized, confirmed and selected on the basis of the loss of BFP expression and maintained surface expression of HLAI (aAPX at **Component 1B')** (Figure 54b), which indicated that the expected conversion of **Component 1D** to **1D'** had occurred. Furthermore, the maintained surface expression of the aAPX indicated that **Component 1B'** was unaffected and isolated from the integration couple event between **Component 1D** and **Component 1E'.** To further characterize the selected eAPC-pa monoclones, genomic DNA was extracted, and confirmation of the integration couple between **Component 1E'** and **Component 1D,** generating **Component 1D'** was conducted by detection of a polymerase chain reaction (PCR) amplicon product specific to **Component 1D'** (10.D.1, 15.H.4). In Figure 54c two monoclones representing each of the three eAPC-pa are shown wherein amplicon products of the expected size for aAM ORF pp28 (0.8kb), pp52 (1.5kb) and pp65 (1.9kb) are observed, further confirming that the expected integration event has occurred.

In summary, this example demonstrates three specific examples of conversion of an eAPC-p to an eAPC-pa, using the multicomponent system, wherein three different aAM are individually delivered (**Component 1E**') and integrated into a single genomic receiver site (**Component 1D**) by RMCE genomic integration method, subsequently creating a small library of three discrete eAPC-pa carrying three different aAM ORF. Furthermore, it was demonstrated that the prior loaded second genomic receiver site **(Component 1B')** was insulated and unaffected by the **Component 1D/Component 1E'** integration couple.

### Example 10: Shotgun integration of multiple analyte antigen molecule ORF into eAPC-p to create a pooled eAPC-pa library in a single step

Herein describes how a pool of primed **Component 1E** vectors (**Component 1E**') collectively encoding multiple aAM ORF (HCMVpp28, HCMVpp52 and HCMVpp65) were integrated in a single step into the parental eAPC-p (described in example 8) to create a pooled eAPC-pa library, wherein each individual cell integrates a single random analyte antigen ORF derived from the original pool of vectors, at **Component 1D**', such that each eAPC-pa expresses a single random aAM, but collectively the pooled library of eAPC-pa represents all of aAM ORF encoded in the original pooled library of vectors. This method of creating a pool of eAPC-pa each expressing a single random ORF from a pool of vectors is referred to as shotgun integration.

In this example, the parental eAPC-p line used was ACL-905 expressing an aAPX (HLA-A*02:01) on the cell surface (the construction of the cell line is described in example 8), **Component 1D** and **Component 1E**' were as described in example 9. In this example, the individual **Component 1E**' vectors of example 9, V9.E.6, V9.E.7, and V9.E.8, comprising of aAM ORFs encoding HCMVpp28, HCMVpp52 and HCMVpp65, respectively, were mixed together in a 1:1:1 molar ratio to create a vector pool. The eAPC-p (ACL-905) was combined with the vector pool and a vector encoding expression of the RMCE recombinase enzyme (Flp, V4.1.8) by electroporation. Cells were incubated for 4-10 days, whereupon, cells were bulk sorted on the basis of having diminished signal for the selection marker of integration, BFP, encoded by **Component 1D** (Figure 55a) generating the pooled cell population ACL-1050 (Figure 55b).

To confirm that the eAPC-pa pool ACL-1050 was comprised of a mixture of eAPC-pa each encoding one of HCMVpp28, HCMVpp52 or HCMVpp65 at **Component 1D**', individual cells were single cell sorted from the polyclonal population and 12 were selected at random for genetic characterisation. Amplification of the **Component 1D**' was conducted using primers that span each aAM (10.D.1 and 15.H.4). In Figure 55c, the amplicons generated for the 12 cells are presented, with controls, wherein for all 12 cells a single amplicon product consistent with the expected size for one of the aAM ORF, pp28 (0.8kb), pp52 (1.5kb) and pp65 (1.9kb) is observed. Furthermore, each aAM ORF is identified at least once indicating that the eAPC-pa pool is comprised of a mixture eAPC-pa wherein each eAPC-pa in the pool has integrated a single random aAM ORF from the original pool of three vectors.

In conclusion, this example demonstrates the use of the multicomponent system for conversion of an eAPC-p into a pooled library of eAPC-pa in a single step, by combining the eAPC-p with a pooled library of three vectors encoding three different analyte antigen molecules (**Component 1E**') and utilizing a RMCE based shotgun integration approach. Furthermore, this example demonstrates that each eAPC-pa within the generated pool of eAPC-pa has integrated a single random aAM ORF from the original vector pool by an integration couple event between **Component 1D** and **Component 1E**', and that all three aAM ORF are represented within the generated pooled eAPC-pa library.

### Example 11: Demonstration of eTPC-t generation in one step

The present example describes the generation of eTPC-t in a standardised manner, wherein the parental eTPC contains distinct synthetic genomic receiver sites **Components 2B and 2D.** All eTPC parental lines described in this example and all further examples were generated with the same techniques as were the eAPC lines presented in above examples. The genetic donor vectors **Components 2C' and 2E'** comprised a single chain of a TCR pair (JG9-TCR) known to engage with the antigenic peptide NLVPMVATV (NLVP) derived from Human Cytomegalovirus polypeptide 65 (HCMV pp65) when presented in HLA-A*02 alleles. Components C' and E' are designed for RMCE, and derived from parental **Components 2C and 2E.**

This example uses a parental eTPC cell line ACL-488, which is TCR null, HLA null, CD4 null and CD8 null, and further containing **Component 2B** and 2**D**. **Component 2B** comprises two unique heterospecific recombinase sites, FRT and F3 that flank a Kozak sequence and ORF encoding the selection marker, blue fluorescent protein (BFP). Encoded 5' of the FRT site, is an EF1a promoter and 3' of the F3 site is a SV40 polyadenylation signal terminator. **Component 2D** comprises two unique heterospecific recombinase sites, F14 and F15, which were different to **Component 2B**. These sites flank a Kozak sequence and ORF that encodes the selection marker, the red fluorescent protein, (RFP). Encoded 5' of the F14 site is an EF1a promoter, and 3' of the F15 site is a SV40 polyadenylation signal terminator.

This example uses genetic donor vectors, **Component 2C' and Component 2E'**, each comprising of two heterospecific recombinase sites, FRT/F3 (2**C**') and F14/F15 (2**E**'), thus being matched to **Component 2B** and 2**D**, respectively. **Component 2C'** further comprises, between the FRT/F3 sites, of a Kozak sequence, start codon and TCR ORF encoding JG9-TCR-beta chain. **Component 2E'** further comprises, between the F14/F15 sites, of a Kozak sequence, start codon and TCR ORF encoding JG9-TCR-alpha chain.

An eTPC-t was created through RMCE by electroporation ACL-488 (eTPC). Four to ten days after electroporation, individual cells displaying diminished fluorescent protein signal, BFP and RFP, encoded by **Components 2D** and **2B** selection markers, were sorted by FACS. Individual monoclones were out grown and then phenotypically assessed. The resulting monoclone, ACL-851, was BFP and RFP negative (Figure 56 a and b). ACL-851 also showed TCR and CD3 surface expression while the parental cell line did not (Figure 56 c and e). Furthermore, the introduced JG9-TCR showed specific staining with the HLA-A*02:01- NLVP tetramer, indicating that it is a functional TCRsp on the surface of the eTPC-t (Figure 56 d to f). ACL-851 was confirmed by PCR to contain the TCRsp encoded by **Component 2B'** and **Component 2D'** integrated into the genome (Figure 56 g and h).

In summary, an eTPC was converted to an eTPC-t, by use of an RMCE based integration method to integrate TCR ORF delivered in **Component 2C'** and 2**E'**, such that **Components 2B** and 2**D** were converted into **Component 2B'** and 2**D**', and where by this eTPC-t expressed a functional TCRsp on the surface of the cell. Furthermore, this example demonstrates operation of a simple eTPC:A system, where a binary composition of an eTPC-t and analyte antigen were combined and the eTPC-t selected based on a complex formation between the soluble analyte antigen (HLA multimer: HLA-A*02:01-N LV PMVATV).

### Example 12: Demonstration of conversion of eTPC-t to an eTPC-x

The present example describes conversion of an eTPC-t to an eTPC-x, wherein the eTPC-x has **component 2B'** encoding a TCR chain ORF and Component 2D is available for integration of complementary TCR chain ORF. Conversion of Component 2D' of the eTPC-t to Component 2D of the eTPC-x is achieved by use of a genetic donor vector (Component 2Z) matched to Component 2D'.

In this example, the parental eTPC-t cell line ACL-851 generated in example 11 was used. Component 2Z is a plasmid vector comprised of two heterospecific recombinase sites, F14/F15 matched to Component 2D', a Kozak sequence, start codon and an ORF encoding a green fluorescent protein (GFP) as a selection marker of integration. The eTPC-t was combined with **Component 2Z** and a vector encoding RMCE recombinase enzyme by electroporation, whereupon the cells were subsequently selected for loss of CD3 presentation and gain of the GFP selection marker of integration. The monolcone ACL-987 was phenotypically characterised by FACS, and it was observed that the ACL-987 has gained GFP and lost CD3 and TCRab (Figure 57b, d), indicating successful exchange of JG9-TCR-alpha with the GFP ORF and conversion of Component 2D' to Component 2D, and thus generation of an eTPC-x. In comparison the parental eTPC-t, ACL-851, is lacking GFP expression and has CD3 and TCRab surface expression (Figure 57 a, c).

In summary, this example demonstrates conversion of an eTPC-t to an eTPC-x, with removal of the JG9-TCR-alpha TCR ORF at Component 2D' in exchange for the GFP selection marker of integration thereby creating Component 2D, for further integration coupling events of alternative complementary TCR chain ORF. This conversion was conducted using the RMCE method for genomic integration.

### Example 13: Demonstration of shotgun integration into eTPC-x to create pool of eTPC-t

The present example describes how a pool of vectors encoding 64 single JG9-TCR-alpha variants (as **Component 2E'**) were integrated as a single step into a parental eTPC-x cell line (described in example 12) to create a pooled eTPC-t library wherein each individual cell integrated a single random TCR ORF encoding alpha chain from the original pool of vectors, such that each eTPC-t expresses a single random pair of TCR ORF as TCRsp. Combined together the individual eTPC-t comprises a pooled a library of eTPC-t wherein the pool of cells potentially represents all possible combinations of the 64 TCR-alpha paired with constant original TCR beta chain. Such a method is now referred to as 'shotgun' integration. The 64 JG9-TCR-alpha variants have been created by modifying the CDR3 sequence which falls at the junction of the V and J fragments.

In this example, the parental eTPC-x cell line ACL-987 (see example 12), with non-surface expressed JG9-TCR-beta (**Component 2B'**) and CD3 complex was used. **Component 2D** encodes GFP, a selection marker, as described in example 12. In this example, the 64 JG9-TCR-alpha variant fragments were cloned into the Component 2E donor vector, creating **Component 2E'**, flanked by F14/ F15 sites, matching to Component 2D. The 64 vectors were subsequently combined into a single pool of vectors.

An eTPC-t pool was created via RMCE based genomic integration, wherein the eTPC-x (ACL-987) and 64 **Components 2E'** and RMCE recombinase vector were combined by electroporation. Polyclones were selected on the basis of the GFP expression. The resulting polyclone, ACL-988, comprised of both GFP positive and GFP negative cell populations, unlike the parental line which comprised of only GFP positive cells (Figure 58 a and b). However, only GFP negative population showed consistently strong CD3 expression, indicating successful conversion of **Component 2D** into **Component 2D'** and therefore the eTPC-x has been converted into a pool of eTPC-t (Figure 58 c and d). Furthermore, ACL-988 GFP negative populations showed two distinct intensities when stained with the JG9-TCR specific multimer reagent (DEX HLA-A*02:01-NLVP), suggesting that this population comprises of cells that express TCR variants with varying binding efficiency.

In parallel, all 64 JG9-TCR-alpha variants were cloned into an expression construct that permitted transient transfection to a parental eTPC-x (ACL-987) and relative staining units (RSU) against the HLA-A*02:01-NLVP multimer reagent to a reference for each TCR pair presented in the above-described pooled eTPC-t expressing variant JG9-TCR were determined. RSU were calculated as the ratio of the mean fluorescence intensity (MFI) of HLA multimer signal for the CD3 positive population over the CD3 negative population, and was indicative of the binding strength of each TCR chain pair variant. After the independent transfection of the parental ACL-987 line with each JG9-TCR-alpha variant, the cells were stained with antibodies against CD3 and with the HLA-multimer reagent and analysed by flow cytometry. Each point plotted in Figure 58e represent the observed RSU for each 64 variants.

Individual cells from the pool of ACL-988 were single cell sorted, from the HLA-multimer positive population and the HLA-multimer negative population. The Component 2D' encoding the variant JG9-TCR-alpha ORF for each single cell were amplified and sequenced and compared to the results of the transient expressions RSU units described above (Figure 58e). Indeed, individual ACL-988 cells that were HLA-multimer positive encoded JG9-TCR-alpha variants that predominantly showed high RSU results in the individually tested variants (Figure 58e, open circles). Moreover, individual ACL-988 cells that were pHLA-multimer negative encoded JG9-TCR-alpha variants that predominantly showed low RSU results (Figure 58e open triangles).

In conclusion, this example demonstrates use of the multi-component system for conversion of an eTPC-x and a pooled library of vectors (component 2E') into a pooled library of eTPC-t containing multiple different TCRsp. This was achieved in a single step using shotgun integration. Moreover, this example demonstrated that the pool of eTPC-t were combined with an analyte antigen (as a soluble affinity reagent) into an eTPC:A system, wherein it was demonstrated that single cells of the pool could be selected on the basis of complex formation with the analyte antigen (HLA-multimer) and wherein the subsequent TCR chain ORF encoded in Component 2D' were extracted and DNA sequences obtained.

### Example 14: Functional demonstration of component 2F in combined eAPC:eTPC system with exogenous aAM

Herein describes an eTPC cell line (ACL-1063, Component 2**A**) engineered with two unique genomic receiver sites (Components 2**B**, **2D**), engineered to be HLA Null, utilizing native CD3 expression, and harbouring an engineered genomic two-component, synthetic response element (Component 2**F**). In this example, the eTPC cell line was converted to an eTPC-t cell line (ACL-1277) as described previously in example 11, wherein the TCR chain ORF at Component 2B' and 2E' encode a TCR pair that is specific for HLA-peptide complex (HLA), HLA-A*02:01-NLVPMVATV. This eTPC-t was combined with above-described eAPC-p in the presence of exogenous aAM in the form of soluble peptide to assemble a eAPC:eTPC systems. The readout of these combined systems was an RFP signal within the eTPC-t, which was the reporter from component 2F.

The response elements defined as Component 2F comprised of a Driver-Activator component and an Amplifier-Report component, wherein both units utilized synthetic promoters. The Driver is a synthetic promoter that is responsive to the native TCR signalling pathways, encoding three sets of tandem transcription factor binding sites for NFAT-AP1-NFkB (3xNF-AP-NB). Upon transcriptional activation, the Driver induces expression of the Activator protein, a synthetic designed transcription factor derived by fusion of the Herpes VP16 activation domain, the GAL4 DNA binding domain and two nuclear localization signals at the N- and C-terminals (NV16G4N), to which the cognate DNA recognition sequence is present 6 times in tandem in the Amplifier promoter. Both the Driver and Amplifier promoters utilized the core promoter sequence (B recognition element (BRE), TATA Box, Initiator (INR) and transcriptional start site) from HCMV IE1 promoter, immediately 3' of the respective transcription factor binding sites. The Amplifier upon transcriptional activation drives expression of the reporter, red fluorescent protein (RFP).

The eTPC-t cell line was then challenged against eAPC-p presenting HLA-A*02:01 (ACL-209) or HLA-A*24:02 (ACL-963) or was HLA-null parental eAPC (ACL-128). Wherein analyte eAPC-pa were prepared by pulsing eAPC-p with analyte antigen of either peptide NLVPMVATV or VYALPLKML, or no peptide. Subsequently, an eTPC-t and analyte eAPC-pa were compiled into an eAPC:eTPC system consisting of 30,000 eTPC-t co-cultured with 10,000 eAPC-pa for 24h. After 24h the cells were harvested, washed, stained with markers specific for the eTPC-t and analyte eAPC-pa in order to distinguish the populations, and analysed by flow cytometry. Strong activation of the eTPC-t, Component 2F (was only observed in eTPC-t challenged with analyte eAPC-pa presenting the known cognate antigen pHLA complex, i.e. the eAPC-pa with HLA-A*02:01 and NLVPMVATV (Figure 59a). In contrast only resting state RFP expression was observed in eAPC:eTPC compilations comprised of non-specific analyte eAPC-pa (Figure 59b, c, d) or control parental eAPC lacking HLA (Figure 59f) or eAPC-p lacking exogenous aAM peptide (Figure 59e).

In conclusion, an eTPC-t cell line containing a functional component 2F was engineered, and subsequently used to create an eTPC-t. Upon interaction of the eTPC-t with analyte eAPC-pa presenting its cognate target T-cell antigen, provided as exogenous soluble aAM, a response was measurable as an increase in RFP expression. Conversely, when contacted with analyte eAPC or eAPC-p or eAPC-pa not presenting a cognate T-cell antigen and HLA, or no HLA, no measurable increase in RFP expression above background was exhibited by the eTPC-t. Furthermore, this example demonstrates an eAPC:eTPC system wherein analyte eAPC-pa and analyte eTPC-t are compiled in discrete binary compositions and the eTPC-t response is used to identify both the analyte eAPC-pa and eTPC-t wherein a co-operative complex between the TCRsp and analyte antigen occurs.

### Example 15: Functional demonstration of component 2F in combined eAPC:eTPC system with aAM ORF integrated to eAPC-pa state

The present example describes the use of an eTPC-t (ACL-1150), (Component 2A) engineered with two unique genomic receiver sites, loaded with the TCR chain ORF at Component 2B' and 2E' encoding a TCR pair that is specific for HLA-peptide complex (HLA), HLA-A*02:01-NLVPMVATV, utilizing native CD3 expression, and harbouring a genomic one-component, synthetic response element (Component 2F), compiled into an eAPC:eTPC system with eAPC-pa that were generated by integration with aAM encoding ORF integrated at site 1D'. In this example, four different eAPC-pa variants were assembled with two different HLA alleles, and two different aAM ORF derived from the HCMV genome; generating four distinct eAPC-pa lines.

The response elements defined as Component 2F comprised of a Driver-Reporter component. The Driver is a synthetic promoter that is responsive to the native TCR signalling pathways, encoding six sets of tandem transcription factor binding sites for NFAT-AP1 (6xNF-AP) and utilizes the core promoter sequence (B recognition element (BRE), TATA Box, Initiator (INR) and transcriptional start site) from HCMV IE1 promoter, immediately 3' of the respective transcription factor binding sites. Upon transcriptional activation, the Driver induces expression of the reporter, red fluorescent protein (RFP).

The first eAPC-pa line (ACL-1046) expresses HLA-A*02:01 and the full-length ORF for HCMV protein pp65, wherein pp65 contains the antigenic peptide recognised by the eTPC-t TCRsp, when presented in HLA-A*02:01. The second eAPC-pa line (ACL-1044) expresses HLA-A*02:01 and the full-length ORF for HCMV protein pp52. The third eAPC-pa line ACL-1045 expresses HLA-B*07:02 and the full-length ORF for HCMV protein pp52. The fourth eAPC-pa line (ACL-1048) expresses HLA-B*07:02 and the full-length ORF for HCMV protein pp65. The second, third and fourth eAPC-pa express aAPX:aAM complexes not recognised by the eTPC-t TCRsp.

The eTPC-t cell line ACL-1150 was compiled into independent eAPC:eTPC system with each of the four eAPC-pa as described above. After 48h the cells were harvested, washed, stained with markers specific for the eTPC-t and analyte eAPC-pa in order to distinguish the populations, and analysed by flow cytometry. Strong activation of the eTPC-t, Component 2F, was only observed in eTPC-t challenged with analyte eAPC-pa presenting the known cognate antigen pHLA complex, i.e. the eAPC-pa with HLA-A*02:01 and pp65 (Figure 60b). In contrast only resting state RFP expression was observed in eAPC:eTPC compilations comprised of non-specific analyte eAPC-pa (Figure 60a, c, d).

In conclusion, an eTPC-t cell line containing a functional component 2F was engineered, and subsequently used to create an eTPC-t. Upon interaction of the eTPC-t with analyte eAPC-pa presenting its cognate target T-cell antigen, provided as endogenous aAM ORF by integration, a response was measurable as an increase in RFP expression. Conversely, when contacted with analyte eAPC-pa not presenting a cognate T-cell antigen and HLA, no measurable increase in RFP expression above background was exhibited by the eTPC-t. Furthermore, this example demonstrates an eAPC:eTPC system wherein analyte eAPC-pa and analyte eTPC-t are compiled in discrete binary compositions and the eTPC-t response is used to identify both the analyte eAPC-pa and eTPC-t wherein a co-operative complex between the TCRsp and analyte antigen occurs.

SEQUENCE LISTING

<110> Genovie AB

<120> An engineered two-part cellular device for discovery and characterisation of T-
cell receptor interaction with cognate antigen

<130> ANO15

<160> 104

<170> BiSSAP 1.3

<210> 1 <223> Analyte Antigenic Peptide

<210> 2 <223> Analyte Antigenic Peptide

<210> 3 <223> HCMV Antigen

<210> 4 <223> HCMV Antigen

<210> 5 <223> pcDNA3.1_GFP vector V1.A.4

<210> 6 <223> pcDNA3.1_RFP vector V1.A.6

<210> 7 <223> pMA-SV40pA vector V1.C.2

<210> 8 <223> pMA-CS-JG9-TCRbeta vector V3.C.5

<210> 9 <223> pMA-F14-GFP-F15 vector V4.H9

<210> 10 <223> pMA-F14-TCR-JG9-alpha-F15 vector V7.A.3

<210> 11 <223> pMA-FRT-TCR-JG9-beta-F3 vector V7.A.4

<210> 12 <223> F14-TCRaF15 CDR3degen.64mix vector V8.F.8

<210> 13 <223> CMVpro-Flp-sv40pA-V2 vector V4.I.8

<210> 14 <223> CDR3 sequence of a JG9-TRA 64 variant VP.7751.RC1_A1

<210> 15 <223> CDR3 sequence of a JG9-TRA 64 variant VP.7751.RC1_A2

<210> 16 <223> CDR3 sequence of a JG9-TRA 64 variant VP.7751.RC1_A3

<210> 17 <223> CDR3 sequence of a JG9-TRA 64 variant VP.7751.RC1_A4

<210> 18 <223> CDR3 sequence of a JG9-TRA 64 variant VP.7751.RC1_A5

<210> 19 <223> CDR3 sequence of a JG9-TRA 64 variant VP.7751.RC1_A6

<210> 20 <223> CDR3 sequence of a JG9-TRA 64 variant VP.7751.RC1_A7

<210> 21 <223> CDR3 sequence of a JG9-TRA 64 variant VP.7751.RC1_A8

<210> 22 <223> CDR3 sequence of a JG9-TRA 64 variant VP.7751.RC1_B1

<210> 23 <223> CDR3 sequence of a JG9-TRA 64 variant VP.7751.RC1_B2

<210> 24 <223> CDR3 sequence of a JG9-TRA 64 variant VP.7751.RC1_B3

<210> 25 <223> CDR3 sequence of a JG9-TRA 64 variant VP.7751.RC1_B4

<210> 26 <223> CDR3 sequence of a JG9-TRA 64 variant VP.7751.RC1_B5

<210> 27 <223> CDR3 sequence of a JG9-TRA 64 variant VP.7751.RC1_B6

<210> 28 <223> CDR3 sequence of a JG9-TRA 64 variant VP.7751.RC1_B7

<210> 29 <223> CDR3 sequence of a JG9-TRA 64 variant VP.7751.RC1_B8

<210> 30 <223> CDR3 sequence of a JG9-TRA 64 variant VP.7751.RC1_C1

<210> 31 <223> CDR3 sequence of a JG9-TRA 64 variant VP.7751.RC1_C2

<210> 32 <223> CDR3 sequence of a JG9-TRA 64 variant VP.7751.RC1_C3

<210> 33 <223> CDR3 sequence of a JG9-TRA 64 variant VP.7751.RC1_C4

<210> 34 <223> CDR3 sequence of a JG9-TRA 64 variant VP.7751.RC1_C5

<210> 35 <223> CDR3 sequence of a JG9-TRA 64 variant VP.7751.RC1_C6

<210> 36 <223> CDR3 sequence of a JG9-TRA 64 variant VP.7751.RC1_C7

<210> 37 <223> CDR3 sequence of a JG9-TRA 64 variant VP.7751.RC1_D1

<210> 38 <223> CDR3 sequence of a JG9-TRA 64 variant VP.7751.RC1_D2

<210> 39 <223> CDR3 sequence of a JG9-TRA 64 variant VP.7751.RC1_D3

<210> 40 <223> CDR3 sequence of a JG9-TRA 64 variant VP.7751.RC1_D4

<210> 41 <223> CDR3 sequence of a JG9-TRA 64 variant VP.7751.RC1_D5

<210> 42 <223> CDR3 sequence of a JG9-TRA 64 variant VP.7751.RC1_D6

<210> 43 <223> CDR3 sequence of a JG9-TRA 64 variant VP.7751.RC1_D7

<210> 44 <223> CDR3 sequence of a JG9-TRA 64 variant VP.7751.RC1_D8

<210> 45 <223> CDR3 sequence of a JG9-TRA 64 variant VP.7751.RC1_E1

<210> 46 <223> CDR3 sequence of a JG9-TRA 64 variant VP.7751.RC1_E2

<210> 47 <223> CDR3 sequence of a JG9-TRA 64 variant VP.7751.RC1_E3

<210> 48 <223> CDR3 sequence of a JG9-TRA 64 variant VP.7751.RC1_E4

<210> 49 <223> CDR3 sequence of a JG9-TRA 64 variant VP.7751.RC1_E5

<210> 50 <223> CDR3 sequence of a JG9-TRA 64 variant VP.7751.RC1_E6

<210> 51 <223> CDR3 sequence of a JG9-TRA 64 variant VP.7751.RC1_E7

<210> 52 <223> CDR3 sequence of a JG9-TRA 64 variant VP.7751.RC1_E8

<210> 53 <223> CDR3 sequence of a JG9-TRA 64 variant VP.7751.RC1_F1

<210> 54 <223> CDR3 sequence of a JG9-TRA 64 variant VP.7751.RC1_F2

<210> 55 <223> CDR3 sequence of a JG9-TRA 64 variant VP.7751.RC1_F3

<210> 56 <223> CDR3 sequence of a JG9-TRA 64 variant VP.7751.RC1_F4

<210> 57 <223> CDR3 sequence of a JG9-TRA 64 variant VP.7751.RC1_F5

<210> 58 <223> CDR3 sequence of a JG9-TRA 64 variant VP.7751.RC1_F6

<210> 59 <223> CDR3 sequence of a JG9-TRA 64 variant VP.7751.RC1_F7

<210> 60 <223> CDR3 sequence of a JG9-TRA 64 variant VP.7751.RC1_F8

<210> 61 <223> CDR3 sequence of a JG9-TRA 64 variant VP.7751.RC1_G1

<210> 62 <223> CDR3 sequence of a JG9-TRA 64 variant VP.7751.RC1_G2

<210> 63 <223> CDR3 sequence of a JG9-TRA 64 variant VP.7751.RC1_G3

<210> 64 <223> CDR3 sequence of a JG9-TRA 64 variant VP.7751.RC1_G4

<210> 65 <223> CDR3 sequence of a JG9-TRA 64 variant VP.7751.RC1_G5

<210> 66 <223> CDR3 sequence of a JG9-TRA 64 variant VP.7751.RC1_G6

<210> 67 <223> CDR3 sequence of a JG9-TRA 64 variant VP.7751.RC1_G7

<210> 68 <223> CDR3 sequence of a JG9-TRA 64 variant VP.7751.RC1_G8

<210> 69 <223> CDR3 sequence of a JG9-TRA 64 variant VP.7751.RC1_H1

<210> 70 <223> CDR3 sequence of a JG9-TRA 64 variant VP.7751.RC1_H2

<210> 71 <223> CDR3 sequence of a JG9-TRA 64 variant VP.7751.RC1_H3

<210> 72 <223> CDR3 sequence of a JG9-TRA 64 variant VP.7751.RC1_H4

<210> 73 <223> CDR3 sequence of a JG9-TRA 64 variant VP.7751.RC1_H5

<210> 74 <223> CDR3 sequence of a JG9-TRA 64 variant VP.7751.RC1_H6

<210> 75 <223> CDR3 sequence of a JG9-TRA 64 variant VP.7751.RC1_H7

<210> 76 <223> CDR3 sequence of a JG9-TRA 64 variant VP.7751.RC1_H8

<210> 77 <223> pMA_F14_HLA-A*02:01-6xHis_F15 vector V4.H.5

<210> 78 <223> pMA_F14_HLA-A*24:02-6xHis_F15 vector V4.H.6

<210> 79 <223> pMA_F14_HLA-B*07:02-6xHis_F15 vector V4.H.7

<210> 80 <223> pMA_F14_HLA-B*35:01-6xHis_F15 vector V4.H.8

<210> 81 <223> FRT_HCMVpp28-3xMYC_F3 vector V9.E.6

<210> 82 <223> FRT_HCMVpp52-3xMYC_F3 vector V9.E.7

<210> 83 <223> FRT_HCMVpp52-3xMYC_F3 vector V9.E.8

<210> 84 <223> SpCas9-2A-GFP Vector V1.A.8

<210> 85 <223> HLA-A-sg-sp-opti1 vector V2.A.1

<210> 86 <223> HLA-B-sg-sp-3 vector V2.A.7

<210> 87 <223> HLA-C-sg-sp-4 vector V2.B.3

<210> 88 <223> HLA-A-ex2-3_sg-sp-opti_1 vector V2.I.10

<210> 89 <223> HLA-A-ex2-3_sg-sp-opti_2 vector V2.J.1

<210> 90 <223> AAVSI_sg-sp-opti_3 vector V2.J.6

<210> 91 <223> TRAC-GT-F1 ddPCR primer/probe

<210> 92 <223> TRAC-GT-R1 ddPCR primer/probe 1.F.8

<210> 93 <223> TRAC-probe-FAM ddPCR primer/probe

<210> 94 <223> TRBC2-GT-F1 ddPCR primer/probe 1.F.9

<210> 95 <223> TRBC2-GT-R1 ddPCR primer/probe 1.F.10

<210> 96 <223> TRBC2-probe-FAM ddPCR primer/probe 1.G.2

<210> 97 <223> TRAC-TCRA-ex1-F1 ddPCR primer/probe 10.A.9

<210> 98 <223> TRAC-TCRA-ex1-F1 ddPCR primer/probe 10.A.10

<210> 99 <223> TRAC-probe(HEX) ddPCR primer/probe 10.B.6

<210> 100 <223> HCMVpp65_GT_F2ddPCR primer/probe 21.I.1

<210> 101 <223> HCMVpp28_GT_F1 ddPCR primer/probe 21.I.2

<210> 102 <223> HCMVpp52_GT_F1 ddPCR primer/probe 21.I.3

<210> 103 <223> Myc-Tag_GT_R1 ddPCR primer/probe 20.H.10

<210> 104 <223> Linker-Myc_Probe_Fam ddPCR primer/probe 20.H.9

### List of abbreviations

- **aAPX**: Analyte antigen-presenting complex
- **aAM**: Analyte antigenic molecule
- **aCT**: Analyte TCR
- **APC**: Antigen-presenting cell
- **APX**: Antigen-presenting complex
- **BFP**: Blue fluorescent protein
- **CAR-T**: CAR T-cell
- **CM**: Cargo molecules
- **CRISPR**: Clustered Regularly Interspaced Short Palindromic Repeats
- **gRNA**: Cas9 guide RNA
- **CAR**: Chimeric antigen receptor
- **CDR**: Complementarity-determining regions
- **C-region**: Constant region
- **CMV**: Cytomegalovirus
- **DAMPS**: Danger associated molecular patterns
- **DC**: Dendritic cells
- **DNA**: Deoxyribonucleic acid
- **D-region**: Diversity region
- **eAPC**: Engineered antigen-presenting cell
- **eAPC-p**: Engineered antigen-presenting cell that present an analyte antigen-presenting complex
- **eAPC-pa**: Engineered antigen-presenting cell that presents an analyte anti-gen-presenting complex and analyte antigenic molecule
- **eAPC-a**: Engineered antigen-presenting cell expressing an analyte antigenic molecule
- **eAPCS**: Engineered antigen-presenting cell system
- **eTPC**: Engineered TCR-presenting cell
- **eTPCS**: Engineered TCR-presenting cell system
- **eTPC-t**: Engineered TCR-presenting cell that present full-length TCR pairs
- **FACS**: Fluorescence-activated cell sorting
- **GEM T-cells**: Germ line-encoded mycolyl-reactive T-cells
- **GFP**: Green fluorescent protein
- **HLAI**: HLA class I
- **HLAII**: HLA class II
- **HDR**: Homology directed recombination
- **HLA**: Human leukocyte antigen
- **IgSF**: Immunoglobulin superfamily
- **IRES**: Internal ribosome entry site
- **iNK T-cells**: Invariant natural killer T-cells
- **J-region**: Joining region
- **MACS**: Magnetic-activated cell sorting
- **MAGE**: Melanoma associated antigen
- **MAIT**: Mucosal-associated invariant T
- **NCBP**: Non-cell based particles
- **ORF**: Open reading frame
- **PAMPS**: Pathogen-associated molecular patterns
- **PCR**: Polymerase chain reaction
- **RMCE**: Recombinase mediated cassette exchange
- **RFP**: Red fluorescent protein
- **DNA**: Ribonucleic acid
- **SH2**: Src homology 2
- **T-cells**: T lymphocytes
- **TCR**: T-cell Receptor
- **TRA**: TCR alpha
- **TRB**: TCR beta
- **TRD**: TCR delta
- **TCRsp**: TCR surface proteins in complex with CD3
- **TALEN**: Transcription activator-like effector nucleases
- **TRG**: TRC gamma
- **TAA**: Tumour-associated-antigens
- **V-region**: Variable region
- **β2M**: β2-microglobulin
- **ZAP-70**: ζ-chain-associated protein of 70 kDa

### Definitions

**A pair of complementary TCR chains**: two TCR chains wherein the translated proteins are capable of forming a TCRsp on the surface of a TCR presenting cell
**Affinity**: Kinetic or equilibrium parameter of an interaction between two or more molecules or proteins
**Affinity reagent**: Any reagent designed with specific affinity for an analyte. Often used in the context of affinity for HLA-antigen complex
**Allele**: Variant form of a given gene
**AM**: Analyte antigenic molecule. Generally, a protein but could also be a metabolite that is expressed by a cell from their genomic DNA and/or a specific introduced genetic sequence. The AM is expressed in the cell and a fragment can then be presented on the cell surface by an APX as cargo or on its own. Either as cargo or not, the AM can then be the target of T-cell receptor bearing cells or related affinity reagents.
**Amplicon**: a piece of DNA or RNA that is the source and/or product of artificial amplification using various methods including PCR.
**Analyte**: an entity that is of interest to be identified and/or measured and/or queried in the combined system
**Antibody**: Affinity molecule that is expressed by specialized cells of the immune system called B-cells and that contains of two chains. B-cells express a very large and very diverse repertoire of antibodies that do generally not bind self proteins but can bind and neutralize pathogens or toxins that would threaten the host. Natural or artificially engineered antibodies are often used as affinity reagents.
**Antigen**: any molecule that may be engaged by a TCR and results in a signal being transduced within the T-cell, often presented by an antigen-presenting complex
**Analyte antigen**: collectively the eAPC:eTPC system representing any entity presenting an antigen for analytical determination
**APC**: Antigen-presenting cell. A cell baring on the surface of the cell an AM, APX, APX
**APX**: Antigen-presenting complex. A protein that is expressed and presented on the cell surface by nucleated cells from genes/ORF encoding genomic DNA and/or a specific introduced genetic sequence. The APX presents a cargo, being either a peptide or other metabolite molecules.
**C-Region**: Constant region. One of the gene segments that is used to assemble the T-cell receptor. The c-region is a distinct segment that rather than driving diversity of the TCR, defines its general function in the immune system.
**Cargo-loading machinery**: Cellular set of proteins that generate and load cargo molecules on APX from proteins or other presented molecules found in the cell.
**CDR**: complementarity-determining regions. Short sequences on the antigen-facing end of TCRs and antibodies that perform most of the target binding function. Each antibody and TCR contains six CDRs and they are generally the most variable part of the molecules allowing detection of a large number of diverse target molecules.
**CM**: Cargo molecules. peptide or metabolite that is presented by an antigen-presenting complex for example a HLA I or HLA II. The CM can be expressed by the cell intrinsically from the genomic DNA, introduced into the culture medium or expressed from a specifically introduced genetic sequence.
**Copy-number**: The whole number occurrence of a defined sequence encoded within the genome of a cell
**Cytogenetic**: The study of inheritance in relation to the structure and function of chromosomes, i.e. determine the karyotype of a cell
**Cytotoxic/Cytotoxicity**: Process in which a T-cells releases factors that directly and specifically damage a target cell.
**D-region**: Diversity region. One of the gene segments that is used to assemble the T-cell receptor. Each individual has a large number of different variations of these regions making it possible for each individual to arm T-cells with a very large variety of different TCR.
**DNA**: Desoxyribonucleic acid. Chemical name of the molecule that forms genetic material encoding genes and proteins.
**eAPC system**: eAPCS, the system by which eAPC-pa, eAPC-p and eAPC-a cells, or libraries thereof, are prepared for combination in the eAPC:eTPC system.
**eTPC system**: eTPCS, the system by which eTPC-t cells, or libraries thereof, are prepared for combination in the eAPC:eTPC system
**eAPC:eTPC system:** the system by which analyte antigen presented by eAPC and analyte TCR presented by eTPC are combined
**Endogenous**: Substance that originated from within a cell
**Engineered Cell**: A cell whereby the genome has been engineered through genetic modification modified.
**Eukaryotic conditional regulatory element**: A DNA sequence that can influence the activity of a promoter, which may be induced or repressed under defined conditions
**Eukaryotic Promoter**: A DNA sequence that encodes a RNA polymerase biniding site and response elements The sequence of the promoter region controls the binding of the RNA polymerase and transcription factors, therefore promoters play a large role in determining where and when your gene of interest will be expressed.
**Eukaryotic terminator/Signal terminator**: A DNA sequence that are recognized by protein factors that are associated with the RNA polymerase II and which trigger the termination process of transcription. It also encodes the poly-A signal
**FACS/Flow Cytometry**: Fluorescence-activated cell sorting. Analytical technique by which individual cells can be analyzed for the expression of specific cell surface and intracellular markers. A variation of that technique, cell sorting, allows cells that carry a defined set of markers to be retrieved for further analysis.
**Family of APX**: A set of several similar genes that encode functionally related proteins, which constitute an antigen pressing complex
**Fluorescent (protein) marker**: Molecule that has specific extinction and emission characteristics and can be detected by Microscopy, FACS and related techniques.
**Genetic Donor vector**: A genetic based vector for delivery of genetic material to the genomic receiver site
**Genomic Receiver Site**: A site within the genome for targeted integration of donor genetic material encoded within a Genetic Donor Vector.
**Heterospecific recombinase sites**: A DNA sequence that is recognized by a recombinase enzyme to promote the crossover of two DNA molecules
**HLA I**: Human Leukocyte Antigen class I. A gene that is expressed in humans in all nucleated cells and exported to the cell surface where it presents as cargo short fragments, peptides, of internal proteins to T-cell receptors. As such it presents fragments of potential ongoing infections along with intrinsic proteins. The HLA I can additionally present as cargo peptides that are added to the culture medium, generated from proteins expressed form introduced genetic elements or generated from proteins that are taken up by the cell. HLA class I genes are polymorphic meaning that different individuals are likely to have variation in the same gene leading to a variation in presentation. Related to HLA class II.
**HLA II**: Human Leukocyte Antigen Class II. A gene that is expressed in humans in specific cells that are coordinating and helping the adaptive immune response for example dendritic cells. Related to HLA class I. HLA class II proteins are exported to the cell surface where they present as cargo short fragments, peptides, of external proteins to T-cell receptors. As such it presents fragments of potential ongoing infections along with intrinsic proteins. The HLA II can additionally present as cargo peptides that are added to the culture medium, generated from proteins expressed form introduced genetic elements or generated from proteins that are taken up by the cell. HLA class II genes are polymorphic meaning that different individuals are likely to have variation in the same gene leading to a variation in presentation.
**Homologous arms**: A stretch of DNA that has near identical sequence identity to a complement homologous arm and therefore promote the exchange of two DNA molecules by the cellular process, homology directed repair.
**Immune surveillance**: Process in which the immune system detects and becomes activated by infections, malignancies or other potentially pathogenic alterations.
**Insulator**: A DNA sequence that prevents a gene from being influenced by the activation or repression of nearby genes. Insulators also prevent the spread of heterochromatin from a silenced gene to an actively transcribed gene.
**Integration**: The physical ligation of a DNA sequence into a chromosome of a cell
**Integration couple**: A paired genetic donor vector and genomic receiver site
**Internal ribosome entry site (IRES)**: A DNA sequence that once transcribed encodes a RNA element that allows the initiation of translation in a cap-independent manner
**J-region**: Joining region. One of the gene segments that is used to assemble the T-cell receptor. Each individual has a large number of different variations of these regions making it possible for each individual to arm T-cells with a very large variety of different TCR.
**Karyotype**: The chromosome composition of a cell
**Kozak Sequence**: Short sequence required for the efficient initiation of translation
**Major HLA class I**: a Family of APX that comprise of the genes HLA-A, HLA-B and HLA-C
**Matched**: When two components encode genetic elements that direct and restrict the interaction between the complemented components
**Meganuclease recognition site**: A DNA sequence that is recognized by a endodeox-yribonuclease, commonly referred to as a meganuclease
**Metabolite**: A molecule created or altered through metabolic pathways of the cell
**Mobile genetic element**: A DNA sequence that can permit the integration of DNA with the activity of transposases enzymes
**Monoclone cell line**: A defined group of cells produced from a single ancestral cell by repeated cellular replication
**Native**: a entity that is naturally occuring to the cell
**Non-coding gene**: A non protein coding DNA sequence that is transcribed into functional non-coding RNA molecules
**ORF**: Open reading frame. Stretch of genetic material that encodes a translation frame for synthesis of a protein (polypeptide) by the ribosome
**Paracrine**: Signalling through soluble factors that directly act on neighboring cells.
**PCR**: Polymerase chain reaction in which a specific target DNA molecule is exponentially amplified
**Peptide**: short string of amino acids between 6-30 amino acids in length
**Phenotypic analysis**: Analysis of the observable characteristics of a cell.
**Polymorphic**: Present in different forms in individuals of the same species through the presence of different alleles of the same gene.
**Polypeptide**: Protein consisting of a stretch of peptides, forming a three-dimensional structure.
**Primary Outputs**: eAPC cells and eTPC cells from which the terminal outputs can be derived and/or determined from
**Primer**: Short DNA sequence that allows specific recognition of a target DNA sequence for example during a PCR.
**Promoter**: Regulatory DNA element for the controlled initiation of gene expression
**Selectable marker**: A DNA sequence that confers a trait suitable for artificial selection methods
**Shotgun Integration**: The process whereby a library of vectors is introduced to a population of cells, whereby only a single copy of any given vector insert may be integrated to the genome of each single cell. Used to refer to pooled vector integration to a given cell population via an integration couple
**Slice acceptor site**: A DNA sequence at the 3' end of the intron AM, APX CM or affinity reagent for interaction with cells with TCRsp on the surface, or TCRsp based reagents
**Slice donor site**: A DNA sequence at the 5' end of the intron
**Synthetic**: an entity that is artificially generated and introduced to a cell
**T-cell**: T lymphocyte. White blood cell that expresses a T-cell receptor on its surface. Selected by the immune system to not react with the own body but have the potential to recognize infections and malignancies as well as reject grafts from most members of the same species.
**TCR**: T-cell Receptor. Affinity molecule expressed by a subgroup of lymphocytes called T-lymphocytes. In humans the TCR recognizes cargo presented by APX CM or APX AM, including fragments from virus or bacterial infections or cancerous cells. Therefore, the TCR recognition is an integral part of the adaptive immune system. The TCR consists of two chains that are paired on the cell surface. The TCR expressed on the surface of each cells is assembled at random from a large pool of varied genes (the v,d,j and c regions) and thus each individual has a pool of T-cells expressing a very large and diverse repertoire of different TCRs.
**TCRsp**: A pair of complementary TCR chains that express as surface proteins in complex with CD3 or a pair of complementary TCR chains expressed as proteins in the form of a soluble reagent, an immobilised reagent or present by NCBP.
**Terminal Outputs**: analyte antigen and TCR sequences, in the form of AM, APX, APX:CM, APX:AM, or TCRsp
**TRA**: TCR alpha encoding locus. One of the four different locus encoding genes that can form a VDJ recombined TCR chain. Translated TCR alpha chain proteins typically pair with translated TCR beta chain proteins to form alpha/beta TCRsp.
**TRB**: TCR beta encoding locus. One of the four different locus encoding genes that can form a VDJ recombined TCR chain. Translated TCR beta chain proteins typically pair with TCR alpha chain proteins to form alpha/beta TCRsp.
**TRD**: TCR delta encoding locus. One of the four different locus encoding genes that can form a VDJ recombined TCR chain. Translated TCR delta chain proteins typically pair with translated TCR gamma chain proteins to form gamma/delta TCRsp.
**TRG**: TCR gamma encoding locus. One of the four different locus encoding genes that can form a VDJ recombined TCR chain. Translated TCR gamma chain proteins typically pair with translate TCR delta chain proteins to form gamma/delta TCRsp.
**V-region**: Variable region. One of the gene segments that is used to assemble the T-cell receptor. Each individual has a large number of different variations of these regions making it possible for each individual to arm T-cells with a very large variety of different TCR.

### Items I

1. A two-part device, wherein a first part is an **engineered antigen-presenting cell system (eAPCS)**, and a second part is an **engineered TCR-presenting cell system (eTPCS).**
2. A two-part device according to **item 1** wherein **eAPCS** provides the one or more of analyte eAPC selected from
   a. eAPC-p and/or
   b. eAPC-a, and/or
   c. eAPC-pa, and/or
   d. one or more libraries of a and/or b and/or c.
3. A two-part device according to **item 2**, wherein an eAPC-p, eAPC-a or eAPC-pa expresses an **analyte antigen** selected from
   a. an aAPX or
   b. an aAM or
   c. an aAPX:aAM or
   d. an aAPX:CM or
   e. a combination thereof.
4. A two-part device according to **item 1 or 2** wherein **eTPCS** provides the one or more analyte eTPC selected from
   a. eTPC-t and/or
   b. one or more libraries thereof.
5. A two-part device according to item 4, wherein an analyte pair of TCR chains are expressed as TCR surface proteins in complex with CD3 (**TCRsp**) by an analyte eTPC.
6. A two-part device according to any of the preceding items wherein the one or more analyte eAPC, is combined with the one or more analyte eTPC.
7. A two-part device according to **item 6**, wherein the combination results in a contact between an analyte TCRsp and an analyte antigen as defined in item 3.
8. A two-part device according to **item 7** wherein the contact can result in the formation of a complex between the analyte TCRsp and the analyte antigen.
9. A two-part device according to **item 8** wherein a formation of a complex, if any, can induce a signal response in the analyte eTPC and/or the analyte eAPC.
10. A two-part device according to item 9, wherein the response is used to select an analyte eTPC or a pool of analyte eTPC with or without a signal response and/or analyte eAPC or a pool of analyte eAPC with or without a signal response.
11. An analyte eTPC obtained from the two-part device according to any of **the preceding items** for use in characterisation of a signal response of the analyte eTPC, expressing analyte **TCRsp**, to an **analyte antigen.**
12. A method for selecting one or more analyte eTPC from an input analyte eTPC or a library of analyte eTPC, to obtain one or more analyte eTPC wherein the expressed TCRsp binds to one or more analyte antigen as defined in item 3, wherein the method comprises
   a. Combining one or more analyte eTPC with one or more analyte eAPC resulting in a contact between an analyte TCRsp with an analyte antigen and at least one of
   b. Measuring a formation, if any, of a complex between one or more analyte TCRsp with one or more analyte antigen and/or
   c. Measuring a signal response by the analyte eTPC, if any, induced by the formation of a complex between one or more analyte TCRsp with one or more analyte antigen and/or
   d. Measuring a signal response by the analyte eAPC, if any, induced by the formation of a complex between one or more analyte TCRsp with one or more analyte antigen and
   e. Selecting one or more analyte eTPC based on step b, c and/or d wherein the selection is made by a positive and/or negative measurement.
13. A method according item **12** wherein the selection step e is performed by single cell sorting and/or cell sorting to a pool.
14. A method according to **item 13** wherein the sorting is followed by expansion of the sorted single cell.
15. A method according to **item 13** wherein the sorting is followed by expansion of the sorted pool of cells.
16. A method according to any of **items 13 to 15** further comprising a step of sequencing component 2B' and/or component 2D' of the sorted and/or expanded cell(s).
17. A method according to **item 16** wherein the sequencing step is preceded by the following
   a. Extracting of genomic DNA and/or
   b. Extracting of component 2B' and/or component 2D' RNA transcript and/or
   c. Amplifying by a PCR and/or a RT-PCR of the DNA and/or RNA transcript of component 2B' and/or component 2D'.
18. A method according to **item 16 or 17** wherein the sequencing step is destructive to the cell and wherein the sequencing information obtained is used for preparing the analyte eTPC selected in step e of item 12.
19. A method according to any of **items 12, 13, 14, 15, 18** wherein the selected analyte eTPC is subjected to characterisation of the signal response wherein the method further comprises
   a. Determining a native signalling response and/or
   b. Determining a synthetic signalling response, if the eTPC contains component 2F.
20. A method according to **item 19** wherein the induced signal response is determined by detecting an increase or decrease in one or more of the following
   a. a secreted biomolecule
   b. a secreted chemical
   c. an intracellular biomolecule
   d. an intracellular chemical
   e. a surface expressed biomolecule
   f. a cytotoxic action of the analyte eTPC upon the analyte **eAPC**
   g. a paracrine action of the analyte eTPC upon the analyte **eAPC** such that a signal response is induced in the analyte eAPC and is determined by detecting an increase or decrease any of a to e
   h. a proliferation of the analyte eTPC
   i. an immunological synapse between the **analyte eTPC** and the analyte **eAPC**
   compared to the non-induced signal response state.
21. An analyte eAPC, obtained from the two-part device as defined in items 1 to 10 to identify the analyte antigen that induces a signal response of one or more analyte eTPC expressing an analyte **TCRsp** to the expressed **analyte antigen.**
22. A method for selecting one or more **analyte eAPC** from an input **analyte eAPC** or a library of **analyte eAPC**, to obtain one or more analyte eAPC that induces a signal response of one or more analyte eTPC expressing an analyte **TCRsp** to the expressed **analyte antigen** as defined in **item 3**, wherein the method comprises
   a. Combining one or more analyte eAPC with one or more analyte eTPC, resulting in a contact between an analyte antigen presented by the analyte eAPC with analyte TCRsp of one or more analyte eTPC and
   b. Measuring a formation, if any, of a complex between one or more analyte antigen with one or more analyte TCRsp and/or
   c. Measuring a signal response in the one or more analyte eTPC, if any, induced by the formation of a complex between the analyte TCRsp with the analyte antigen and/or
   d. Measuring a signal response, if any, by the analyte eAPC induced by the formation of a complex between one or more analyte TCRsp with one or more analyte antigen and
   e. Selecting one or more analyte eAPC from step b, c and/or d wherein the selection is made by a positive and/or negative measurement.
23. A method according item **22** wherein the selection step e is performed by single cell sorting and/or cell sorting to a pool.
24. A method according to **item 23** wherein the sorting is followed by expansion of the sorted single cell.
25. A method according to **item 24** wherein the sorting is followed by expansion of the sorted pool of cells.
26. A method according to any of **items 23 to 25** further comprising a step of sequencing component 1B' and/or component 1D' of the sorted and/or expanded cell(s).
27. A method according to **item 26** wherein the sequencing step is preceded by the following
   a. Extracting of genomic DNA and/or
   b. Extracting of component 1B' and/or component 1D' RNA transcript
      and/or
   c. Amplifying by a PCR and/or a RT-PCR of the DNA and/or RNA transcript of component 1B' and/or component 1D'.
28. A method according to **item 26 or 27** wherein the sequencing step is destructive to the cell and wherein the sequencing information obtained is used for preparing the analyte eAPC selected in step e of item 22.
29. A method according to any of **items 22, 23, 24, 25, 28** wherein the selected **analyte eAPC** is select based on the signal response of an analyte eTPC wherein the method further comprises
   a. Determining a native signalling response and/or
   b. Determining a synthetic signalling response.
30. A method according to **item 29** wherein the induced signal response is determined by detecting an increase or decrease in one or more of the following
   a. a secreted biomolecule
   b. a secreted chemical
   c. an intracellular biomolecule
   d. an intracellular chemical
   e. a surface expressed biomolecule
   f. a cytotoxic action of the analyte eTPC upon the analyte **eAPC**
   g. a paracrine action of the analyte eTPC upon the analyte **eAPC** such that a signal response is induced in the analyte eAPC and is determined by detecting an increase or decrease any of a to e
   h. a proliferation of the analyte eTPC
   i. an immunological synapse between the analyte eTPC and the analyte **eAPC**
   compared to the non-induced signal response state.
31. A method to select and identify an **aAM** cargo or a **CM** cargo, wherein the cargo is a metabolite and/or a peptide, that is loaded in an **aAPX** of an analyte e**APC** selected and obtained by methods defined in items 22 to 30 wherein the method comprises
   a. isolating an **aAPX:aAM** or an **aAPX:CM** or the cargo aM or the cargo CM and
   b. identifying the loaded cargo.
32. A method according to item 31 wherein step b comprises subjecting the isolated **aAPX:aAM** or an **aAPX:CM** to one or more
   a. Mass-spectroscopy analysis
   b. Peptide sequencing analysis.
33. A pair of TCR chain sequences or library of pairs of TCR chain sequences selected by the method as defined in items 12 to 20 for use in at least one of the following
   a. diagnostics
   b. medicine
   c. cosmetics
   d. research and development.
34. An antigenic molecule and/or ORF encoding said antigenic molecule, or libraries thereof selected by the method as defined in items 22 to 32 for use in at least one of the following
   a. diagnostics
   b. medicine
   c. cosmetics
   d. research and development.
35. A antigen-presenting complex loaded with an antigenic molecule as cargo and/or ORF(s) encoding said complex, or libraries thereof selected by the method as defined in items 22 to 32 for use in at least one of the following
   a. diagnostics
   b. medicine
   c. cosmetics
   d. research and development.
36. An eAPC, or library of eAPC selected by the method as defined in items 22 to 30 for use in at least one of the following
   a. diagnostics
   b. medicine
   c. cosmetics
   d. research and development.
37. An eTPC, or library of eTPC selected by the method as defined in items 12 to 20 for use in at least one of the following
   a. diagnostics
   b. medicine
   c. cosmetics
   d. research and development.
38. A device according to any of items 1-10 for use in at least one of the following
   a. diagnostics
   b. medicine
   c. cosmetics
   d. research and development.

### Items 2

1. A two-part device, wherein a first part is an **engineered antigen-presenting cell system (eAPCS)**, and a second part is an **engineered TCR-presenting cell system (eTPCS).**
2. A two-part device according to **item 1** wherein **eAPCS** provides the one or more of analyte eAPC selected from
   a. eAPC-p and/or
   b. eAPC-a, and/or
   c. eAPC-pa, and/or
   d. one or more libraries of a and/or b and/or c.
3. A two-part device according to **item 2**, wherein an eAPC-p, eAPC-a or eAPC-pa expresses an **analyte antigen** selected from
   a. an aAPX or
   b. an aAM or
   c. an aAPX:aAM or
   d. an aAPX:CM or
   e. a combination thereof.
4. A two-part device according to **item1 or 2** wherein **eTPCS** provides one or more analyte eTPC selected from
   a. eTPC-t and/or
   b. one or more libraries thereof.
5. A two-part device according to item 4, wherein an analyte pair of TCR chains are expressed as TCR surface proteins in complex with CD3 (analyte **TCRsp**) by an analyte eTPC.
6. A two-part device according to item 5, wherein the eTPC contains a component 2F, representing a synthetic TCR signal response element engineered to the genome of the eTPC, and which is used to report the formation of complex between analyte TCRsp and a analyte antigen presented by eAPC -p, -a or -pa, and which results in a signal response in the eTPC.
7. A two-part device according to any of the preceding items, wherein one or more analyte eAPC, is combined with one or more analyte eTPC.
8. A two-part device according to **item 7**, wherein the combination results in a contact between an analyte TCRsp and an analyte antigen as defined in item 3, wherein the contact may or may not result in a signal response.
9. A two-part device according to **item 8**, wherein the contact may result in the formation of a complex between the analyte TCRsp and the analyte antigen.
10. A two-part device according to **item 9** wherein a formation of a complex, if any, can induce a signal response in the analyte eTPC and/or the analyte eAPC.
11. A two-part device according to any of items 7-9, wherein the signal response is used to select an analyte eTPC or a pool of analyte eTPC with or without a signal response and/or analyte eAPC or a pool of analyte eAPC with or without a signal response.
12. An analyte eTPC obtained from the two-part device according to any of **the preceding items** for use in characterisation of a signal response of the analyte eTPC, expressing analyte **TCRsp**, to an **analyte antigen.**
13. A method for selecting one or more eTPC from an input analyte eTPC or a library of analyte eTPC, to obtain one or more analyte eTPC wherein the expressed TCRsp binds to one or more analyte antigen as defined in item 3, wherein the method comprises
   a. Combining one or more analyte eTPC with one or more analyte eAPC resulting in a contact between an analyte TCRsp with an analyte antigen and at least one of
   b. Measuring a formation, if any, of a complex between one or more analyte TCRsp with one or more analyte antigen and/or
   c. Measuring a signal response by the analyte eTPC, if any, induced by the formation of a complex between one or more analyte TCRsp with one or more analyte antigen and/or
   d. Measuring a signal response by the analyte eAPC, if any, induced by the formation of a complex between one or more analyte TCRsp with one or more analyte antigen and
   e. Selecting one or more eTPC based on step b, c and/or d wherein the selection is made by a positive and/or negative measurement.
14. A method according item **13** wherein the selection step e is performed by single cell sorting and/or cell sorting to a pool.
15. A method according to **item 14** wherein the sorting is followed by expansion of sorted single cell.
16. A method according to **item 14** wherein the sorting is followed by expansion of sorted pool of cells.
17. A method according to any of **items 13, 14, 15, 16,** wherein the selected eTPC is subjected to characterisation of the signal response wherein the method further comprises
   a. Determining a native signalling response and/or
   b. Determining a synthetic signalling response, if the eTPC contains component 2F
18. An eAPC, obtained from the two-part device as defined in items 1 to 11 to identify the analyte antigen that induces a signal response of one or more analyte eTPC expressing an analyte **TCRsp** to the expressed **analyte antigen.**
19. A method for selecting one or more **eAPC** from an input **analyte eAPC** or a library of **analyte eAPC**, to obtain one or more analyte eAPC that induces a signal response of one or more analyte eTPC expressing an analyte **TCRsp** to the expressed **analyte antigen** as defined in **item 3**, wherein the method comprises
   a. Combining one or more analyte eAPC with one or more analyte eTPC, resulting in a contact between an analyte antigen presented by the analyte eAPC with analyte TCRsp of one or more analyte eTPC and
   b. Measuring a formation, if any, of a complex between one or more analyte antigen with one or more analyte TCRsp and/or
   c. Measuring a signal response in the one or more analyte eTPC, if any, induced by the formation of a complex between the analyte TCRsp with the analyte antigen and/or
   d. Measuring a signal response, if any, by the analyte eAPC induced by the formation of a complex between one or more analyte TCRsp with one or more analyte antigen and
   e. Selecting one or more eAPC from step b, c and/or d wherein the selection is made by a positive and/or negative measurement.
20. A method according item 19 wherein the selection step e is performed by single cell sorting and/or cell sorting to a pool.
21. A method according to **item 20** wherein the sorting is followed by expansion of the sorted single cell.
22. A method according to **item 21** wherein the sorting is followed by expansion of the sorted pool of cells.

## Claims

1. A method for selecting one or more engineered antigen-presenting cell (**eAPC)** from an input **analyte eAPC** or a library of **analyte eAPC,** to obtain one or more analyte eAPC that induces a signal response of one or more analyte **engineered** T-cell receptor (**TCR)-presenting cell** (eTPC) expressing an analyte expressed TCR surface proteins in complex with CD3 (**TCRsp)** to the expressed **analyte antigen** selected from:
a. an analyte antigen presenting complex (aAPX), or
b. an analyte antigen molecule (aAM), or
c. an aAPX:aAM, or
d. an aAPX:CM (cargo molecule), or
e. a combination thereof,
wherein the method comprises:
i. combining one or more analyte eAPC with one or more analyte eTPC, resulting in a contact between an analyte antigen presented by the analyte eAPC with analyte TCRsp of one or more analyte eTPC and
ii. measuring a formation, if any, of a complex between one or more analyte antigen with one or more analyte TCRsp and/or
iii. measuring a signal response in the one or more analyte eTPC, if any, induced by the formation of a complex between the analyte TCRsp with the analyte antigen and/or
iv. measuring a signal response, if any, by the analyte eAPC induced by the formation of a complex between one or more analyte TCRsp with one or more analyte antigen and
v. selecting one or more eAPC from step ii, iii and/or iv wherein the selection is made by a positive and/or negative measurement.

2. A method according claim 1 wherein the selection step v is performed by single cell sorting and/or cell sorting to a pool.

3. A method according to claim 2 wherein the sorting is followed by expansion of the sorted single cell.

4. A method according to claim 2 wherein the sorting is followed by expansion of the sorted pool of cells.

5. A method according to any of claims 2 to 4 further comprising a step of sequencing component 1B' and/or component 1D' of the sorted and/or expanded cell(s).

6. A method according to claim 5 wherein the sequencing step is preceded by the following
a. Extracting of genomic DNA and/or
b. Extracting of component 1 B' and/or component 1 D' RNA transcript and/or
c. Amplifying by a PCR and/or a RT-PCR of the DNA and/or RNA transcript of component 1B' and/or component 1D'.

7. A method according to claims 5 or 6 wherein the sequencing step is destructive to the cell and wherein the sequencing information obtained is used for preparing the analyte eAPC selected in step v of claim 1.

8. A method according to any of **claims 1-4** and 7 wherein the selected **analyte eAPC** is selected based on the signal response of an analyte eTPC wherein the method further comprises
a. Determining a native signalling response and/or
b. Determining a synthetic signalling response.

9. A method according to **claim 5** wherein the induced signal response is determined by detecting an increase or decrease in one or more of the following
a. a secreted biomolecule
b. a secreted chemical
c. an intracellular biomolecule
d. an intracellular chemical
e. a surface expressed biomolecule
f. a cytotoxic action of the analyte eTPC upon the analyte **eAPC**
g. a paracrine action of the analyte eTPC upon the analyte **eAPC** such that a signal response is induced in the analyte eAPC and is determined by detecting an increase or decrease any of a to e
h. a proliferation of the analyte eTPC
i. an immunological synapse between the analyte eTPC and the analyte **eAPC**
compared to the non-induced signal response state.
